(19) Europäisches Patentamt
European Patent Office
Office européen des brevets

(11) EP 4 167 248 A1

# (12) EUROPEAN PATENT APPLICATION
published in accordance with Art. 153(4) EPC

(43) Date of publication:
**19.04.2023 Bulletin 2023/16**

(21) Application number: **22793498.1**

(22) Date of filing: **25.08.2022**

(51) International Patent Classification (IPC):
***G16H 50/50*** (2018.01) ***G16H 50/70*** (2018.01)
***G16H 50/20*** (2018.01) ***G16H 50/30*** (2018.01)
***G06N 3/08*** (2023.01) ***A61B 5/11*** (2006.01)
***A61B 5/00*** (2006.01)

(86) International application number:
**PCT/KR2022/012766**

(87) International publication number:
**WO 2023/033459 (09.03.2023 Gazette 2023/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.08.2021 KR 20210114931**
**16.08.2022 KR 20220102045**

(71) Applicant: **Fitogether Inc.**
**Seoul 04378 (KR)**

(72) Inventors:
- **KIM, Jeong Bin**
  **Seoul 08758 (KR)**
- **LEE, Jae Chan**
  **Yongin-si**
  **Gyeonggi-do 16874 (KR)**
- **LEE, Jong Hyun**
  **Seoul 04759 (KR)**
- **KIM, Hyun Sung**
  **Seoul 04116 (KR)**
- **KO, Sang Ki**
  **Chuncheon-si**
  **Gangwon-do 24433 (KR)**
- **YOON, Jin Sung**
  **Seoul (KR)**

(74) Representative: **Frenkel, Matthias Alexander**
**Wuesthoff & Wuesthoff**
**Patentanwälte PartG mbB**
**Schweigerstraße 2**
**81541 München (DE)**

## (54) METHOD FOR PROVIDING EXERCISE LOAD INFORMATION

(57) A method for providing exercise load information of a target entity is provided. The method includes obtaining a target data set of the target entity for a target exercise session including a plurality of time units, wherein the target data set includes a sequence of kinematic information of the target entity for each of the plurality of time units; and determining, based on the target data set, an estimated load index reflecting a level of exercise load of the target entity for the target exercise session using an artificial neural network (ANN).

FIG. 5

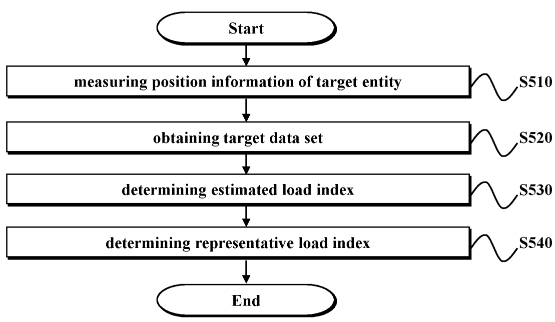


EP 4 167 248 A1

## Description

### Technical field

The present disclosure generally relates to the field of sports analytics. More specifically, the present disclosure relates to calculating information reflecting exercise load, or techniques using the same, but is not limited thereto.

### Background art

Sports analytics is becoming increasingly important as the sports industry has expanded immensely and sports science has evolved. In this trend, electronic performance and tracking system (EPTS) for tracking and monitoring players during a game or training session has been increasingly introduced recently, in major sports including football.

The EPTS for tracking player positions or movements in real time allows objective analysis on sports by quantifying not only simple indicators such as the distance that a player ran or average running speed but also relatively complex information such as the heat map for each player. Thus, the EPTS is a new approach to sports analytics that greatly relied on the observation and intuition of coaches or sports analyzers in the past.

However, such data-based sports analysis techniques still provide simple information on positions or movements, or indicators obtained by simply processing the information. There is a demand for development of indicators or analysis information from various viewpoints.

### Detailed description of invention

### Technical task

Hereinafter, a summary of some embodiments of the present disclosure will be provided. It should be understood that the aspects provided in the following summary are only to summarize some embodiments in a simplified form and are not intended to limit the scope of the present disclosure. Thus, the present disclosure may include various aspects that are not presented below.

The present disclosure and concept of invention disclosed provide methods for calculating information indicating exercise load or devices, and computer-readable storage media using the same.

It is a task of the present disclosure to provide a method for determining exercise load information of a target entity using kinematic information of the target entity.

It is a task of the present disclosure to provide a method for providing exercise load information which outputs a notification message based on an estimated load index and a response load index of the target entity.

It is a task of the present disclosure to provide a method for providing exercise load information which adaptively determines the estimated load index of the corresponding target entity based on at least one of identification information or characteristic information of the target entity.

It is a task of the present disclosure to provide a method for providing exercise load information which determines the estimated load index in consideration of both kinematic information of the target entity and condition information reflecting a characteristic of a corresponding exercise session.

The tasks of the present disclosure sought to be solved are not limited to the aforementioned tasks, and may be variously extended in a scope not deviating the idea and area of the present disclosure.

### Means for solving technical task

The embodiments include methods for calculating information indicating exercise load or devices, and computer-readable storage media using the same.

According to an embodiment of the present disclosure, a method for providing exercise load information of a target entity, which comprises: obtaining a target data set of the target entity for a target exercise session including a plurality of time units, wherein the target data set includes a sequence of kinematic information of the target entity for each of the plurality of time units; and determining, based on the target data set, an estimated load index reflecting a level of exercise load of the target entity for the target exercise session using an artificial neural network (ANN), may be provided.

According to another embodiment of the present disclosure, a method for providing exercise load information of a target entity, which comprises: calculating, based on kinematic information of the target entity for a target exercise session, an estimated load index reflecting an estimate for a level of exercise load of the target entity for the target exercise session; receiving a response load index reflecting a self-evaluation value for a level of exercise load of the target exercise session by the target entity; and outputting a notification message based on a result of comparison between the estimated load index and the response load index, may be provided. According to yet another embodiment of the present disclosure, a method for providing exercise load information of a target entity, which comprises: obtaining target-specific information reflecting at least one of identification information or characteristic information of the target entity; obtaining a target data set of the target entity for the target exercise session including a plurality of time units, wherein the target data set includes a sequence of kinematic information of the target entity for each of the plurality of time units; and determining, based on the target-specific information and the target data set, an estimated load index reflecting a level of exercise load of the target entity for the target exercise session using an artificial neural network (ANN), may be provided.

According to still another embodiment of the present disclosure, a method for providing exercise load information of a target entity, which comprises: obtaining a target data set of the target entity for a target exercise session including a plurality of time units, wherein the target data set includes a sequence of kinematic information of the target entity for each of the plurality of time units and condition information reflecting a characteristic related to the target exercise session; and determining, based on the target data set, an estimated load index reflecting a level of exercise load of the target entity for the target exercise session using an artificial neural network (ANN), may be provided.

The above-mentioned exemplary embodiments and other exemplary embodiments will be explained or clarified by the following detailed description on exemplary embodiments that will be read in associated with the accompanying drawings.

**Effect of invention**

The techniques disclosed may have the following effects. However, this does not mean that a specific embodiment should have all of the following effects or only the following effects. Thus, it should not be construed that the scope of the present disclosure is limited thereby.

According to the embodiments of the present disclosure, quantified indices related to exercise load may be provided or management of training sessions or sports players may be improved.

According to the embodiments of the present disclosure, the exercise load information of the target entity may be determined using kinematic information of the target entity, thereby obtaining information related to the level of exercise load of the target entity for the target session without going through an additional response procedure.

According to the embodiments of the present disclosure, the notification message may be outputted based on the estimated load index and response load index of the target entity, thereby performing a condition monitoring of the target entity. According to the embodiments of the present disclosure, the estimated load index may be adaptively determined for the corresponding target entity based on at least one of the identification information or characteristic information of the target entity, thereby determining exercise load information more accurately in accordance with the characteristic of the target entity.

According to the embodiments of the present disclosure, the estimated load index may be determined in consideration of the kinematic information of the target entity and condition information reflecting the characteristic of the corresponding exercise session, thereby obtaining more accurate exercise load information reflecting the condition of the target session intended to determine the level of exercise load.

The above summary is not intended to be an exhaustive list of all aspects of the present disclosure. It should be understood that the present disclosure includes all methods, apparatuses and systems that can be practiced from all suitable combinations of the various aspects summarized above, as well as those disclosed in the following detailed description and claims.

**Brief description of drawings**

Fig. 1 illustrates a system for providing exercise load information according to an embodiment of the present disclosure;

Fig. 2 is a block diagram of an attachable device according to an embodiment of the present disclosure;

Fig. 3 is a block diagram of a server according to an embodiment of the present disclosure;

Fig. 4 illustrates an example of an exercise load index according to an embodiment of the present disclosure;

Fig. 5 is a schematic flow chart illustrating a method for providing exercise load information according to an embodiment of the present disclosure;

Fig. 6 is a detailed flow chart illustrating a step of determining an estimated load index in Fig. 5;

Fig. 7 illustrates an exemplary artificial neural network (ANN) used for calculating the exercise load index according to an embodiment of the present disclosure;

Fig. 8 illustrates an example of a training data set of the ANN according to an embodiment of the present disclosure;

Fig. 9 is a detailed view of an example of a sequence of kinematic information in Fig. 8;

Fig. 10 illustrates an example of an input data set of the ANN according to an embodiment of the present disclosure;

Fig. 11 illustrates another example of the input data set of the ANN according to an embodiment of the present disclosure;

Fig. 12 exemplarily illustrates an entity-based coordinate system;

Fig. 13 exemplarily illustrates a field-based coordinate system;

Fig. 14 illustrates a difference between a movement direction at a previous time point and a movement direction at a present time point;

Fig. 15 illustrates an example of kinematic information according to an embodiment of the present disclosure;

Fig. 16 illustrates an example of the kinematic information according to an embodiment of the present disclosure;

Fig. 17 illustrates an example of coordinate systems capable of expressing the kinematic information according to an embodiment of the present disclosure;

Fig. 18 illustrates an example of static information;

Fig. 19 illustrates an example of first kinematic information including information related to velocity;

Fig. 20 illustrates an example of second kinematic information including information related to acceleration;

Fig. 21 illustrates an example of third kinematic information including information related to jerk;

Fig. 22 illustrates an example of angular movement information based on an inertial coordinate system;

Fig. 23 illustrates an example of angular movement information based on a hybrid coordinate system;

Fig. 24 is a schematic flow chart illustrating a method for providing exercise load information according to an embodiment of the present disclosure;

Fig. 25 is a detailed flow chart illustrating a step of determining an estimated load index in Fig. 24;

Fig. 26 illustrates activity information according to an embodiment of the present disclosure;

Fig. 27 illustrates an example of a Wellness survey for securing daily readiness response information according to an embodiment of the present disclosure;

Fig. 28 is a schematic flow chart illustrating a method for providing exercise load information according to an embodiment of the present disclosure;

Fig. 29 is a detailed flow chart illustrating an embodiment of a step of determining an estimated load index in Fig. 28;

Fig. 30 is a detailed flow chart illustrating another embodiment of the step of determining the estimated load index in Fig. 28;

Fig. 31 is an exemplary view illustrating determination of a match load index;

Fig. 32 is an exemplary view illustrating determination of the estimated load index using a match data set and a training data set;

Fig. 33 illustrates a model of an early fusion in a network fusion;

Fig. 34 illustrates a model of a middle fusion in the network fusion;

Fig. 35 illustrates a model of a late fusion in the network fusion;

Fig. 36 is a conceptual diagram illustrating a method for providing exercise load information for entity monitoring and early risk detection according to an embodiment of the present disclosure;

Fig. 37 is a schematic flow chart illustrating a method for providing exercise load information according to an embodiment of the present disclosure;

Fig. 38 is a detailed flow chart illustrating a step of calculating the estimated load index in Fig. 37;

Fig. 39 is a detailed flow chart illustrating a step of determining the estimated load index in Fig. 38;

Fig. 40 is a schematic structure of the ANN for implementing the method for providing exercise load information according to an embodiment of the present disclosure;

Fig. 41 illustrates an example of providing exercise load information using kinematic information;

Fig. 42 is an exemplary view illustrating the ANN in consideration of time information;

Fig. 43 is an exemplary view illustrating a method for providing real-time exercise load information according to an embodiment of the present disclosure;

Fig. 44 is an exemplary view illustrating kinematic data to the N time point in Fig. 43;

Fig. 45 is an exemplary view illustrating kinematic data to the M time point in Fig. 43; and

Fig. 46 illustrates a procedure for warning threshold load according to real-time exercise load measurement.

**Best mode for carrying out the invention**

Since the present disclosure may be modified in various ways and have various embodiments, some embodiments of the present disclosure will be exemplified in the drawings and described in detail with reference to the drawings.

However, it should be understood that the present disclosure is not limited to a specific embodiment but includes all changes, equivalents and substituents included in the spirit and scope of the present disclosure.

While the terms "first," "second," etc., may be used herein to describe various components, such components should not be limited by the above terms. The above terms are used only to distinguish one component from another. For example, a first component may be named a second component, or vice versa, without departing from the scope of the present disclosure. As used herein, the term "and/or" includes any or all combinations of one or more of the associated listed items.

It should be understood that if a component is referred to, or depicted, as being "connected" to another component, it may be "directly connected to" the other component, or intervening components may be present. However, if a component is referred to, or depicted, as being "directly connected to" another component, it should be construed that no intervening components are present therebetween. The terms used herein are only for the purpose of describing particular embodiments, and are not intended to limit the scope of the present disclosure. As used herein, the singular forms are intended to include the plural forms, unless the context clearly indicates otherwise. It should be understood that the terms "comprises" or "has," etc., specify the presence of described features, integers, steps, operations, elements, components, or combinations thereof, but do not preclude the presence or addition of one or more other features, integers, steps, operations, elements, components, or combinations thereof

Unless defined otherwise, each technical or scientific term used herein has the same meaning as commonly understood by those skilled in the art to which the present disclosure pertains. Terms such as those defined in a generally used dictionary should be interpreted to have the same meanings as the contextual meanings in the relevant field of art, and should not be interpreted to have ideal or excessively formal meaning unless clearly defined in the present disclosure.

Hereinafter, preferred embodiments of the present disclosure will be described in detail with reference to the accompanying drawings. For a full understanding of the present disclosure, like reference numerals designate corresponding components in the drawings and detailed description thereof will be omitted.

The embodiments described in the present disclosure are to clearly explain the spirit of the present disclosure to those skilled in the art to which the present disclosure pertains, and the present disclosure is not limited to the described embodiments. It should be construed that the scope of the present disclosure includes alternations or modifications without departing from the present disclosure.

The terms used herein are general terms selected as those being now used as widely as possible in the art to which the present disclosure pertains, but they may vary depending on the intention of those skilled in the art or the convention or the emergence of new technology. In certain cases, there may be terms arbitrarily defined, and in this case, the meaning thereof will be described separately.

Accordingly, the terms used herein should be defined based on the meaning of the terms and the context throughout the specification, rather than simply the name of the terms.

The accompanying drawings are only for the purpose of illustrating the present disclosure, and certain features or structures in the drawings may be exaggerated or diminished according to the need. Thus, the present disclosure is not limited to the drawings.

The detailed description of known features or functions related to the present disclosure will be omitted when it is determined to make the gist of the present disclosure obscure.

A method for providing exercise load information of a target entity according to an aspect of the present disclosure may include: obtaining a target data set of the target entity for a target exercise session including a plurality of time units, wherein the target data set includes a sequence of kinematic information of the target entity for each of the plurality of time units; and determining, based on the target data set, an estimated load index reflecting a level of exercise load of the target entity for the target exercise session using an artificial neural network (ANN).

Here, the target entity may be at least one sports team, and the method may further include determining a representative load index of the sports team for the target exercise session based on estimated load indices for each of the at least some sports players belonging to the sports team.

Here, determining the estimated load index may include: preparing the ANN trained based on a plurality of training data sets including a first training data set, wherein the first training data set includes a sequence of kinematic information of a first entity for a first exercise session, and is labeled with a score collected from the first entity for the exercise load of the first exercise session; inputting the target data set to an input layer of the ANN; and obtaining the estimated load index based on a result of an output layer of the ANN.

Here, the method may further include measuring position information of the target entity for the target exercise session using a positioning device corresponding to the target entity, wherein the kinematic information is generated based on the position information.

Here, the kinematic information may be measured using an inertial sensor corresponding to the target entity.

Here, the kinematic information may include first kinematic information related to a velocity of the target entity; and second kinematic information related to an acceleration of the target entity.

Here, the kinematic information may further include third kinematic information related to a jerk of the target entity.

Here, the first kinematic information may include information related to the magnitude of velocity in a target time unit; and information related to the angular change between a direction of velocity in a time unit prior to the target time unit and a direction of velocity in the target time unit.

Here, the second kinematic information may include information related to the magnitude of acceleration in the target time unit; and information related to the angular change between a direction of acceleration in the time unit prior to the target time unit and a direction of acceleration in the target time unit.

Here, the second kinematic information may include information related to the magnitude of acceleration in the target time unit; and information related to the angular change between a direction of velocity in the time unit prior to the target time unit and a direction of velocity in the target time unit.

Here, the third kinematic information may include information related to the magnitude of jerk in the target time unit; and information related to the angular change between a direction of jerk in the time unit prior to the target time unit and a direction of jerk in the target time unit.

Here, the third kinematic information may include information related to the magnitude of jerk in the target time unit; and information related to the angular change between a direction of acceleration in the time unit prior to the target time unit and a direction of acceleration in the target time unit.

Here, the third kinematic information may include information related to the magnitude of jerk in the target time unit; and information related to the angular change between a direction of velocity in the time unit prior to the target time unit and a direction of velocity in the target time unit.

Here, the kinematic information may be expressed based on a field-based coordinate system, which includes a first axis for a length of a field in which the exercise session is performed; and a second axis for a width of the field in which the exercise session is performed.

Here, the kinematic information may be expressed based on an entity-based coordinate system, which includes a forward-backward axis corresponding to a heading direction of the target entity; and a left-right axis corresponding to a side direction of the target entity.

Here, the kinematic information may include information related to at least one of a rotational movement in a roll direction, a rotational movement in a pitch direction, or a rotational movement in a yaw direction of the target entity; and information related to at least one of angular velocity, angular acceleration or angular jerk of the target entity.

Here, the estimated load index may be an estimate of a ratio of perceived exertion (RPE) of the target entity for the target exercise session.

Here, the target data set may be configured to have a predetermined length by performing zero-padding prior to the sequence of the kinematic information.

An apparatus for providing exercise load information of a target entity according to an aspect of the present disclosure may include a processor and a memory, and the processor may be configured to obtain a target data set of the target entity for a target exercise session including a plurality of time units, wherein the target data set includes a sequence of kinematic information of the target entity for each of the plurality of time units; and determine, based on the target data set, an estimated load index reflecting a level of exercise load of the target entity for the target exercise session using an artificial neural network (ANN).

A non-transitory computer-readable storage medium having processor-executable instructions stored thereon according to an aspect of the present disclosure may be configured such that the instructions are executed by the processor, allowing the processor to obtain a target data set of a target entity for a target exercise session including a plurality of time units, wherein the target data set includes a sequence of kinematic information of the target entity for each of the plurality of time units; and determine, based on the target data set, an estimated load index reflecting a level of exercise load of the target entity for the target exercise session using an artificial neural network (ANN).

A method for providing exercise load information of a target entity according to an aspect of the present disclosure may include: calculating, based on kinematic information of the target entity for the target exercise session, an estimated load index reflecting an estimate for a level of exercise load of the target entity for the target exercise session; receiving a response load index reflecting a self-evaluation value for a level of exercise load of the target exercise session by the target entity; and outputting a notification message based on a result of comparison between the estimated load index and the response load index.

Here, calculating the estimated load index may include: obtaining a target data set of the target entity for the target exercise session including a plurality of time units, wherein the target data set includes a sequence of kinematic information of the target entity for each of the plurality of time units; and determining, based on the target data set, the estimated load index of the target entity using an artificial neural network (ANN).

Here, determining the estimated load index may include: preparing the ANN trained based on a plurality of training data sets including a first training data set, wherein the first training data set includes a sequence of kinematic information of a first entity for a first exercise session, and is labeled with a score collected from the first entity for the exercise load of the first exercise session; inputting the target data set to an input layer of the ANN; and obtaining the estimated load index based on a result of an output layer of the ANN.

Here, the kinematic information may include first kinematic information related to a velocity of the target entity; and second kinematic information related to an acceleration of the target entity.

Here, the kinematic information may further include third kinematic information related to a jerk of the target entity.

Here, the estimated load index may be an estimate of a ratio of perceived exertion (RPE) of the target entity for the target exercise session, and the response load index may reflect the RPE of the target entity for the target exercise session.

Here, the target entity may be at least one sports team, and each of the estimated load index and the response load index may be a representative index determined based on indices for each of the at least some sports players belonging to the sports team.

Here, the target entity may be one sports player.

Here, outputting the notification message may be configured to output the notification message in response to a determination that a difference between the estimated load index and the response load index is greater than or equal to a predetermined first threshold value.

Here, outputting the notification message may be configured to output the notification message in response to a determination that a difference between the estimated load index and the response load index for consecutive exercise sessions greater than or equal to a predetermined first threshold, is greater than or equal to a predetermined second threshold value.

Here, outputting the notification message may be configured to output the notification message in response to a determination that a ratio of a second exercise session, in which a difference between the estimated load index and the response load index among a plurality of consecutive exercise sessions is greater than or equal to a predetermined third threshold value, is greater than or equal to a predetermined first threshold ratio.

Here, the notification message may include a message for warning a risk of injury of the target entity.

Here, the notification message may include a message for warning an abnormal condition of the target entity.

Here, the notification message may include a message for requesting a coach interview of the target entity.

Here, the notification message may include a message for recommending a type change of the ANN.

An apparatus for providing exercise load information of the target entity according to an aspect of the present disclosure may include a processor and a memory, and the processor may be configured to calculate, based on kinematic information of the target entity for the target exercise session, an estimated load index reflecting an estimate for a level of exercise load of the target entity for the target exercise session; receive a response load index reflecting a self-evaluation value for a level of exercise load of the target exercise session by the target entity; and output a notification message based on a result of comparison between the estimated load index and the response load index.

A non-transitory computer-readable storage medium having processor-executable instructions stored thereon according to an aspect of the present disclosure may be configured such that the instructions are executed by the processor, allowing the processor to calculate, based on kinematic information of the target entity for the target exercise session, an estimated load index reflecting an estimate for a level of exercise load of the target entity for the target exercise session; receive a response load index reflecting a self-evaluation value for a level of exercise load of the target exercise session by the target entity; and output a notification message based on a result of comparison between the estimated load index and the response load index.

A method for providing exercise load information of a target entity according to an aspect of the present disclosure may include: obtaining target-specific information reflecting at least one of identification information or characteristic information of the target entity; obtaining a target data set of the target entity for the target exercise session including a plurality of time units, wherein the target data set includes a sequence of kinematic information of the target entity for each of the plurality of time units; and determining, based on the target-specific information and the target data set, an estimated load index reflecting a level of exercise load of the target entity for the target exercise session using an artificial neural network (ANN).

Here, determining the estimated load index may include: preparing the ANN trained based on a plurality of training data sets including a first training data set, wherein the first training data set includes a sequence of kinematic information of a first entity for a first exercise session, and is labeled with a score collected from the first entity for the exercise load of the first exercise session; inputting the target data set to an input layer of the ANN; and obtaining the estimated load index based on a result of an output layer of the ANN.

Here, the target-specific information may include a modifier for the estimated load index corresponding to the target entity, and obtaining the estimated load index may be configured to obtain the estimated load index by multiplying the result of the output layer by the modifier.

Here, the modifier may reflect evaluation information of a fitness level of the target entity.

Here, the modifier may reflect a ratio of a match load index to a maximum value of a load index, wherein the match load index is obtained based on kinematic information of the target entity for at least one recent match session using the ANN.

Here, the modifier may reflect evaluation information of a league to which the target entity belongs.

Here, the modifier may reflect a ratio of an evaluation value of the league to which the target entity belongs to an evaluation value of a league to which the first entity belongs.

Here, determining the estimated load index may include: preparing the ANN trained based on a plurality of training data sets each including a first training data set and a first match data set, wherein the first training data set includes a sequence of kinematic information of a first entity for a first exercise session, and is labeled with a score collected from the first entity for the exercise load of the first exercise session, and wherein the first match data set includes a sequence of kinematic information of the first entity for at least one match session, and is labeled with a maximum score; inputting the target data set and the target-specific information to an input layer of the ANN; and obtaining the estimated load index based on a result of an output layer of the ANN.

Here, the ANN may have been trained using a fusion of a training characteristic map corresponding the first training data set and a match characteristic map corresponding the first match data set.

Here, the target-specific information may include a sequence of kinematic information of the target entity for at least one recent match session.

Here, the target-specific information may include identification information of a league to which the target entity belongs, and determining the estimated load index may be configured to use the ANN trained based on a plurality of training data sets corresponding the league to which the target entity belongs among a plurality of ANNs.

An apparatus for providing exercise load information of a target entity according to an aspect of the present disclosure may include a processor and a memory, and the processor may be configured to obtain target-specific information reflecting at least one of identification information or characteristic information of the target entity; obtain a target data set of the target entity for a target exercise session including a plurality of time units, wherein the target data set includes a sequence of kinematic information of the target entity for each of the plurality of time units; and determine, based on the target-specific information and the target data set, an estimated load index reflecting a level of exercise load of the target entity for the target exercise session using an artificial neural network (ANN).

A non-transitory computer-readable storage medium having processor-executable instructions stored thereon according to an aspect of the present disclosure may be configured such that the instructions are executed by the processor, allowing the processor to obtain target-specific information reflecting at least one of identification information or characteristic information of a target entity; obtain a target data set of the target entity for a target exercise session including a plurality of time units, wherein the target data set includes a sequence of kinematic information of the target entity for each of the plurality of time units; and determine, based on the target-specific information and the target data set, an estimated load index reflecting a level of exercise load of the target entity for the target exercise session using an artificial neural network (ANN).

A method for providing exercise load information of a target entity according to an aspect of the present disclosure may include: obtaining a target data set of the target entity for a target exercise session including a plurality of time units, wherein the target data set includes a sequence of kinematic information of the target entity for each of the plurality of time units and condition information reflecting a characteristic related to the target exercise session; and determining, based on the target data set, an estimated load index reflecting a level of exercise load of the target entity for the target exercise session using an artificial neural network (ANN). Here, determining the estimated load index may include: preparing the ANN trained based on a plurality of training data sets including a first training data set, wherein the first training data set includes a sequence of kinematic information of a first entity for a first exercise session and condition information for the first exercise session, and is labeled with a score collected from the first entity for the exercise load of the first exercise session; inputting the target data set to an input layer of the ANN; and obtaining the estimated load index based on a result of an output layer of the ANN.

Here, the condition information may include daily readiness response information that is collected for the target exercise session from the target entity. Here, the daily readiness response information may include at least one of a sleep time evaluation value; a sleep quality evaluation value; a fatigue level evaluation value; a soreness level evaluation value; a stress level evaluation value; or a mood evaluation value.

Here, the condition information may include exercise type information reflecting whether the target exercise session is a match session or a training session.

Here, the condition information may include an evaluation value for importance based on a type of the target exercise session.

Here, the condition information may include information of a length of elapsed time from a previous match session to the target exercise session.

Here, the condition information may include information of a length of elapsed time from an opening day of a sports season including the target exercise session to the target exercise session.

Here, the condition information may include information of physical condition of the target entity.

Here, the information of physical condition may include at least one of age; height; weight; evaluation value for fitness; evaluation value for strength; maximum heart rate; or maximum oxygen consumption.

Here, the condition information may include environmental information reflecting at least one of weather information, temperature information, humidity information, or season information for the target exercise session.

Here, the condition information may include biological measurement information of the target entity for the target exercise session.

Here, the biological measurement information may include at least one of heart rate information; information of a ratio of heart rate to maximum heart rate; body temperature information; oxygen consumption information; or information of a ratio of oxygen consumption to maximum oxygen consumption.

An apparatus for providing exercise load information of a target entity according to an aspect of the present disclosure may include a processor and a memory, wherein the processor may be configured to obtain a target data set of the target entity for a target exercise session including a plurality of time units, wherein the target data set includes a sequence of kinematic information of the target entity for each of the plurality of time units and condition information reflecting a characteristic related to the target exercise session; and determine, based on the target data set, an estimated load index reflecting a level of exercise load of the target entity for the target exercise session using an artificial neural network (ANN).

A non-transitory computer-readable storage medium having processor-executable instructions stored thereon according to an aspect of the present disclosure may be configured such that the instructions are executed by the processor, allowing the processor obtain a target data set of the target entity for a target exercise session including a plurality of time units, wherein the target data set includes a sequence of kinematic information of the target entity for each of the plurality of time units and condition information reflecting a characteristic related to the target exercise session; and determine, based on the target data set, an estimated load index reflecting a level of exercise load of the target entity for the target exercise session using an artificial neural network (ANN).

## Background

In modern sports, a training method that improves performance by accumulating cycles of a fatigue period - a recovery period - an excessive compensation period through repetition of training and relaxation has been widely used. In order to obtain maximum training effects through this training method, it is important to properly adjust exercise load of the training performed during the fatigue period. The exercise load may be divided into external load or internal load. In general, the external load may be evaluated in consideration of the amount of exercise such as total run-distance, etc., and the intensity of exercise such as high load exercise performances, etc. (e.g., high-speed running, high-weight lifting, etc.), and the internal load may be evaluated by the ratio of the exercise effort performed to the maximum exercise capacity of a sports player such as heart rate reserve % (HRR%) or maximum oxygen consumption ($VO_2max$), etc.

The external load are relatively easy to quantify. However, the internal load is difficult to quantify and objectify because 1) a set-up for measuring the heart rate or oxygen consumption is required, 2) it is difficult to express the measured values with a single index, and 3) there is a large error depending on the condition of the subject or environmental conditions. Therefore, despite the advantage of reflecting the characteristics of each individual player, it was generally difficult to utilize internal load in the sports field.

## Terms

Specific technical terms may be used in the present disclosure. Hereinafter, in order to establish definitional support for terms used in the present disclosure, the terms used in the present disclosure will be explained.

The following is a summary of preferable definitions for some terms used in the present disclosure. The definitions below are merely exemplary definitions, and are not comprehensive or limiting.

The term "sports player" refers to a person who performs sports activities such as a match, training or practice, etc., and should be interpreted as a general meaning including all athletes without any limitations in the game of sports (e.g., soccer, basketball, football, baseball, boxing, track-and-field, swimming, etc.) and playing levels (e.g., professional, amateur, adult, youth, etc.).

The term "attachable device" refers to a device which is directly or indirectly attached to a sports player. For example, an attachable device may be provided in the form of a pocket device which is inserted into clothes of sports players or of a band-shaped wearable device which is wrapped around a part of the body of sports players such as wrists or ankles.

The methods, devices, and systems for providing exercise load information according to the embodiments of the present disclosure may provide exercise load information. Here, exercise load information may refer to information reflecting exercise load caused by exercise sessions. Specifically, but non-limitedly, exercise load information may refer to information reflecting exercise load related to the activity performed by a sports player participating in an exercise session, in the corresponding exercise session. As an example, the exercise load information may be expressed in the form of a score or a single index. Additionally, the methods, devices and systems for providing exercise load information may provide scores or indices reflecting the exercise load related to the exercise session of the sports player based on the information of the activity of the sports player performing the exercise session.

## System

Fig. 1 illustrates a system for providing exercise load information according to an embodiment of the present disclosure. Hereinafter, a system 1000 for providing exercise load information according to an embodiment of the present disclosure will be explained with reference to Fig. 1.

As illustrated in Fig. 1, the system 1000 may include a sensing platform 1100, a server 1500, and a terminal 1700.

The system 1000 may provide exercise load information by sensing information of a sports player 10 participating in an exercise session through the sensing platform 1100, calculating the exercise load information from the information sensed through the server 1500, and displaying the exercise load information calculated through the terminal 1700.

Hereinafter, constitutional elements of the system for providing exercise load information according to an embodiment of the present disclosure will be explained.

## Sensing platform

The sensing platform 1100 may sense various information of the sports player 10. For example, the sensing platform 1100 may perform positioning for the sports player 10, detect a movement of the sports player 10, or sense a physical condition of the sports player 10.

As an example, the sensing platform 1100 may sense kinematic information of the sports player 10. Kinematic information is information related to a position or a posture, and a movement of the sports player 10. Kinematic information may include at least one of position information, orientation information and movement information, and movement information may include at least one of velocity, acceleration, jerk, angular velocity, angular acceleration, angular jerk, magnitude thereof (e.g., in the case of velocity, speed), and directions thereof (e.g., in the case of velocity, moving direction).

As an example, the sensing platform 1100 may sense physical condition information of the sports player 10. Physical condition information is information reflecting the physical condition of the sports player 10, and may include at least one of heart rate, body temperature, blood pressure, respiratory rate, and electrocardiogram (ECG). Hereinafter, information sensed by the sensing platform 1100 is referred to as activity information.

The sensing platform 1100 may be provided in various forms in order to sense various aforementioned information. As an example, the sensing platform 1100 may be implemented as a sensor-based platform using an attachable device 1300 attached to the sports player 10, an image-based platform using a camera 1200 arranged in or around a stadium, or a combination thereof.

## The sensor-based platform

Hereinafter, the sensor-based sensing platform will be explained.

The sensor-based sensing platform may include an attachable device 1300. The sensing platform may obtain activity information of the sports player 10 to which the attachable device 1300 is attached using a sensor mounted on the attachable device 1300.

The attachable device 1300 may be attached to the sports player 10, and may be utilized for sensing the activity information of the sports player 10. For example, the attachable device 1300 may be used for positioning the sports player 10 having the attachable device 1300 including a global positioning system (GPS) sensor attached. One or a plurality of attachable devices 1300 may be attached to one sports player 10. Especially, when it is difficult for the sensing platform to obtain all the information to be obtained through the attachable device 1300 with a single attachable device 1300, it may be necessary to attach a plurality of attachable devices 1300 to one sports player 10. For example, one attachable device including a GPS sensor and an inertial measurement unit (IMU) sensor, and another attachable device including a heart rate sensor are attached to the torso and wrist of the sports player 10, respectively. The attachable device attached to the torso may sense the position and movement of the sports player 10, and the attachable device attached to the wrist may sense the heart rate.

As an example, the sensor-based sensing platform may obtain kinematic information using the attachable device 1300.

Hereinafter, some examples in which a sensor-based sensing platform obtains kinematic information will be explained.

The sensing platform may obtain position information using a positioning module of the attachable device 1300.

For example, the attachable device 1300 may include a global positioning module (in other words, a satellite positioning module or a global navigation satellite system (GNSS)), and the sensing platform may measure the position of the sports player 10 by performing global positioning using the same. Specifically, the sensing platform may perform global positioning related to the sports player 10 by the GNSS module receiving a satellite signal from a navigation satellite 20, and calculating a global position (e.g., latitude, longitude) from the received satellite signal through a triangulation technique. Meanwhile, the sensing platform may further include a base station to perform real-time kinematic (RTK) for more accurate positioning. The sensing platform may use the global position as it is as activity information, but may also process the global position to a local position defined by a reference coordinate system for the stadium, and utilize the same as activity information. Here, the reference coordinate system may be a two-dimensional planar coordinate system having the longitudinal direction and width direction of the stadium as axes and one point in or around the stadium (e.g., center or a borderline of the stadium) as the origin point. Meanwhile, all position-related information processed below may be processed according to the reference coordinate system in a non-limited way.

As another example, the attachable device 1300 may include a local positioning module, and the sensing platform may measure the position of the sports player 10 by performing local positioning using a local positioning sensor network including the attachable device 1300. The local positioning sensor network may include a tag node which is attached to an object to be positioned and moves, and an anchor node 30 fixedly installed in the positioning region, and the positioning may be performed using a local positioning system (LPS) signal transmitted and received between the tag node and the anchor node. The sensing platform may include a local positioning module, and may perform local positioning of the sports player 10 using a result of transmitting and receiving the LPS signal between the attachable device 1300 attached to the sports player 10 to be positioned operating as a tag node, and the anchor node 30 fixedly installed in or around the stadium.

The sensing platform may obtain movement information and/or direction information using a motion sensing module of the attachable device 1300.

For example, the attachable device 1300 may include an IMU sensor (or posture azimuth angle (attitude and heading reference system (AHRS) sensor)). Additionally, the sensing platform may obtain movement information and/or direction information of the sports player 10 or a body part of the sports player 10 using acceleration, angular velocity and azimuth sensed by the IMU sensor. When the attachable device 1300 is attached to the torso of the sports player 10, movement information according to the position movement of the sports player 10 may be obtained.

Additionally, when the attachable device 1300 is attached to the arm or leg of the sports player 10, the movement information of the arm or leg of the sports player 10 may be obtained.

Meanwhile, the sensing platform may calculate other kinematic information using the kinematic information measured. For example, the sensing platform may output a speed based on the global position continuously measured by the GPS module and its sampling interval. The calculation of kinematic information is possible through simple mathematical operations including a vector operation or differential and integral calculus with respect to the time axis, and thus detailed explanation thereon will be omitted. Here, the calculation of kinematic information may be performed internally in the attachable device 1300 or may be performed by the server 1500 receiving information from the attached device 1300, and may be performed directly by the sensing module in the attachable device 1300 or may be performed by a controller of the attachable device 1300 based on a sensing result of the sensing module.

As an example, the sensor-based sensing platform may obtain physical condition information using the attachable device 1300. Specifically, the attachable device 1300 may include a bio sensing module, and the sensing platform may sense the physical condition of the sports player 10 using the same. Here, the bio sensing module may include at least one of a heart rate sensor, a body temperature sensor, a blood pressure sensor, a respiratory sensor, and an ECG sensor.

## Attachable device

Fig. 2 is a block diagram of an attachable device according to an embodiment of the present disclosure.

As illustrated in Fig. 2, the attachable device 1300 according to an embodiment of the present disclosure may include a sensing module 1310, a communication module 1320, a controller 1330, a memory 1340, and a battery 1350.

The sensing module 1310 may sense various signals to obtain activity information. The sensing module may be a positioning module, a motion sensing module or a bio sensing module.

The positioning module may be a satellite positioning module 1311 or a local positioning module 1313. The satellite positioning module 1311 may use a navigation satellite system, i.e., GNSS to perform the positioning. Here, the GNSS may include a global positioning system (GPS), GLONASS, BeiDou, Galileo, etc. Specifically, the global positioning module may include a satellite antenna which receives a satellite signal, and a positioning processor which performs positioning using the received satellite signal. For example, the satellite positioning module may be a GPS module including a GPS antenna which receives the GPS signal and a GPS processor which performs positioning using the GPS signal. In this case, the attachable device 1300 may operate as a GPS receiver, and may receive the GPS signal from the satellite to obtain latitude, longitude, altitude, speed, azimuth, dilution of precision (DOP) and time.

The local positioning module 1313 may perform positioning by cooperating with a sensor network. The attachable device 1300 including a local positioning module may operate as a tag node of the local positioning sensor network to transmit and receive LPS signals to and from neighboring anchor nodes, and the positioning may be performed using the result of transmitting and receiving the LPS signals. For example, the attachable device 1300 may transmit LPS signals as a transmitter of the sensor network for local positioning and the anchor nodes may receive the LPS signals, or the anchor nodes may transmit the LPS signals and the attachable device 1300 may receive the LPS signals as a receiver of the sensor network for local positioning. In this case, the LPS signal may be periodically broadcasted according to communication methods such as ultra-wide band (UWB) communication, Bluetooth, Wifi, RFID, etc., and the LPS signal may include a visual or an instrumental identifier. Position calculation may be performed based on a measurement result according to the transmission and reception of the LPS signal, and techniques such as a received signal strength (RSS) technique, a time-of-arrival (ToA) technique, a time difference-of-arrival (TDoA) technique, an angle-of-arrival (AOA) technique, etc., a triangulation technique, a hyperbolic triangulation technique, etc. may be used. Position calculation may be performed by a master of the sensor network, and any one of the tag node and the anchor node or the server may serve as the master of the sensor network. When the sensing platform further includes a RTK base station, the base station may have a function of calculating the position of the master.

The motion sensing module 1315 may be referred to as a kinematic sensing module, and sense the movement and/or posture. Here, the motion sensing module 1315 may include an IMU module or an AHRS module. The IMU module and the AHRS module may include sensors including an accelerometer, a gyroscope, and optionally a magnetometer, and measure the movement and posture from the sensing result of the sensor.

The bio sensing module 1317 may sense physical condition. Specifically, the bio sensing module 1317 may include at least one of the heart rate sensor, body temperature sensor, blood pressure sensor, respiratory sensor, and ECG sensor, and measure information of various physical conditions using these sensors.

Meanwhile, all of the above-mentioned sensor modules do not necessarily have to be provided in one attachable device 1300. A plurality of sensing modules of the same kind may be provided in one attachable device 1300, and different sensing modules may be provided for each attachable device 1300. For example, the sensing platform may include a GPS sensor and an IMU sensor in order to obtain position information and movement information of the sports player 10, and may include two different attachable devices 1300 including one attached to the torso of the sports player 10, and another one including a heart rate meter to obtain the heart rate of the sports player 10 attached to the wrist of the sports player 10.

The attachable device 1300 may transmit and receive data to and from external devices such as the server 1500 or other attachable devices 1300 through a communication module 1320. The communication module 1320 may perform wired communication and/or wireless communication. The attachable device 1300 may transmit and receive data to and from external devices using a mobile communication network (e.g., LTE, 5G, etc.), Wi-Fi, Bluetooth, Zigbee or other wireless communication modules performing various standards of wireless communication. For example, the wireless communication module 1320 may deliver to the server 1500 in real-time the information obtained by the attachable device 1300 using the sensing module 1310 in order for the system to perform monitoring of the sports player 10 using activity information, or may transmit and receive data to and from the attachable devices 1300 when a plurality of attachable devices 1300 are attached to one sports player 10. Additionally, the attachable device 1300 may transmit and receive data to and from the external device using a wired communication module performing universal serial bus (USB) communication or wired LAN communication. For example, the wired communication module may deliver data collected by the attachable device 1300 at once to the server 1500 or docking station, etc. which performs charging and/or data management of the attachable device 1300.

The controller 1330 may control the overall operation of the attachable device 1300. The controller 1330 may be implemented by a hardware configuration, a software configuration or a combination thereof. In terms of hardware, the controller 1330 may be provided in various forms capable of performing calculation or data processing such as an electronic circuit, an integrated circuit (IC), a microchip, and a processor. Additionally, since the physical configuration of the controller 1330 is not necessarily limited as a single physical entity, the controller 1330 may be provided as one processor which handles all kinds of processing of the attachable device 1300 or as a plurality of processors, each of which performs different functions, or in a form combined with a part of other constitutional elements of the attachable device 1300. For example, the controller 1330 may be provided to include a GPS processor which processes the GPS signal to perform positioning, an IMU processor which uses the result sensed by the sensor of the IMU module to perform various calculations, and a main processor which operates the attachable device 1300 and controls the overall operation thereof.

The memory 1340 may store various data related to the operation in the attachable device 1300. For example, the memory 1340 may store the firmware which manages the operation of the attachable device 1300 or the sensing results of sensors of the attachable device 1300. The memory 1340 may be provided as various volatile and non-volatile memories.

The battery 1350 may provide a power source required for driving the operation of the attachable device 1300. The battery 1350 may be provided in an embedded form or a detachable form, and may be charged by receiving power from an external power source.

**Image-based sensing platform**

Hereinafter, the image-based sensing platform will be explained.

The image-based sensing platform may include a camera 1200. The image-based sensing platform may obtain various activity information through image processing and analysis of the images captured by the camera 1200.

The camera 1200 may be arranged to capture the stadium or sports player 10 playing in or around the stadium. The camera 1200 may be semi-permanently installed (e.g., installed in affiliated facilities of the stadium), temporarily installed (installed in a portable pole) or arranged in the form carried by a photographer. A sports image captured by the camera 1200 may be a tactical view, a broadcasting view or a player-focused view. The tactical view is an image generally used to analyze sports tactics and may be an image captured so that most of the sports players 10 are included in the image for team tactic analysis, and the horizontal axis of the tactical view may correspond to the longitudinal direction or width direction of the field. The broadcasting view is an image mainly used for sports broadcasting, and thus it may be generally captured with a smaller angle of view than the tactical view. The player-focused view is an image captured along a specific sports player 10, and thus it may be mainly used to analyze individual capabilities of the sports player 10. Also, the camera 1200 may not necessarily have a fixed field of view, and viewing direction adjustment and zoom adjustment may be performed manually or automatically.

The image-based sensing platform may include one or a plurality of cameras. The plurality of cameras may be provided in the form of a multi-camera capable of capturing a panorama view at a single spot, or in a form dispersedly arranged at multi spots, or in a form in which the two are combined.

The sensing platform may analyze the image captured through the camera 1200 to obtain activity information therefrom. For example, the sensing platform may obtain position information of the sports player 10 by performing object recognition for the sports player 10 on the image, extracting a position (e.g., pixel coordinates) in the image of the sports player 10, and projecting the position in the image on the ground using the posture information of the camera 1200. Here, a deep learning algorithm may be used for image processing such as object detection. Specifically, for object detection (e.g., detection of sports player 10), a deep learning algorithm for detecting various objects from region based convolutional neural network (R-CNN) to you-only-look-once (YOLO) may be used. Additionally, for coordinate conversion, for example, top-view conversion using a camera parameter (e.g., installation position, imaging posture, etc.) may be used. For example, the server 1500 may obtain a sports image from the camera 1200 arranged to capture a tactical view, obtain a bounding box for sports objects such as a ball or a sports player 10 in the sports image through an artificial neural network model which performs object detection, determine a representative pixel (e.g., a lower center of the bounding box) for the sports object in consideration of the bounding box, and perform top-view conversion using a coordinate conversion metric between the pixel coordinate system and the reference coordinate system within the sports image generated in consideration of the camera parameter for the determined representative pixel to obtain position data for the sports object. Here, object detection using artificial neural networks may be replaced by object segmentation. Accordingly, the server 1500 may obtain position data for the sports object in the sports image captured by the camera 1200 through image analysis.

The sensor-based sensing platform and the image-based sensing platform have been explained in the above. As mentioned above, in the present disclosure, the sensing platform 1100 may be implemented in a sensor-based form, an image-based form, or a combined form thereof. In other words, the sensing platform 1100 may include at least one of an attachable device 1300 and a camera 1200. When the sensing platform 1100 operates the attachable device 1300 and the camera 1200 at the same time, that is, when the sensing platform 1100 is implemented in a form in which a sensor-based platform and an image-based platform are mixed, activity information may be obtained using the information obtained through the attachable device 1300 and the information obtained through the image analysis at the same time. For example, the sensing platform 1100 may perform a more accurate positioning by comparing a position value obtained as a result of performing global positioning using the attachable device 1300 with a position value obtained through sports image analysis.

As mentioned above, the sensing platform may optionally further include a base station for RTK correction, and an anchor node for constructing a local positioning sensor network as needed.

In addition, some of the various calculations performed in the sensing platform 1100 may be processed by a processor or a controller mounted on the attachable device 1300, while others may be processed by the server 1500. For example, object detection for the image may be processed by the server 1500, position calculation using the result of transmitting and receiving the LPS signal between each node in the local positioning sensor network may be processed by the server 1500, and various processing related with image analysis may be processed by the server 1500. Therefore, in this case, it may be understood that the server 1500 is included in the sensing platform 1100.

**Server**

The server 1500 may receive activity information from the sensing platform 1100 or cooperate with the sensing platform 1100 to obtain activity information, and may obtain exercise load information using the same.

The server 1500 may be provided as a local server located in the vicinity of the stadium and/or a web server connected through a web. Additionally, the server 1500 does not necessarily have to be implemented as a single subject. For example, the web server may be implemented by including a main server which processes the calculation and a data server which stores various data. Meanwhile, the local server may be provided as a device independently operated to perform only the original function of the server, but also may be provided as a device having a complex function combined with other constitutional elements of the system providing exercise load information. For example, the server may be provided in the form of a docking station, which is a container for accommodating, storing and managing the attachable device 1300. Here, the docking station may have an internal space in which a plurality of attachable devices 1300 are accommodated, including a docking part in which the attachable device 1300 is accommodated, and may store the attachable device 1300 when the attachable device 1300 is not used. Furthermore, the docking station may provide various convenient functions required for using the attachable device 1300 other than simply storing the attachable device 1300. For example, the docking station may perform functions such as charging attachable device 1300, displaying battery condition, updating firmware, collecting data, etc.

Fig. 3 is a block diagram of a server according to an embodiment of the present disclosure.

The server 1500 may include a communication module 1510, a controller 1520 and a memory 1530.

The communication module 1510 may perform data transmission and reception between the server 1500 and other constitutional elements the system for providing exercise load information or an external device. For example, the server 1500 may collect data from the attachable device 1300 through the communication module 1510, receive an image from the camera 1200, or deliver various information to the terminal 1700 through the web.

The controller 1520 may control the overall operation of the server 1500. Like the controller of the attachable device 1300, the controller 1520 of the server may be implemented as a hardware configuration, a software configuration or a combination thereof. From a hardware point of view, the controller may be provided in various forms capable of performing calculation or data processing such as an electronic circuit, an integrated circuit (IC), a microchip, and a processor, and a physical configuration thereof is not necessarily limited to a single physical entity. Meanwhile, specific operations performed by the server 1500 will be mentioned below, and it may be construed that methods or operations according to the embodiments of the present disclosure which will be mentioned below are performed by the controller 1520 in one server, unless mentioned otherwise.

However, as mentioned above, the embodiments of the present disclosure are not necessarily performed by the controller 1520 of the server. It should be construed that the embodiments of the present disclosure may be performed by a processor capable of calculating such as the controller 1330 of the attachable device 1300, or at least some procedures may be performed by the server, or at least some procedures may be performed by another processor.

## Terminal

The terminal (or terminal device) 1700 may function as a user interface which provides various data or information collected or calculated by the system to the user or receives a user input from the user. For example, the terminal 1700 may receive a user input indicating a specific exercise session whose exercise load information is to be known from the user, request and receive exercise load information for a corresponding exercise session to/from the server 1500, and display the same to provide the exercise load information to the user. The terminal 1700 may be an electronic device having a smart device such as a smart phone or a tablet, a personal computer such as a laptop or a desktop computer, or an input interface which receives other user inputs, and an output interface such as a display.

## Method for providing exercise load information

Hereinafter, a method for providing exercise load information according to an embodiment of the present disclosure will be explained. In the following description, it is explained that the method for providing exercise load information is performed by the aforementioned system for providing exercise load information, but this is only for the sake of convenience in explanation, and the method is not limited by the system.

### *Exercise load information*

In the present disclosure, exercise load information means information reflecting the exercise load of a specific exercise session, and for example, may be expressed as an exercise load index which indicates the exercise or a level of the exercise load with a score. As a non-limited example, the exercise load information may mean information reflecting an exercise load related to an activity performed by the sports player(s) participating in the exercise session in the corresponding exercise session. Exercise load may be divided into external load which expresses an exercise physically performed with an objective physical quantity, and internal load which considers the degree of effort subjectively felt by the subject performing the exercise. The rating of perceived exertion (RPE) (hereinafter, referred to as "perceived fatigue rating") is a representative example of internal load. In order to precisely measure internal load, it is necessary to consider how hard the body of the exercise subject feels about the exercise based on the physical sense and feeling of the subject performing the exercise. In order to quantify the degree that the body feels about the exercise, that is, the internal load, there have been attempts to measure the degree of increase in heart rate, the degree of increase in respiratory rate, the degree of increase in body temperature, the degree of accumulation of muscle fatigue or the degree of pain generation, etc. and index some of them or all of them individually or collectively. However, since the internal load may be affected by very complicated factors acting on the human body, it cannot be calculated accurately with the aforementioned information alone. Additionally, since it is practically impossible to measure the aforementioned information from the exercise subject for every exercise session, a method of identifying the internal load for the exercise through surveys about the intensity felt by the exercise subject after the exercise session, that is, the extent to which the exerciser perceives the exercise intensity is generally used.

In the present disclosure, the exercise load information provided by the system should be understood as a general concept including information meaning the level of external exercise load, information meaning the level of internal exercise load or information meaning the level of exercise load combined with the internal/external load. As will be mentioned later, the exercise load information in the present system may be viewed as a concept close to reflecting the internal load in terms of being calculated in consideration of the RPE felt by the subject performing the exercise. However, since the exercise load information is calculated by comprehensively using the RPE obtained from a plurality of exercise subjects, the objectivity thereof is guaranteed, and considering that the exercise load information is calculated based on physical quantities such as velocity and acceleration according to the movement performed by the exercise subject during the exercise session, it appears that there is an aspect reflecting the external load. Therefore, in the present disclosure, exercise load information can be interpreted as information reflecting the internal load for the exercise, but depending on the point of view, it can be interpreted as information independently or additionally reflecting the external load. Accordingly, it should be noted in advance that the exercise load reflected by the exercise load information of the present disclosure should not be interpreted limitedly as an internal load and/or an external load.

Fig. 4 illustrates an example of an exercise load index according to an embodiment of the present disclosure.

The exercise load index may be expressed as an exercise load index indicating a level of exercise load with a score. Here, the expression form of the exercise load index may be the same as or similar to the RPE. Specifically, the exercise load index may be an index expressed as a specific score within a predetermined score range indicating the intensity of exercise or the magnitude of the exercise load for each score. For example, the exercise load index may be expressed as RPE of a borg scale or a borg-CR10 scale. Here, the RPE of the borg scale is a scale expressing a condition from no exercise load to an extreme exercise load on a scale of 6 to 20 points, and the RPE of the borg-CR10 scale is a scale expressing a condition from no load to maximum load on a scale of 1 to 10 points. Meanwhile, in general, when the RPE value of borg scale is multiplied by 10, the heart rate of the exercise subject according to the exercise expressed in the borg scale may be roughly predicted. The exercise load index provided by the system of the present disclosure may express the exercise load through a score in a predetermined range that is the same as or similar to the RPE of the above-mentioned borg scale or borg-CR10 scale. However, in the present disclosure, exercise load index does not necessarily have to be expressed as above, and may be expressed in various index forms such as percentile (for example, percentile of the sports player's maximum physical strength or maximum exercise effort capacity versus the exercise effort performed), physical quantity (for example, calories burned according to exercise), or letter grade such as grades.

In the present disclosure, exercise load index may include an estimated load index or a response load index. The "estimated load index" may be an exercise load index determined using an artificial neural network from a data set according to the embodiments of the present disclosure. The "response load index" may be an exercise load index received from a target entity to be measured after the end of a target session for which an exercise load is to be measured.

Hereinafter, embodiments of the method for providing exercise load information according to the present disclosure will be explained in detail. However, it should be noted that the scope of technical idea of the present disclosure is not limited to the following examples, and the following examples are only for illustratively explaining at least a part of the technical idea of the present description.

## Determining exercise load based on kinematic information

Fig. 5 is a schematic flow chart illustrating a method for providing exercise load information according to an embodiment of the present disclosure. Fig. 6 is a detailed flow chart illustrating a step of determining an estimated load index. Hereinafter, the method for providing exercise load information according to an embodiment of the present disclosure will be explained in detail with reference to Figs. 5 and 6.

The method and/or processes according to an embodiment of the present disclosure may be performed by a computing device. According to an aspect, the computing device may be the server 1500 or the controller 1520 included in the server 1500 as explained with reference to Figs. 1 to 3, but is not limited thereto. Any computing device including a processor and a memory may be used, and a combination of a plurality of physically separated devices may be referred to as a computing device.

According to the method for providing exercise load information according to an embodiment of the present disclosure, an estimated load index may be determined based on kinematic information of a target entity during a target exercise session for which the load information is to be provided, and provided as exercise load information. Accordingly, information of the level of exercise load of the target entity during the target exercise session may be easily collected and managed without directly asking a question to the target entity.

In the present disclosure, the term "target entity" may mean a subject for which exercise load information is to be determined. For example, the target entity may be one sports player, and information as to how much exercise load an individual player felt during the exercise session may be provided. Or, the target entity may be one sports team including a plurality of sports players, and information as to how much exercise load the corresponding team as a whole felt during the exercise session may be provided.

Additionally, in the present disclosure, the term "target exercise session" may mean an exercise session, which is a subject for which exercise load information is to be provided. The exercise session may be a section having a predetermined time length, and may include a plurality of time units. At least some of the basic data which become the subject for which exercise load information is to be determined may be determined for each of the plurality of time units. Therefore, at least some of the basic data may include a sequence of predetermined information. A set of the basic data which becomes the subject for which exercise load information is to be determined may be referred to as a "target data set" in the present disclosure.

When referring to Fig. 5, the method for providing exercise load information according to an embodiment of the present disclosure may include at least one of measuring position information of a target entity S510, obtaining a target data set S520, determining an estimated load index S530, or determining a representative load index S540.

Hereinafter, each step of an embodiment of the method for providing exercise load information will be explained.

As illustrated in Fig. 5, the computing device may measure position information of the target entity S510. For example, the computing device may use a positioning device corresponding to the target entity to measure position information of the target entity for the target exercise session. Here, the positioning device may mean at least a part of the sensing platform 1100 as exemplarily explained above. Therefore, the computing device may use, for example, a sensing platform using one or more of an attachable device 1300 and/or a camera 1200 attached to a sports player to measure position information of the target entity at a predetermined time interval during the target exercise session. Accordingly, a sequence of the position information of a target entity during the target exercise session may be obtained.

The sequence of the position information may include, for example, position information of the target entity at a first time point, position information of the target entity at a second time point, and position information of the target entity at an $n^{th}$ time point.

When referring to Fig. 5 again, the computing device may obtain a target data set for a target exercise session S520. More specifically, the computing device may obtain a target data set for a target entity during a target exercise session including a plurality of time units. Here, the target data set may include a sequence of kinematic information of the target entity for each of the plurality of time units. For example, the kinematic information may be generated based on the position information of the target entity.

According to an aspect, the target exercise session may include a plurality of time units having a predetermined time interval, and kinematic information of the target entity may be determined for each of the time units. Here, the position information for one time point does not necessarily have to correspond to each time unit for which the kinematic information is determined. For example, kinematic information of the target entity for a first time unit may be determined based on the position information of the target entity at a plurality of time points corresponding to the first time unit, and kinematic information of the target entity for a second time unit may be determined based on the position information of the target entity at a plurality of other time points corresponding to the second time unit.

The kinematic information, as mentioned above in the present disclosure, may indicate information of at least one of the position or posture and movement of the target entity, and may include at least one of position information, direction information and movement information. The movement information will be described in detail later in the present disclosure.

The sequence of the kinematic information included in the target data set may include, for example, kinematic information of the target entity for the first time unit, kinematic information of the target entity for the second time unit, and kinematic information of the target entity for the $n^{th}$ time unit. The conventional measurement of the external load is based on statistical values for the entire exercise session such as the total movement amount or exercise time, and average velocity during the exercise session, whereas the provision of exercise load information according to an embodiment of the present disclosure determines an evaluation value for a level of exercise load based on the sequence of kinematic information for a plurality of time points and/or time units included in the target exercise session, and thus may be distinguished from the conventional measurement.

When referring to Fig. 5 again, the computing device may determine an estimated load index S530. More specifically, the computing device may determine, from the target data set, an estimated load index reflecting the level of the exercise load of the target entity for the target exercise session using the ANN. According to an aspect, the determined estimated load index may be provided as an estimate of the exercise load for the target exercise session. Here, the estimated load index may include, for example, a plurality of numerical values indicating a level of exercise load. For example, the estimated load index may be configured to have a value from 1 to 10 where 10 indicates the highest exercise intensity, but is not limited to such specific numerical range.

According to an embodiment of the present disclosure, it is possible to conveniently secure and provide information of the level of exercise load of the target entity during the target exercise session without going through a passive question and answer procedure for the target entity by automatically determining the estimated load index using the ANN based on the target data sets measured and/or calculated for the target entity.

The estimated load index according to an aspect of the present disclosure may be an estimate of RPE of the target entity for the target exercise session. For example, as exemplarily illustrated in Fig. 4, the estimated load index determined according to the present disclosure may be provided instead of the RPE.

In this regard, Fig. 6 is a detailed flow chart illustrating a step of determining an estimated load index in Fig. 5. As illustrated in Fig. 6, the computing device may first prepare a trained ANN S531, input a target data set to an input layer of the ANN S533, and obtain an estimated load index based on a result of an output layer of the ANN S535. Hereinafter, with reference to Fig. 6, a process for determining the estimated load index will be explained in detail.

As illustrated in Fig. 6, the computing device may prepare the ANN trained based on a plurality of training data sets including a first training data set S531. Specifically, for example, an ANN model may be stored or loaded in a memory of a server so that the controller may be in a state in which the ANN model may be operated.

Fig. 7 illustrates an exemplary artificial neural network (ANN) used for calculating the exercise load index according to an embodiment of the present disclosure.

The ANN may include an input layer, a hidden layer and an output layer. The input layer may include a plurality of nodes and may receive an input data set. The output layer may include at least one node, and may be provided, for example, in a form having a single node which outputs an output value that can be interpreted as a value of the exercise load index by a numerical value, or in a form having multiple nodes in which values of different exercise load indices are assigned, and output values thereof are probability values. The hidden layer may include a plurality of layers which connect the input layer and the output layer, and have at least one node. The connection between the nodes may be defined by parameters such as weight or bias. Through this, information is delivered from the input layer through the hidden layer to the output layer, so that an output value can be calculated from the input data set.

The learning of the ANN may be performed using a learning data set and an exercise load index labeled therein. Specifically, the learning may be performed by adjusting parameters of the ANN in a way to reduce errors based on the error between a result value output by the ANN receiving the learning data set, and a ground truth of the exercise load index labeled in the learning data set.

In an embodiment of the present disclosure, the exercise load index may be learned using the learning data set labeled with the exercise load index so that the exercise load index of the target exercise session may be output upon receiving activity information of the target entity for the target exercise session. Basically, the learning data set may be generated from the activity information related to the target entity performing the exercise session, and the labeled exercise load index may be a value collected from the target entity performing the exercise session.

More specifically, but non-limitedly, the ANN according to an embodiment of the present disclosure may be trained based on a plurality of training data sets including a first training data set. Here, the first training data set may include a sequence of kinematic information of the first entity for the first exercise session, and may be labeled with a score collected from the first entity for an exercise load of the first exercise session.

In this regard, Fig. 8 illustrates an example of a training data set of the ANN according to an embodiment of the present disclosure, and Fig. 9 is a detailed view of an example of a sequence of kinematic information in Fig. 8.

As illustrated in Fig. 8, a plurality of training data sets 800 for training the ANN according to an aspect of the present disclosure may include a first training data set 810. The first training data set 810 may include a sequence 811 of kinematic information of a first entity for each of the plurality of time units included in a first exercise session, and may be labeled with a score 813 collected for the exercise load of the first exercise session from the first entity. In other words, for example, when a sports player A participates in a specific exercise session A, a sequence of kinematic information for each of time units for the sports player A may be collected and/or measured. Here, after the corresponding exercise session A ends, for example, an evaluation value of the exercise load may be answered by asking a question to the sports player A as a measurement of the RPE. Here, one training data set may be secured by labeling the sequence of kinematic information of sports player A with the answered RPE value (e.g., 8).

As illustrated in Fig. 9, the sequence 811 of kinematic information of the first entity may include kinematic information (e.g., velocity, acceleration, jerk, angular velocity, angular acceleration, and angular jerk) of the first entity measured for a plurality of time units included in the first exercise session, for example, for each of the time units from the first time unit 811a to the $n^{th}$ time unit 811n.

When the target data set is inputted by learning the ANN based on a plurality of training data sets as illustrated in Figs. 8 and 9, a corresponding evaluation value of the exercise load may be outputted.

More specifically, when referring to Fig. 6 again, the computing device may input the target data set to the input layer of the ANN S533. Here, the target data set, as mentioned above, may include a sequence of kinematic information of the target entity for a plurality of time units during the target exercise session.

Meanwhile, according to an aspect of the present disclosure, zero padding may be performed for the input data set (e.g., the target data set) so as to correspond to the number of nodes in the input layer of the ANN. For example, the target data set may be configured to have a predetermined length by performing zero-padding preceding or following the sequence of the kinematic information. In this regard, Fig. 10 illustrates an example of an input data set of the ANN according to an embodiment of the present disclosure, and Fig. 11 illustrates another example of the input data set of the ANN according to an embodiment of the present disclosure. Fig. 10 illustrates performing zero-padding preceding the sequence, and Fig. 11 illustrates performing zero-padding following the sequence.

In other words, the ANN according to the embodiments of the present disclosure may be configured to receive data of a fixed form or length as an input, and may perform zero-padding on a feature factor sequence so as to adjust the input data set to a predetermined length. In this regard, considering that matches or trainings generally last for about 2 hours, the maximum length may be set to, for example, 2,000, and for a target data set with a shorter length, a plurality of '0' may be appended to fill the front or rear of the sequence of kinematic information.

Padding '0' at the end of the sequence may correspond to a situation in which the target entity recognizes the RPE score after taking a rest as long as the zero-padded period. That is, the shorter the original sequence of the model, it can be wrongly reflected that the rest taken between the end of the activity and the examination of the RPE is long. Accordingly, more preferably, in the present disclosure, it may be configured to perform zero-padding preceding the sequence, not following the sequence.

The ANN outputs the result value to the output layer in response to the input of the target data set. The computing device may obtain an estimated load index based on the result value of the output layer of the ANN S535. According to an aspect, the estimated load index may be provided as information indicating the level of exercise load of the target entity for the target exercise session.

As such, the conventional manual RPE collection process may be easily replaced by determining an estimated load index using the ANN based on the target data set collected for the target entity and providing the same as exercise load information. Therefore, the RPE errors (e.g., change in time of collecting RPE after training or occurrence of unsubmitted players) resulting from the collection according to the conventional questioning-answering may be improved, and it is possible to manage the exercise load information for a period having a predetermined continuity such as a daily basis, a weekly basis and a monthly basis. Therefore, the training session can be set more efficiently, and performance improvement can be expected. Additionally, in the past, it was possible to actually manage the exercise load information in a team unit. However, according to the embodiments of the present disclosure, it is possible to manage the exercise load at a level of each individual player, so that conditioning can be performed for each individual player.

Meanwhile, according to an aspect of the present disclosure, it is also possible to obtain and provide exercise load information for a sports team. According to an aspect, a target entity for which the exercise load information is to be determined may be at least one sports team.

In this case, when referring to Fig. 5 again, the computing device may determine a representative load index S540. More specifically, the computing device may determine the representative load index of the sports team during the target exercise session based on the estimated load indices for each of the at least some sports players belonging to the sports team S540. For example, when the target entity is sports team A, the estimated load index for each sports player may be determined by measuring the position information of sports players A, B and C belonging to the sports team S510, obtaining a target data set S520, and determining an estimated load index S530. Afterwards, the representative load index for the sports team A may be determined using the estimated load indices of each sports player A, B, and C. The representative load index may be, for example, an average of the estimated load indices for each sports player, but is not limited thereto, and a representative value indicating the trend of the entire team may be determined according to various rules.

As mentioned above, according to an aspect of the present disclosure, the estimated load index reflecting the estimate for the level of exercise load for the target entity may be determined based on, for example, kinematic information of the target entity.

As mentioned above in the present disclosure, for example, exercise load information such as an estimated load index may be an estimate for the RPE. The RPE may reflect the difficulty of the exercise. Therefore, for example, at least one of various kinematic information may be used to determine exercise load information such as the estimated load index.

For example, in order to determine the difficulty of the exercise, at least one of the following may be considered: i) whether it consumes a lot of energy, ii) whether it requires a lot of strength, or iii) whether it causes instantaneous sudden change.

With regard to the energy consumption, kinetic energy is defined as below.

$$KE = 1/2\ mv^2$$

In other words, for example, velocity v of the target entity may be included in kinematic information as a factor for evaluating the energy consumption.

With regard to the strength required, Newton's second law is defined as below.

$$F=ma$$

In other words, acceleration a of the target entity may be included in kinematic information as a factor for evaluating the strength required.

Meanwhile, the muscle fatigue may be affected mainly by the jerk. Accordingly, the jerk of the target entity may be included in kinematic information as a factor for evaluating the fatigue resulting from an instantaneous sudden change.

In other words, at least one of velocity, acceleration, or jerk of the target entity may be included in kinematic information.

Unlike the conventional method for determining the external load evaluated by fragmentary numerical values such as the total movement distance or average velocity according to statistical results, the method for providing exercise load information according to an embodiment of the present disclosure may estimate the evaluation value for the exercise load with more improved accuracy by inputting the sequence of kinematic information (e.g., at least one of velocity, acceleration, or jerk) for each of a plurality of time units included in the target exercise session for which the exercise load information is to be determined to the ANN.

Meanwhile, even for the same magnitude of kinematic element (for example, the same displacement, velocity, acceleration, or jerk), the exercise load applied to the target entity may be different depending on the direction of the kinematic element. For example, even when moving the same distance at the same speed, the exercise load applied to the target entity may be different in the cases where the target entity moves toward the front of the target entity, where the target entity moves backward in a direction opposite to the orientation direction of the target entity, or where the target entity moves laterally with respect to the orientation direction of the target entity. As an example, the target entity may experience greater exercise load when the target entity moves backward in a direction opposite to the orientation direction of the target entity, than when advancing toward the orientation direction with the same displacement or velocity. Therefore, according to an aspect of the present disclosure, the relation of direction of the kinematic element to the orientation direction of the target entity may be considered when determining the exercise load information.

In this regard, the kinematic information according to the embodiment of the present disclosure may be expressed based on any one of a plurality of coordinate systems. For example, the kinematic information according to an aspect may be expressed based on a field-based coordinate system, or an entity-based coordinate system, but is not limited thereto.

In this regard, Fig. 12 exemplarily illustrates an entity-based coordinate system. As illustrated in Fig. 12, the entity-based coordinate system may be a coordinate system set based on the orientation direction of the target entity such as the sports player. For example, the entity-based coordinate system may include a forward-backward axis 121 corresponding to a heading direction of the target entity and a left-right axis 123 corresponding to a side direction of the target entity. When the target entity moves forward, for example, when a value for the displacement is expressed based on the entity-based coordinate system, a positive value in the direction of the forward-backward axis 121 may be obtained. When the target entity moves backward in a direction opposite to the orientation direction, the displacement value thereof may have a negative value in the direction of the forward-backward axis 121. Meanwhile, the displacement occurring to the left side or right side with respect to the orientation direction of the entity may have a negative value or a positive value, respectively, in the direction of the left-right axis 123. Equally for the velocity, acceleration, or jerk which can be calculated based on the displacement, the moving direction for the orientation direction may be expressed with the value of kinematic information alone.

For example, Fig. 12 illustrates a first movement m1 and a second movement m2. The m1 may be a forward movement for the orientation direction of the entity, and m2 may be a side step movement facing the left side based on the orientation direction of the entity. Here, the m1 may be expressed as a displacement having a positive value in the direction of the forward-backward axis 121, and for example, a movement vector may be expressed as (0, 10). Additionally, the m2 may be expressed as a displacement having a positive value in the direction of the left-right axis 123, and for example, the movement vector may be expressed as (10, 0).

In other words, the kinematic information expressed based on the entity-based coordinate system may include, for example, information of a relative movement direction with respect to the orientation direction of the entity when the information involves direction and magnitude such as displacement, velocity, acceleration, or jerk. Therefore, the kinematic information according to an aspect of the present disclosure may include at least one of first kinematic information related to velocity of the target entity, second kinematic information related to acceleration of the target entity, or third kinematic information related to jerk of the target entity. Even when the kinematic information only includes information of linear movement such as velocity, acceleration, or jerk, when the information of the linear movement is expressed based on the entity-based coordinate system, a difference in the level of exercise load according to the direction of the movement (for example, forward, backward, or side movement) with respect to the orientation direction of the entity may be reflected.

In order for the kinematic information to be expressed based on the entity-based coordinate system, information of the orientation direction of the entity is required. As mentioned in the present disclosure, the sensing platform according to an aspect may include, for example, an attachable device, and the attachable device may have an inertial sensor such as an IMU sensor. According to an aspect of the present disclosure, the kinematic information may be obtained based on the information measured by an inertial sensor corresponding to the target entity and/or the information measured by a position measuring device such as a GPS sensor.

In comparison, according to an aspect of the present disclosure, the kinematic information may be obtained by using the position information of the target entity measured using a position measuring device such as a GPS sensor. For example, in consideration of the economic aspect or in consideration of various circumstances such as at least one of improvement in fit, weight reduction, and reduction in computational amount, the sensing platform may not be provided with an inertial sensor, but only be provided with a position measuring device such as a GPS. In this case, since the information of the orientation of the target entity cannot be obtained, kinematic information may be expressed based on the field-based coordinate system, not by the entity-based coordinate system.

Here, the field-based coordinate system may express the local position as mentioned above in the present disclosure. The local position may be obtained, for example, by converting the coordinate according to a global position measuring device such as a GPS through calculation, or may be directly obtained through a local positioning module, but is not limited thereto.

More specifically, Fig. 13 exemplarily illustrates a field-based coordinate system. As illustrated in Fig. 13, the field-based coordinate system may include a first axis 133 for a length of the field in which the exercise session is performed and a second axis 131 for a width of the field in which the exercise session is performed.

The kinematic information of the linear movement expressed according to the field-based coordinate system does not reflect the relation between the orientation direction and movement direction of the target entity. For example, as illustrated in Fig. 12, the m1 may be a forward movement for the orientation direction of the entity, and the m2 may be a side step movement facing the left side with respect to the orientation direction of the entity. However, as illustrated in Fig. 13, when the same m1 and m2 are expressed through the field-based coordinate system, they are only expressed as movements toward different predetermined directions, and the information of the direction of the movement (for example, forward, backward, or lateral movement) with respect to the orientation direction of the entity cannot be expressed. Accordingly, for example, although the exercise loads for the forward movement and for the side step can actually be different, said movements can be incorrectly reflected to have the same level of exercise load when determining the estimated load index.

In this regard, according to an aspect of the present disclosure, the estimated load index may be determined by reflecting the relation between the orientation direction and the movement direction of the target entity based on a difference between the movement direction at a previous time point and the movement direction at a present time point. For example, when the target exercise session includes a plurality of time units, in addition to the information of the linear movement in the present time unit, the difference between the direction of information of the linear movement in the previous time unit and the direction of information of the linear movement in the present time unit is further reflected to determine the estimated load index. In other words, for example, the kinematic information for the second time unit may include information of the linear movement of the target entity in the second time unit (for example, information related to velocity, acceleration, jerk or magnitude thereof), and information of a difference between at least one direction of the information of the linear movement for the target entity in the first time unit and at least one direction of the information of the linear movement for the target entity in the second time unit. Therefore, for example, when the kinematic information is expressed through the field-based coordinate system, the estimated load index may be determined by reflecting the relation between the orientation direction and the movement direction of the target entity.

Fig. 14 illustrates a difference between a movement direction at a previous time point and a movement direction at a present time point. More specifically with reference to Fig. 14, the movement direction 141 at the previous time point may be assumed as the orientation direction of the target entity at the present time point. Since a case that the movement direction of the target entity during the exercise session is most often observed to be forward, assuming that the target entity moves forward at the previous time point, it may be assumed that the movement direction at the previous time point (for example, direction of velocity at the previous time point) is the same as the orientation direction of the target entity at the present time point. Here, considering the movement direction 143 at the present time point as illustrated in Fig. 14, an angular change 145 between the movement direction 143 (for example, direction of velocity at the present time) at the present point and the movement direction 141 at the previous time may be utilized as a factor reflecting the relation between the orientation direction of the target entity at the present time point and the movement direction of the target entity at the present time point. Accordingly, according to an aspect of the present disclosure, the kinematic information for the target time unit, which is any one of the plurality of time units included in the target exercise session, may include information of at least one magnitude of the linear movement of the target entity in the target time unit, and information of an angular change between at least one direction of the linear movement of the target entity in the target time unit and at least one direction of the linear movement of the target entity in the previous time unit of the target time unit. For example, as exemplarily illustrated in Fig. 14, the kinematic information for the target time unit may include information of magnitude of velocity (speed) 147 of the target time unit, and information of an angular change 145 between the direction of velocity 141 in the previous time unit of the target time unit and the direction of velocity 143 in the target time unit.

According to an aspect, the kinematic information for the target time unit may be configured to include at least one of the speed for the target time unit, the change in the movement direction from the previous time unit, the magnitude of acceleration, the change in the direction of acceleration from the previous time unit, the magnitude of jerk, the directional change of jerk from the previous time unit. Fig. 15 illustrates an example of kinematic information according to an embodiment of the present disclosure. As illustrated in Fig. 15, the kinematic information may be obtained by calculation based on the position information of the target entity measured based on the positioning sensor 15. The kinematic information obtained may include at least one of the horizontal speed 151 of the target entity, the change of horizontal moving direction 152, the magnitude of horizontal acceleration 153, the directional change of horizontal acceleration 154, the magnitude horizontal jerk 155, or the directional change of horizontal jerk 156. The kinematic information may be obtained for each of the plurality of time units included in the target exercise session, thereby forming the sequence 811 of the kinematic information included in the target data set.

Hereinafter, more various examples of the kinematic information according to the embodiments of the present disclosure are explained.

Fig. 16 illustrates an example of the kinematic information according to an embodiment of the present disclosure. As illustrated in Fig. 16, the kinematic information 1600 may include at least one of position/orientation information 1610, linear movement information 1620, and angular movement information 1630.

In this regard, Fig. 17 illustrates an example of coordinate systems capable of expressing the kinematic information according to an embodiment of the present disclosure. As illustrated in Fig. 17, the kinematic information according to an aspect may be expressed, for example, by any one of a global coordinate system 171, a local coordinate system 173, an inertial coordinate system 175, and a hybrid coordinate system 177.

The global coordinate system 171 may include, for example, latitude, longitude and altitude elements measured using a global positioning module such as a GPS. The local coordinate system 173 may include, for example, the field-based coordinate system mentioned above in the present disclosure. The local coordinate system may include an axis in the longitudinal direction of the field in which the exercise session is performed, and an axis in the width direction of the field, and may further include an axis in the vertical direction from the ground of the field according to an aspect. Additionally, according to an aspect, for example, the local coordinate system may have a specific point in or around the field such as a center or a borderline of the field as the origin point.

The inertial coordinate system 175 may be, for example, an aviation coordinate system. The inertial coordinate system may include forward-backward movement, slide movement, up-down movement elements of a player body. Additionally, the inertial coordinate system may include rotational movement elements of a roll axis, a pitch axis, and a yaw axis related to the rotation of the player body.

Meanwhile, the hybrid coordinate system 177 may be, for example, a maritime coordinate system. The hybrid coordinate system is fixed in the yaw axis direction, and may include horizontal heading elements, horizontal lateral elements, and vertical elements.

The coordinate system for expressing the kinematic information according to an aspect of the present disclosure is not limited to the above-mentioned coordinate systems, but the kinematic information may be expressed based on any one of various coordinate systems.

When referring to Fig. 16 again, the kinematic information 1600 may include position/orientation information 1610. The position/orientation information may not affect the exercise load as it is. However, the position/orientation information may be data for calculating various kinematic information. The position/orientation information may be indicated as static information. Fig. 18 illustrates an example of static information. The static information 180 may include position information 181. For example, the position information 181 may include latitude, longitude, and altitude, or length, width, and height. Meanwhile, the position information 181 may include orientation information 183. The orientation information 183 may include, for example, orientation in the longitudinal direction, orientation in the width direction, and orientation in the height direction. The static information may be obtained for a plurality of time points included in the target session or each of the time units to form the sequence. The additional kinematic information (for example, linear movement information or angular movement information) may be obtained based on the sequence of the static information.

When referring to Fig. 16 in this regard, the kinematic information 1600 may include linear movement information 1620. The linear movement information may be expressed based on the field-based coordinate system, entity-based coordinate system, or optional coordinate system as reviewed above.

More specifically, the linear movement information may include first kinematic information 1621 related to velocity of the target entity. According to an aspect of the present disclosure, the first kinematic information may include at least one of information of the magnitude of velocity in the target time unit, or information of angular change between the direction of velocity in the previous time unit of the target time unit and the direction of velocity in the target time unit.

Fig. 19 illustrates an example of first kinematic information including information of velocity.

As exemplarily explained above, when the kinematic information is expressed based on the entity-based coordinate system, even if the estimated load index is determined using only velocity information, a difference in the level of exercise load resulting from the difference between the orientation and movement direction of the entity may be reflected. Therefore, the first kinematic information may include velocity information v1, v2, v3 of the target entity. The velocity information may be expressed based on various coordinate systems. For example, at least one of velocity information v1 based on the coordinate system in the longitudinal direction, width direction, and height direction, velocity information v2 based on the coordinate system in the orientation direction, side direction and vertical direction, and velocity information v3 based on the coordinate system in the forward direction, backward direction, left direction, right direction, upward direction, and downward direction may be included in the first kinematic information as velocity information. Meanwhile, when the kinematic information is expressed, for example, based on the field-based coordinate system, the difference between the orientation and movement direction of the entity cannot be reflected on the determination of the exercise load with the velocity information alone. In this case, the first kinematic information may include magnitude of velocity (speed) information s1, s2, s3, s4, and movement direction change information vd1, vd2. The speed information may be expressed based on various coordinate systems. For example, at least one of information of the magnitude of velocity, i.e., speed information s1 based on the coordinate system in the orientation direction, side direction and vertical direction, velocity information s2 based on the coordinate system in the forward direction, backward direction, side direction and vertical direction, horizontal speed information s3 which is magnitude information for combined velocity of the velocity in the longitudinal direction and velocity in the width direction, and total speed information s4 which is the magnitude information for combined velocity of the velocity in the longitudinal direction, velocity in the width direction and velocity in the height direction may be included in the first kinematic information as speed information. Also, the movement direction change information may be expressed based on various coordinate systems, for example, may be determined from previously sampled orientation. For example, the horizontal movement direction vd1, which is the angular difference between the velocity direction in the previous time unit and the velocity direction in the present time unit, may be utilized as movement direction change information. Or, the magnitude of angular difference vd2 between the velocity direction in the previous time unit and the velocity direction in the present time unit may be utilized as movement direction change information.

According to an aspect, the information of the linear movement may include second kinematic information 1623 related to acceleration of the target entity. According to an aspect of the present disclosure, the second kinematic information may include at least one of information of the magnitude of acceleration in the target time unit, or information of angular change between the direction of acceleration in the previous time unit of the target time unit and direction of acceleration in the target time unit. Meanwhile, in terms of information of acceleration, the magnitude thereof utilizes the magnitude of acceleration in the target time unit. However, for information to reflect the relation between the orientation direction and movement direction of the target entity, the change in velocity direction may be more suitable than the change in acceleration direction. Therefore, according to another aspect of the present disclosure, the second kinematic information may include at least one of information of the magnitude of acceleration in the target time unit, or information of angular change between the direction of velocity in the previous time unit of the target time unit and direction of velocity in the target time unit.

Fig. 20 illustrates an example of second kinematic information including information of acceleration.

As exemplarily explained above in the present disclosure, when the kinematic information is expressed based on the entity-based coordinate system, even if the estimated load index is determined using only acceleration information, a difference in the level of exercise load resulting from the difference between the orientation and movement direction of the entity may be reflected. Therefore, the second kinematic information may include acceleration information a1, a2, a3 of the target entity. The acceleration information may be expressed based on various coordinate systems. For example, at least one of acceleration information a1 based on the coordinate system in the longitudinal direction, width direction, and height direction, acceleration information a2 based on the coordinate system in the orientation direction, side direction and vertical direction, and acceleration information a3 based on the coordinate system in the forward direction, backward direction, left direction, right direction, upward direction and downward direction may be included in the second kinematic information as acceleration information.

Meanwhile, when the kinematic information is expressed, for example, based on the field-based coordinate system, the difference between the orientation and movement direction of the entity cannot be reflected on the determination of exercise load with the acceleration information alone. In this case, the second kinematic information may include acceleration magnitude information a4, a5, a6, a7, and movement direction change information ad1, ad2, ad3, ad4. The acceleration magnitude information may be expressed based on various coordinate systems. For example, at least one of acceleration magnitude information a4 based on the coordinate system in the orientation direction, side direction, and vertical direction, acceleration magnitude information a5 based on the coordinate system in the forward direction, backward direction, side direction and vertical direction, horizontal acceleration magnitude information a6 which is magnitude information of combined acceleration of the acceleration in the longitudinal direction and the acceleration in the width direction, and total acceleration magnitude information a7 which is the magnitude information of combined acceleration of the acceleration in the longitudinal direction, acceleration in the width direction and acceleration in the height direction may be included in the second kinematic information as acceleration magnitude information. Additionally, the movement direction change information may be expressed based on various coordinate systems, and for example, may be determined from previously sampled orientation. For example, the horizontal acceleration direction ad1, which is the angular difference between the acceleration direction in the previous time unit and the acceleration direction in the present time unit may be utilized as movement direction change information. Or, the magnitude of angular difference ad2 between the acceleration direction in the previous time unit and the acceleration direction in the present time unit may be utilized as movement direction change information. According to another aspect, the movement direction change may be determined based on the change in the velocity direction. More specifically, the horizontal movement direction ad3, which is the angular difference between the velocity direction in the previous time unit and the velocity direction in the present time unit may be utilized as movement direction change information. Or, the magnitude of angular difference ad4 between the velocity direction in the previous time unit and the velocity direction in the present time may be utilized as movement direction change information.

Additionally, according to an aspect, the information of the linear movement may further include third kinematic information 1625 related to jerk of the target entity. According to an aspect of the present disclosure, the third kinematic information may include at least one of information of the magnitude of jerk in the target time unit, or information of angular change between the direction of jerk in the previous time unit of the target time unit and the direction of jerk in the target time unit.

Similar to acceleration, for information of jerk, the magnitude thereof utilizes the magnitude of jerk in the target time unit. However, for information to reflect the relation between the orientation direction and movement direction of the target entity, the directional change of acceleration may be more suitable than the directional change of jerk, and the directional change of velocity may be more suitable than the directional change of acceleration. Therefore, according to another aspect of the present disclosure, the third kinematic information may include at least one of information of magnitude of jerk in the target time unit, or information of angular change between the direction of acceleration in the previous time unit of the target time unit and the direction of acceleration in the target time unit. Additionally, according to another aspect, the third kinematic information may include at least one of information of magnitude of jerk in the target time unit, or information of angular change between the direction of velocity in the previous time unit of the target time unit and the direction of velocity in the target time unit.

Fig. 21 illustrates an example of third kinematic information including information of jerk.

As exemplarily explained above in the present disclosure, when the kinematic information is expressed based on the entity-based coordinate system, even if the estimated load index is determined using only the jerk information, the difference in the level of exercise load resulting from the difference between the orientation and movement direction of the entity may be reflected. Therefore, the third kinematic information may include jerk information j1, j2, j3 of the target entity. The jerk information may be expressed based on various coordinate systems. For example, at least one of jerk information j1 based on the coordinate system in the longitudinal direction, width direction, and height direction, jerk information j2 based on the coordinate system in the orientation direction, side direction, and vertical direction, and jerk information j3 based on the coordinate system in the forward direction, backward direction, left direction, right direction, upward direction, and downward direction may be included in the third kinematic information as jerk information. Meanwhile, when the kinematic information is expressed, for example, based on the field-based coordinate system, the difference between the orientation and movement direction of the entity cannot be reflected on the determination of exercise load with the jerk information alone. In this case, the third kinematic information may include jerk magnitude information j4, j5, j6, j7, and movement direction change information jd1, jd2, jd3, jd4, jd5, jd6. The jerk magnitude information may be expressed based on various coordinate systems. For example, at least one of jerk magnitude information j4 based on the coordinate system in the orientation direction, side direction, and vertical direction, jerk magnitude information j5 based on the coordinate in the forward direction, backward direction, side direction, and vertical direction, horizontal jerk magnitude information j6 which is magnitude information for combined jerk of the jerk in the longitudinal direction and the jerk in the width direction, and total jerk magnitude information j7 which is magnitude information for combined jerk of the jerk in the longitudinal direction, the jerk in the width direction and the jerk in the height direction may be included in the third kinematic information as jerk magnitude information. Also, the movement direction change information also may be expressed based on various coordinate systems, and for example, may be determined from previously sampled orientation.

For example, the horizontal jerk direction jd1 which is an angular difference between the jerk direction in the previous time unit and the jerk direction in the present time unit may be utilized as movement direction change information. Or, the magnitude of angular difference jd2 between the jerk direction in the previous time unit and the jerk direction in the present time unit may be utilized as jerk direction change information. According to another aspect, the movement direction change may be determined based on the change in acceleration direction. More specifically, the horizontal acceleration direction jd3 which is an angular difference between the acceleration direction in the previous time unit and the acceleration direction in the present time unit may be utilized as movement direction change information. Or, the magnitude of angular difference jd4 between the acceleration direction in the previous time unit and the acceleration direction in the present time unit may be utilized as movement direction change information. According to another aspect, the movement direction change may be determined based on the change in acceleration direction. More specifically, the horizontal movement direction jd5 which is an angular difference between the velocity direction in the previous time unit and the velocity direction in the present time unit may be utilized as movement direction change information. Or, the magnitude of angular difference jd6 between the velocity direction in the previous time unit and the velocity direction in the present time unit may be utilized as movement direction change information.

When referring to Fig. 16 again, the kinematic information 1600 may include angular movement information 1630. Even if the position of the target entity does not change in the exercise session, there may be a case where the target entity performs a rotational movement. For example, in case where the target entity has the ball in a soccer game and takes action to keep the ball while changing the orientation direction in response to an opposing team player's pressure defense, if only linear movement is included as kinematic information, such change in orientation direction may not affect the exercise load. Additionally, when a Cruyff turn is performed while dribbling the ball, it may be determined that the dribbling performing the Cruyff turn and the dribbling not performing the Cruyff turn exert the same exercise load. According to an aspect of the present disclosure, by allowing the kinematic information to include angular movement information 1630, the exercise load generated by the body rotational movement of the target entity which does not accompany a position change can also be reflected on the determination of the estimated load index. For example, the kinematic information according to an aspect of the present disclosure may include information of at least one of a rotational movement in a roll direction, a rotational movement in a pitch direction or a rotational movement in a yaw direction of the target entity, and may also include information of at least one of angular velocity, angular acceleration or angular jerk of the target entity.

In this regard, as mentioned above in the present disclosure, at least one of the inertial coordinate system or the hybrid coordinate system may be used as a coordinate system for angular movement.

Fig. 22 illustrates an example of angular movement information based on an inertial coordinate system. As illustrated in Fig. 22, angular movement information 1631 based on the inertial coordinate system may include at least one of angular velocity information av1, angular acceleration information aa1, and angular jerk information aj1 for the rotational movement of the target entity. The angular velocity information av1 may include angular velocity based on a roll axis, angular velocity based on a pitch axis, and angular velocity based on a yaw axis. The angular acceleration information aa1 may include angular acceleration based on the roll axis, angular acceleration based on the pitch axis, and angular acceleration based on the yaw axis. The angular jerk information aj1 may include angular jerk based on the roll axis, angular jerk based on the pitch axis, and angular jerk based on the yaw axis.

Fig. 23 illustrates an example of angular movement information based on a hybrid coordinate system. For example, in a team sports match such as soccer, the rotational movement of players may mainly occur based on a vertical axis, whereas the frequency of occurrence of rotational movement based on the remaining axes may be considered to be insignificant. The hybrid coordinate system may consider only rotational movement based on a vertical axis as rotational movement. As illustrated in Fig. 23, angular movement information 1633 based on the hybrid coordinate system may include at least one of angular velocity information av2, angular acceleration information aa2, and angular jerk aj2 of the rotational movement of the target entity. The angular velocity information av2 may include angular velocity based on the vertical axis, angular acceleration information aa2 may include angular acceleration based on the vertical axis, and angular jerk information aj2 may include angular jerk based on the vertical axis.

It should be noted that kinematic information according to the embodiments of the present disclosure may be utilized in various combinations including at least some of the various elements explained above.

## Determination of exercise load based on condition information

Fig. 24 is a schematic flow chart illustrating a method for providing exercise load information according to an embodiment of the present disclosure, and Fig. 25 is a detailed flow chart illustrating a step of determining an estimated load index in Fig. 24. Hereinafter, the method for providing exercise load information according to an embodiment of the present disclosure will be explained in more detail with reference to Figs. 24 and 25.

The method and/or processes according to an embodiment of the present disclosure may be performed by a computing device. According to an aspect, the computing device may be the server 1500 or the controller 1520 included in the server 1500 as explained with reference to Figs. 1 to 3, but is not limited thereto. Additionally, any arithmetic device including a processor and a memory may be used, and a combination of a plurality of physically separated devices may be referred to as a computing device.

The method for providing exercise load information according to an embodiment of the present disclosure further considers not only kinematic information of a target entity during a target exercise session, but also condition information reflecting the characteristic related to the target exercise session to determine an estimated load index, and thus it is possible to determine information of the level of exercise load with more improved accuracy. For example, even if the same intensity of training is performed, the exercise load felt by the players may be different depending on various factors such as the condition of the player on the training day or environmental factors such as weather. According to an aspect of the present disclosure, by estimating the estimated load index by reflecting not only movement information of the target entity, but also various additional information that can affect the level of exercise load of the target entity, it is possible to reflect the level of exercise load according to factors other than the level of movement.

In this regard, the method for providing exercise load information according to an embodiment of the present disclosure may determine the estimated load index based on "activity information." Here, activity information may include kinematic information mentioned above in the present disclosure, and condition information reflecting the characteristic related to the target exercise session. Activity information includes information detected by the sensing platform 1100 as explained above, and may be understood as a concept encompassing any information that can be used to determine the level of exercise load.

Fig. 26 illustrates activity information according to an embodiment of the present disclosure. As illustrated in Fig. 26, activity information 2600 may include at least one of the kinematic information 1600 as mentioned above, session condition information 1610, and physical condition information 2620. Here, physical condition information may include heart rate information 2621, body temperature information 2623, and respiratory information 2625. Heart rate information may include indices such as $\%HR_{max}$ ($HR_{act}/HR_{max}$), %HRR (($HR_{act}$ - $HR_{rest}$) / ($HR_{max}$ - $HR_{rest}$)), and $\%VO_{2max}$ ($VO_{2act}$ / $VO_{2max}$). Condition information may be understood as a concept including at least one of session condition information and physical condition information. Examples for more various conditions are disclosed in detail below in the present disclosure.

As illustrated in Fig. 24, the method for providing exercise load information according to an embodiment of the present disclosure may include the steps of obtaining a target data set including activity information S2410, and determining an estimated load index S2420.

Hereinafter, each step of an embodiment in the method for providing exercise load information will be explained.

As illustrated in Fig. 24, the computing device may obtain a target data set including activity information S2410. More specifically, the computing device may obtain a target data set of the target entity during a target exercise session including a plurality of time units. Here, the target data set may include condition information reflecting the characteristic related to the target exercise session in addition to the sequence of the kinematic information of the target entity for each of the plurality of time units. Here, the condition information may be configured to have a sequence of condition information obtained for each of the plurality of time units included in the target exercise session similarly to the kinematic information, or have one condition information for one exercise session. Or, some of the plurality of condition information may be obtained for each time unit to form a sequence, and other condition information may have one condition information for an exercise session. Condition information will be mentioned later in detail in the present disclosure.

When referring to Fig. 24 again, the computing device may determine the estimated load index S2420. More specifically, the computing device may use an ANN to determine the estimated load index reflecting the level of exercise load of the target exercise session from the target data set. According to an aspect, the determined estimated load index may be provided as an estimate of the exercise load for the target exercise session. Here, the estimated load index may include, for example, a plurality of numerical values indicating the level of exercise load, and may be configured, for example, to have a value of 1 to 10 where 10 indicates the highest exercise intensity, but is not limited to this specific numerical range.

The estimated load index according to an aspect of the present disclosure may be an estimated value of the RPE of the target entity for the target exercise session. For example, as exemplarily illustrated in Fig. 4, the estimated load index determined according to an aspect of the present disclosure may be provided instead of the RPE. In this regard, Fig. 25 is a detailed flow chart illustrating a step of determining an estimated load index in Fig. 24. As illustrated in Fig. 25, the computing device may first prepare a trained ANN S2421, input a target data set to an input layer of the ANN S2423, and obtain an estimated load index based on a result of the output layer of the ANN S2425.

Here, the ANN may be learned using a learning data set labeled with an exercise load index so as to output the exercise load index of the target exercise session when activity information of the target entity for the target exercise session is inputted. Basically, the learning data set may be generated from the activity information for the target entity that performed the exercise session, and the labeled exercise load index may be a value collected from the target entity that performed the exercise session.

More specially, but non-limitedly, the ANN according to an embodiment of the present disclosure may be trained based on a plurality of training data sets including the first training data set. Here, the first training data set may include a sequence of kinematic information of the first entity for the first exercise session, and may be labeled with a score collected from the first entity for the exercise load of the first exercise session.

A process for determining the estimated load index using the ANN may proceed in a similar manner to the process as explained with reference to Figs. 6 to 8 above in relation to providing exercise load information based on kinematic information, but is slightly different from said process in that the training data set further includes condition information.

Hereinafter, the condition information according to the embodiments of the present disclosure is explained in more detail. As illustrated in Fig. 26, the condition information may include session condition information 2610 for the characteristic of the target exercise session itself for which the estimated load index is to be determined, and condition information 2620 of the target entity itself. For the same training program, if a particular player's physical condition is not good on the day, the player may feel a greater exercise load. Even the same training program may be felt as a greater exercise load when the program is performed under harsh climatic conditions such as high temperature and high humidity.

According to an embodiment of the present disclosure, the condition information may include daily readiness response information collected for the target exercise session from the target entity. In this regard, according to the trend of operating modern sports clubs, there are many cases where every player participating in a corresponding exercise session preceding or following each exercise session goes through a procedure of surveying and recording the daily readiness at the time of participating in the exercise session. The survey for daily readiness is also referred to as a Wellness survey. The Wellness survey may include various questions related to the health or condition of the target entity participating in the exercise session. The daily readiness response information according to an aspect may include at least one of a sleep time evaluation value, a sleep quality evaluation value, a fatigue level evaluation value, a soreness level evaluation value, a stress level evaluation value or a mood evaluation value, but is not limited thereto, and may include any evaluation value information related to the readiness of the target entity.

Fig. 27 illustrates an example of a Wellness survey for securing daily readiness response information according to an embodiment of the present disclosure. As exemplarily illustrated in Fig. 27, a plurality of items for evaluating daily readiness may be asked, and for example, an evaluation value may be received for every question. The collected evaluation values for each item may be included in the condition information. The condition information may include the evaluation values for each item in parallel, and may include a sum of the evaluation values of each item.

For the same amount of exercise, the level of the exercise load may be different depending on psychological factors of the target entity, for example, the sports player. The psychological factors that can affect the level of the exercise load may include factors of the target entity itself, or various conditions or condition factors of the target exercise session itself.

In this regard, according to an aspect of the present disclosure, the condition information may include exercise type information. For example, the exercise type information may reflect information as to whether the target exercise session is a match session or a training session. The exercise session may include a match session in which the victory or defeat is recorded, and a result such as league ranking or whether to advance to a tournament is determined, etc., and a training session in which training is performed in preparation for the match session. Relatively, compared to a training session, in a match session, even if the same amount of exercise is obtained, a greater exercise load may be felt due to psychological factors. According to an aspect of the present disclosure, by allowing the condition information to include exercise type information such as, for example, information as to whether the session is a match session or a training session, the estimated load index may be determined in consideration of the difference in the level of exercise load according to the type of the target exercise session.

Additionally, according to an aspect of the present disclosure, the condition information may include an importance evaluation value according to the type of the target exercise session. Even if the same amount of exercise is experienced, if the importance of the corresponding exercise session is higher, the target entity such as the sports player may have a greater psychological burden, and as a result, may experience a greater level of exercise load. For example, as mentioned above, the importance of the match session may be greater than that of the training session, and the psychological burden of the target entity may be high, and thereby the target entity could experience a greater level of exercise load for the same amount of exercise. Additionally, for example, in the case of rival matches which draw a lot of attention such as 'Korea-Japan match' or 'El Clasico,' the psychological burden can be much higher than that of a general match session. Or, in the case of an important match in the tournament such as a 'final' or a special match such as 'league winning decision match,' the session may have a higher importance and cause a greater psychological burden. By reflecting these various factors, the importance according to the type of the target exercise session may be reflected as an evaluation value. The importance evaluation value may be expressed, for example, as numerical values from 1 to 5, and may be classified into an importance type, and thus may be included in the learning data set and target data set, respectively.

According to an aspect of the present disclosure, the condition information may include information of the length of time elapsed from the previous match session to the target exercise session. According to modern sports science, a training schedule may be designed to exert maximum performance in the match session, and the condition of the target entity participating in the exercise session may be different depending on the elapsed time from the match session. For example, the exercise loads felt by the sports player on the second and third day of training after the match session may be different even for the same amount of exercise. Therefore, the condition information according to an aspect of the present disclosure may include information of the length of time elapsed from the previous match session to the target exercise session, and may determine the estimated load index by reflecting the difference in the level of exercise load according to the elapsed degree from the match session.

According to an aspect of the present disclosure, the condition information may include information of the length of time elapsed from the opening day of the sports season including the target exercise session to the target exercise session. In general, the team sports matches are performed over a long period of time in the corresponding season. After playing over a long season ranging from at least several months to a year, various conditions such as physical fitness or physical condition of the target entity such as the sports player participating in the exercise session may change. For example, towards the end of the season, the target entity may experience a relatively high level of exercise load for the same amount of exercise. On the contrary, in the very beginning of the season, despite the training for the season, for reasons such as adaptation to the real game, the target entity may feel a higher level of exercise load for the same amount of exercise. According to an aspect of the present disclosure, by allowing the target exercise session for which the level of exercise load is to be determined to include information of the length of time elapsed from the opening day of the sports season including the corresponding target exercise session, the estimated load index may be determined by reflecting how far the season has elapsed such as whether the target exercise session is in the beginning, in the middle or near the end of the season.

According to an aspect of the present disclosure, the condition information may include information of physical condition of the target entity. Even for the same amount of exercise, different levels of exercise load may be felt depending on the physical condition of the target entity such as the sports player. Therefore, according to an aspect of the present disclosure, by allowing the condition information to include information of the physical condition of the target entity, the estimated load index may be determined reflecting the same.

Here, information of physical condition may include at least one of age, height, weight, strength evaluation value, muscle strength evaluation value, maximum heart rate or maximum oxygen consumption. Meanwhile, the physical condition is not limited thereto, and various evaluation factors capable of expressing the physical condition of the target entity may be included in the information of the physical condition.

According to an aspect of the present disclosure, the condition information may include environment information for the target exercise session. For example, the environment information may reflect at least one of weather information, temperature information, humidity information or season information, but is not limited thereto. As mentioned above, when the weather is too hot or humid, a great degree of fatigue may be felt for the same amount of exercise. Additionally, a relatively high level of exercise load may be felt in an exercise session performed in the rain. Therefore, according to an aspect of the present disclosure, by allowing the condition information to include environment information for the target exercise session, the estimated load index may be determined reflecting environmental factors for the target exercise session.

According to an aspect of the present disclosure, the condition information may include biological measurement information of the target entity for the target exercise session. Here, the biological measurement information may include at least one of heart rate information, information of a ratio of heart rate to maximum heart rate, body temperature information, oxygen consumption information, or information of a ratio of oxygen consumption to maximum oxygen consumption. As explained with reference to Fig. 26, the physical condition information may include biological measurement information of the physical condition information such as heart rate information, body temperature information, or respiratory information. Even for the same amount of exercise, the perceived exercise load may be different depending on the biological conditions of the target entity. According to an aspect of the present disclosure, by allowing the condition information to include biological information as additional information, the estimated load index may be determined by more accurately reflecting different levels of exercise load depending on the target entity. Here, the biological measurement information may be obtained, for example, through various sensors such as the bio sensing module 1317 provided in the sensing platform.

As mentioned above, although various activity information has been explained, it should be noted that the determination of the estimated load index according to the method for providing exercise load information according to an embodiment of the present disclosure may be performed based on the target data set according to a combination of at least some of the aforementioned plurality of activity information, kinematic information, and condition information.

## Calibration of estimated load index

Fig. 28 is a schematic flow chart illustrating a method for providing exercise load information according to an embodiment of the present disclosure, Fig. 29 is a detailed flow chart illustrating an embodiment of a step of determining an estimated load index in Fig. 28, and Fig. 30 is a detailed flow chart illustrating another embodiment of the step of determining the estimated load index in Fig. 28. Hereinafter, the method for providing exercise load information according to an embodiment of the present disclosure is explained in more detail with reference to Figs. 28 to 30.

The method and/or processes according to an embodiment of the present disclosure may be performed by a computing device. According to an aspect, the computing device may be the server 1500 or the controller 1520 included in the server 1500 as explained with reference to Figs. 1 to 3, but is not limited thereto. Any arithmetic device including a processor and a memory may be used, and a combination of a plurality of physically separated devices may be referred to as a computing device.

The method for providing exercise load information according to an embodiment of the present disclosure may further reflect the characteristic of the target entity when providing information of the level of exercise load of the target entity for the target exercise session to further improve the accuracy of the estimated load index. According to an embodiment of the present disclosure, when determining the estimated load index, the estimated load index may be determined to be adaptive for a target entity by using "target-specific information"

In the present disclosure, "target-specific information" may be understood as including a characteristic of a target entity for which exercise load information is to be provided, or information for identifying this target entity. For example, since the level of exercise load experienced may be different even for the same amount of activity depending on the fitness level of the target entity, the target-specific information may include the fitness level of the target entity as a characteristic of the target entity. Or, the information capable of evaluating a strength level such as the league to which the target entity belongs may be included in the target-specific information as identification information of the target entity.

According to an aspect of the method for determining the exercise load adaptive for target entity using the target-specific information, the calibrated estimated load index may be determined by applying a modifier considering target-specific information to a normal estimated load index determined based on the kinematic information of the target entity.

According to another aspect of the present disclosure, when making the ANN learn, for example, by using a network fusion structure, the estimated load index adaptive for target entity may be obtained by utilizing data considering the characteristic of the target entity together with the normal data.

According to another aspect of the present disclosure, the estimated load index adaptive for target entity may be obtained by classifying the learning data according to the classification to which the target entity belongs, preparing ANN separately for each classification so as to train the ANN based on the learning data corresponding thereto, and using the corresponding learned ANN when determining the estimated load index.

Hereinafter, the embodiments for providing exercise load information adaptive for target entity according to the present disclosure will be explained in more detail.

As illustrated in Fig. 28, the method for providing exercise load information according to an embodiment of the present disclosure may include the steps of obtaining the target-specific information S2810, obtaining the target data set S2820, and determining the estimated load index S2830.

Hereinafter, each step in an embodiment of the method for providing exercise load information will be explained.

As illustrated in Fig. 28, the computing device may obtain the target-specific information S2810. More specifically, the computing device may obtain the target-specific information reflecting at least one of identification information or characteristic information for the target entity.

As mentioned above, the target-specific information which can be utilized to determine the estimated load index adaptive for target entity may include unique information of the target entity, characteristic information expressing the characteristic itself of the target entity, or identification information for identifying the classification or type of the corresponding target entity. The target-specific information may include a modifier which is applied ex post facto to the estimated load index which can be determined without considering the unique characteristic of the target to reflect the unique characteristic of the target, and data which is inputted to the ANN together with kinematic information to determine the estimated load index. The target-specific information may be obtained, for example, by being inputted from previously delivered information, and may be calculated through a specific process related to the target entity.

When referring to Fig. 28 again, the computing device may obtain the target data set S2820. More specifically, the computing device may obtain the target data set of the target entity during the target exercise session including a plurality of time units. Here, the target data set may include a sequence of kinematic information of the target entity for each of the plurality of time units. As to the procedure of obtaining the sequence of kinematic information, at least some of the various processes explained above in the present disclosure can be applied.

When referring to Fig. 28, the computing device may use the ANN to determine the estimated load index S2830. More specifically, the computing device may determine, from the target-specific information and the target data set, an estimated load index reflecting the level of exercise load of the target entity for the target exercise session using the ANN. According to an aspect, the determined estimated load index may be provided as an estimate of the exercise load for the target exercise session. Here, the estimated load index may include, for example, a plurality of numerical values indicating the level of exercise load. For example, the estimated load index may be configured to have a value from 1 to 10 where 10 indicates the highest exercise intensity, but is not limited to such specific numerical range.

The estimated load index according to an aspect of the present disclosure may be an estimate of the RPE of the target entity for the target exercise session. For example, as exemplarily illustrated in Fig. 4, the estimated load index determined according to an aspect of the present disclosure may be provided instead of the RPE.

In this regard, Fig. 29 is a detailed flow chart illustrating an embodiment of a step of determining an estimated load index in Fig. 28. According to an embodiment, the estimated load index may be determined based on the kinematic information without considering the characteristic of the target entity, and the estimated load index adaptive for target entity may be determined with improved accuracy by applying the modifier considering the characteristic of the target entity to the determined estimated load index.

More specifically, as illustrated in Fig. 28, the computing device may first prepare a trained ANN S2831, input a target data set to an input layer of the ANN S2833, and obtain an estimated load index based on a result of the output layer of the ANN S2835.

Here, the ANN may be learned by using a learning data set labeled with an exercise load index so as to output the exercise load index of the target exercise session when activity information of the target entity for the target exercise session is inputted. Basically, the learning data set may be generated from the activity information for the target entity that performed the exercise session, and the labeled exercise load index may be a value collected from the target entity that performed the exercise session.

More specially, but non-limitedly, the ANN according to an embodiment of the present disclosure may be trained based on a plurality of training data sets including the first training data set. Here, the first training data set may include a sequence of kinematic information of the first entity for the first exercise session, and may be labeled with a score collected from the first entity for the exercise load of the first exercise session.

As to the process for determining the estimated load index using the ANN, at least some of the processes explained with reference to Figs. 6 to 8 above in relation to providing exercise load information based on the kinematic information may be applied.

For example, as illustrated in Fig. 29, the computing device may input the target data set to the input layer of the ANN S2833, and obtain the estimated load index based on the result of the output layer of the ANN S2835.

Meanwhile, here, the target-specific information according to an aspect of the present disclosure may include a modifier for the estimated load index corresponding to the target entity. In other words, information for determining a calibrated estimated load index with improved accuracy by reflecting the unique characteristic of the target entity may be included. In this case, obtaining the estimated load index S2835 may be performed by multiplying the result of the output layer of the ANN by the modifier. Therefore, the obtained estimated load index may be an estimate related to the level of exercise load closer to that actually felt by the target entity by reflecting the unique characteristic of the target entity.

Hereinafter, the examples of the modifier according to the embodiments of the present disclosure will be explained in more detail.

According to an aspect, the modifier may reflect the evaluation information of fitness level of the target entity. More specifically, the modifier may reflect a ratio of a match load index to a maximum value of the load index, wherein the match load index may be obtained based on the kinematic information of the target entity for at least one recent match session using the ANN.

In this regard, the modifier may be provided to evaluate the level of exercise load by reflecting individuality of the target entity. For example, if the ANN for determining the estimated load index is trained based on a learning data set obtained from a plurality of entities, it is assumed that the fitness levels of the plurality of entities are the same. In general, considering the degree of difference in fitness level among the sports players in a league of the same level in which the learning data is collected, even if it is assumed that the fitness levels of the plurality of entities are the same in some embodiments, there may be no serious errors in calculating the estimated load index. However, each individual sports player has different fitness levels, and in order to estimate the level of exercise load more accurately, it is more advantageous to determine the estimated load index by reflecting the fitness level of each sports player. For the same amount of exercise, an entity with a high fitness level would experience a relatively low exercise load, and an entity with a low fitness level would experience a relatively high exercise load, and thus the estimated load index may be appropriately calibrated based on the fitness level.

As a factor for reflecting the fitness level of the target entity, the estimated load index for the match session, that is, the match load index may be considered. The exercise session may be divided into a match session which is a session performing an actual match, and a training session performing training for this.

In a team sports match, all players perform the exercise by exerting their maximum strength or fitness levels. Additionally, when surveying the RPE using a response manner, all players would respond with a maximum RPE (for example, 10 points of RPE in the calibrated BORG manner). In consideration of the above, the estimated load index calculated by inputting to the ANN the kinematic information of the target entity collected for the match session, and the modifier reflecting the ratio of maximum value (e.g., 10) of the load index to reflect the fitness level of the target entity may be determined.

Fig. 31 is an exemplary view illustrating determination of a match load index. As illustrated in Fig. 31, the match load index 3130 may be determined based on the value outputted by inputting to the ANN 3120 the kinematic information 3110 of the target entity for at least one recent match session. Here, as to the ANN 3120, the ANN for determining the estimated load index before reflecting the modifier of the target entity may be utilized, and an additionally learned ANN may be utilized. When the match load index 3130 determines the estimated load index, for example, by using the kinematic information for one recent match, the determined estimated load index may be used as the match load index. Or, a plurality of estimated load indices may be determined using the kinematic information for a plurality of recent matches, and a representative value of the plurality of estimated load indices, for example, an average value may be used as the match load index.

As to the predetermined first target entity, the match load index determined based on the kinematic information for the match session may be smaller than the maximum value of the load index. For the match session, the response load index obtained by asking a question about the first target entity may obtain a perfect score (e.g., 10 points), but the match load index for the first entity based on the ANN may be determined to be 8 points, for example. Therefore, as to the first entity, a modifier reflecting the ratio (8:10) of maximum value of the load index to match load index may be determined. For example, as a simplest example, the estimated load index of the first target entity may be determined by multiplying the result outputted by the ANN based on the target data set of the target exercise session by 10/8 as a modifier. When the output value of the ANN for the first target entity determined based on the target data set of the target exercise session is 7, the estimated load index of the first target entity reflecting the modifier may be determined to be 8.75 (7 * 10/8).

Meanwhile, this is only an exemplary method for determining the modifier, and various calibration standards may be calculated based on the ratio of maximum value of the load index to match load index.

According to an aspect of the present disclosure, the modifier may reflect evaluation information of the league to which the target entity belongs. More specifically, the modifier may reflect the ratio of evaluation value of the league to which the target entity belongs to evaluation value of the league to which the first entity in the training data set belongs.

As mentioned above, the difference in fitness level among the plurality of sports players belonging to the same level league may not be great. However, for example, depending on the league to which the target entity belongs such as professional league, amateur league, and youth league, the expected fitness level may be significantly different. In this regard, the modifier may be determined by reflecting evaluation information of the league corresponding to the learning data used to train the ANN, and evaluation information of the league to which the target entity belongs.

For example, the ANN used to determine the estimated load index is learned based on the kinematic information of the target entities of a professional league, whereas when the target entity is a sports player belonging to a youth league, a modifier of 10/6 may be determined as a simple example by reflecting the evaluation information of the professional league (e.g., 10 points) and the evaluation information of the youth league (e.g., 6 points). In other words, assuming that the value outputted by inputting the target data set of the second target entity belonging to the youth league for the target exercise session to the ANN learned based on the training data set corresponding to the professional league is 5, the estimated load index of the second target entity reflecting the modifier may be determined to be 8.34 (5 * 10/6). However, this is only an exemplary method for determining the modifier, and various calibration standards may be calculated based on the ratio of evaluation information of the league corresponding to the training data set to evaluation information of the league corresponding to the target entity.

Fig. 30 is a detailed flow chart illustrating another embodiment of the step of determining the estimated load index in Fig. 28. According to an embodiment, as to determining the estimated load index, the estimated load index with improved accuracy adaptive for target entity may be determined by determining the estimated load index based on the ANN in consideration of the information of characteristic of the target entity and the kinematic information.

In this regard, according to an aspect of the present disclosure, when making the ANN learn, for example, by using a network fusion structure, data obtained in the match session to consider the characteristic of the target entity, and data obtained in a normal exercise session may be utilized together. The estimated load index adaptive for target entity may be obtained by inputting, to the ANN trained as above, the target data set of the target entity during the target exercise session and the data of the target entity in the match session.

More specifically, as illustrated in Fig. 30, the computing device may first prepare a trained ANN S2837, input a target data set and target-specific information to an input layer of the ANN S2838, and obtain an estimated load index based on a result of the output layer of the ANN S2839.

Here, the ANN may be learned using a learning data set labeled with an exercise load index, and a match data set labeled with the maximum score of the exercise load index so as to output the exercise load index of the target exercise session when activity information of the target entity for the target exercise session and the activity information of the target entity for the match session are inputted together.

The learning data set for training the ANN may be generated, for example, from the activity information of the entity performing the exercise session, and the labeled exercise load index may be a value collected from the entity that performed the exercise session. Additionally, the match data set for training the ANN may be generated from the activity information of the entity that performed the exercise session in at least one match session, and may be labeled with the maximum value of the exercise load index.

More specifically, but non-limitedly, the ANN according to an embodiment of the present disclosure may be trained based on a plurality of training data sets including the first training data set and the first match data set, respectively.

Here, the first training data set may include a sequence of kinematic information of the first entity for the first exercise session, and may be labeled with a score collected from the first entity for the exercise load of the first exercise session.

Additionally, the first match data set may include a sequence of kinematic information of the first entity for at least one match session, and may be labeled with the maximum score.

According to an aspect, the ANN may be trained using a fusion between a training characteristic map corresponding to the first training data set and a match characteristic map corresponding to the first match data set. For example, in the same manner as training the ANN using data related to image RGB value and data related to depth, respectively, it is possible to train the ANN by utilizing two or more different types of characteristic values as learning data. For example, like the network fusion structure, the ANN may include a first network generating a first characteristic map for a first characteristic value and a second network generating a second characteristic map for a second characteristic value. A value outputted to the output layer may be determined using a fusion of the first characteristic map and the second characteristic map.

According to an aspect of the present disclosure, for an entity such as, for example, a sports player, the first characteristic value may be kinematic information obtained from a match session in which the entity participates in the sports match.

The maximum value for the exercise load may be labeled for the kinematic information of the match session. The second characteristic value may be kinematic information obtained from any exercise session in which the entity participates. The any exercise session may include both a match session and a training session, or may include only a training session excluding the match session. As to kinematic information for the any exercise session, a response score for an exercise load of the entity participating in the corresponding exercise session may be labeled.

A training characteristic map may be generated based on the first training data set including a sequence of kinematic information for any exercise session of the first entity, and a match characteristic map may be generated based on the first match data set including a sequence of kinematic information during the match session of the first entity. The training characteristic map and the match characteristic map are concatenated, and thus may be provided to a fully connected (FC) layer directly or through an additional layer. Various network fusion structures are possible depending on when the training characteristic map and match characteristic map are concatenated. In this regard, Fig. 33 illustrates a model of an early fusion in a network fusion, Fig. 34 illustrates a model of a middle fusion in the network fusion, and Fig. 35 illustrates a model of a late fusion in the network fusion. As illustrated in Figs. 33 to 35, the time when the data resulting from the match data set and the data resulting from the training data set are concatenated (i.e., bonding in the early layer or bonding in the late layer) may be determined according to at least one of the various types of network fusion such as the early, middle and late fusions, but is not limited to a specific network model.

As illustrated in Fig. 30, when the ANN trained as above is prepared S2837, the computing device may input the target data set and target-specific information to the input layer of the ANN S2838, and obtain the estimated load index based on the result of the output layer of the ANN S2839. Here, the target-specific information obtained in the step of obtaining the target-specific information S2810 and inputted to the ANN S2838 may include a sequence of kinematic information of the target entity during at least one or more recent match session.

More specifically, Fig. 32 is an exemplary view illustrating determination of the estimated load index using a match data set and a training data set. As illustrated in Figs. 30 and 32, the computing device may input the target data set 3220 and target-specific information 3210 to the input layer of the ANN 3230 S2838. As mentioned above, the target data set 3220 may include a sequence of kinematic information of the target entity for the target exercise session, and the target-specific information 3210 may include kinematic information of the target entity during at least one or more recent match session.

As illustrated in Figs. 30 and 32, the computing device may obtain the estimated load index 3240 of the target entity for the target exercise session based on the result of the output layer of the ANN 3230 S2839. Therefore, the method for providing exercise load information according to an aspect of the present disclosure may determine the estimated load index according to the kinematic information of the target exercise session by reflecting the kinematic information of the target entity in the match session, and provide the exercise load information adaptive for target entity with more improved accuracy.

According to another aspect of the present disclosure, the estimated load index adapted for target entity may be obtained by classifying or collecting the learning data according to the classification to which the target entity belongs, preparing ANN separately for each classification so as to train the ANN based on the learning data corresponding thereto, and using the corresponding learned ANN when determining the estimated load index.

According to an aspect, the target-specific information obtained in the step of obtaining the target-specific information S2810 may include identification information of the league to which the target entity belongs. Also, the step of determining the estimated load index S2830 may be configured to use the ANN trained based on the plurality of training data sets corresponding to the league to which the target entity belongs among the plurality of ANNs.

More specifically, a unique characteristic of the entity such as fitness level may be different depending on a group to which the corresponding entity belongs. For example, a sports player belonging to a youth league and a sports player belonging to a professional league may have various different unique characteristics including fitness levels, and may experience different levels of exercise load with the same amount of exercise. Therefore, according to an aspect of the present disclosure, the estimated load index may be determined by classifying entities, which are subjects to the measurement of exercise load, into a plurality of groups according to a predetermined standard, training the ANN corresponding to each classification using the training data sets corresponding to each classification, and inputting the target data set of the target entity for the target exercise session to the ANN corresponding to the target entity. Therefore, the estimate for the level of exercise load may be determined with improved accuracy by reflecting the unique characteristic of the target entity.

For example, information for classifying entities into a plurality of groups may be an affiliate league. According to an aspect, the ANN for a youth league trained based on training data sets for sports players included in the youth league, and the ANN for a professional league trained based on training data sets for sports player included in the professional league may be prepared, respectively. According to an aspect, an affiliate league identifier for the target entity may be obtained as target-specific information. By using the same, the estimated load index of the target entity may be determined based on a value outputted by inputting a target data set for the target entity to an input layer of the ANN corresponding to a league to which the target entity belongs.

Various embodiments of the methods for providing exercise load information are explained as examples in the present disclosure. However, at least some of the characteristics in each embodiment may be applied to another embodiment. For example, when target-specific information is used in order to consider the characteristic of the target entity, the target data set may be configured to include condition information in addition to kinematic information. In other words, it should be understood that the method for determining exercise load information in which partial characteristics for the embodiments of the present disclosure are mutually combined are also included within the scope of technical idea of the present disclosure.

## Entity monitoring and early risk detection

In the embodiments of the present disclosure, the load index that may reflect the evaluation value for the level of exercise load may include an "estimated load index" and a "response load index." Here, the "estimated load index" may be an estimate for the exercise load calculated based on the activity information of the target entity during the target exercise session. For example, the estimated load index may be determined using the ANN. The "response load index" may be an experience by the target exercise session, for example, such as RPE, which may be a response of the degree of fatigue evaluated by the target entity itself directly received from the target entity.

According to an aspect of the present disclosure, monitoring and early risk detection for an entity such as sport players are possible by comparing the estimated load index with the response load index. When the difference between the level of exercise load predicted by the sports player and the level of exercise load actually felt by the sports player satisfies a predetermined standard, it may be determined that an abnormality has occurred in the corresponding sports player.

Recently, when operating a modern professional sports club, a system for submitting the RPE after each exercise session ended has been settled. A discrepancy between the RPE predicted for a specific player and the RPE submitted by the player may mean that there is a problem in the condition of the corresponding player. Therefore, it is possible to perform management of individual players at the club by detecting that there is a significant difference between the predicted and submitted data for a certain period, for example, weekly RPE.

Additionally, even when there is a significant difference between the predicted data and submitted data of the representative RPE for a specific team, it is possible to ask for a review on whether there are any problems such as external factors other than the training program, for example, improper meals or facilities, and excessive performance of out-of-training schedules.

Fig. 36 is a conceptual diagram illustrating a method for providing exercise load information for entity monitoring and early risk detection according to an embodiment of the present disclosure. As illustrated in Fig. 36, an estimated load index 3630 may be calculated by collecting activity information of the target entity 3610 to be monitored during the exercise session, and inputting the same to the ANN 3620. Additionally, by performing a survey procedure 3640 for the level of exercise load of the target entity 3610, a response load index 3645 reflecting a self-evaluation value for the corresponding exercise session may be obtained. By comparing 3650 the estimated load index 3630 and the response load index 3650, when a difference between the two satisfies a predetermined condition, a notification message 3660 for notifying the abnormality of the target entity may be generated and delivered to a manager. Here, the standard for the difference between the estimated load index and the response load index may include a determination that the difference between the two occurs consecutively (for example, over a week) or significantly (the difference between the estimated load index and the response load index is greater than or equal to a threshold value). In this case, it may be determined that the target entity is not in a normal condition, and the manager may take a corresponding action.

Fig. 37 is a schematic flow chart illustrating a method for providing exercise load information according to an embodiment of the present disclosure, Fig. 38 is a detailed flow chart illustrating a step of calculating the estimated load index in Fig. 37, and Fig. 39 is a detailed flow chart illustrating a step of determining the estimated load index in Fig. 38. Hereinafter, the method for providing exercise load information according to an embodiment of the present disclosure will be explained in detail with reference to Figs. 37 to 39.

The method and/or processes according to an embodiment of the present disclosure may be performed by a computing device. According to an aspect, the computing device may be the server 1500 or the controller 1520 included in the server 1500 as explained with reference to Figs. 1 to 3, but is not limited thereto. Additionally, any arithmetic device including a processor and a memory may be used, and a combination of a plurality of physically separated devices may be referred to as a computing device.

As illustrated in Fig. 37, the method for providing exercise load information according to an embodiment of the present disclosure may include the steps of calculating an estimated load index S3710, receiving a response load index S3720, and outputting a notification message S3730.

More specifically, when referring to Fig. 37, the computing device may calculate the estimated load index S3710. More specifically, the computing device may calculate the estimated load index reflecting an estimate for the level of exercise load of the target entity for the target exercise session based on kinematic information of the target entity during the target exercise session.

Here, the estimated load index may be understood as an estimate of RPE of the target entity for the target exercise session.

The process of calculating the estimated load index may be performed by reflecting at least some process included in various embodiments as explained above in the present disclosure. For example, the step of calculating the estimated load index S3710, as illustrated in Fig. 38, may include the steps of obtaining the target data set S3711, and determining the estimated load index 3713. More specifically, the computing device may obtain the target data of the target entity during the target exercise session including a plurality of time units S3711. Here, the target data set may include a sequence of kinematic information of the target entity for each of the plurality of time units. Additionally, the computing device may determine, from the target data set, an estimated load index of the target entity using the ANN S3713. More specifically, as illustrated in Fig. 38, the computing device may prepare the ANN trained based on a plurality of training data sets including the first training data set S3713a. Here, the first training data set may include a sequence of kinematic information of the first entity during the first exercise session, and may be labeled with a score collected from the first entity for the exercise load of the first exercise session. Also, the computing device may input a target data set to an input layer of the ANN S3713b, and obtain an estimated load index based on a result of the output layer of the ANN S3713c.

Although an exemplary process for obtaining the estimated load index has been explained in the above, it should be construed that at least some characteristics of the various processes (for example, activity information-based determination, calibration procedure, consideration of target-entity-specific characteristic, etc.) for obtaining the estimated load index explained above in the present disclosure or processes are combined to obtain the estimated load index.

When referring to Fig. 37 again, the computing device may receive a response load index reflecting the self-evaluation value for the level of exercise load of the target entity for the target exercise session S3720. Here, the response load index may reflect the RPE of the target entity for the target exercise session. For example, after the target entity participates in the target exercise session, it is possible to make the target entity go through a survey procedure for RPE. The target entity may answer the level of exercise load according to the target exercise session felt by itself, for example, with a numerical value from 1 to 10, and the corresponding information may be inputted through a predetermined input device and provided to the computing device. In other words, the response load index may reflect the evaluation value for the RPE felt by the actual target entity.

As illustrated in Fig. 37, the computing device may output a notification message based on the comparison result between the estimated load index and the response load index S3730. In other words, when the difference between the estimated load index and the response load index satisfies a predetermined standard, the target entity may be considered to be in an abnormal condition, and the computing device may notify the manager as to whether an abnormal condition has occurred by outputting a notification message.

Calculating the estimated load index and receiving the response load index may be consecutively performed for one or more exercise sessions. In other words, according to an aspect, the target exercise session may be a group of a plurality of exercise sessions. Therefore, the comparison between the estimated load index and the response load index may be performed for a plurality of exercise sessions. The standard for the difference between the two may be set for the difference with respect to one exercise session, or may be set for whether the difference with respect to a plurality of exercise sessions is continued.

More specifically, according to an aspect of the present disclosure, the step of outputting the notification message S3730 may be configured to output the notification message in response to the determination that the difference value between the estimated load index and the response load index is greater than or equal to a predetermined first threshold value. In other words, the computing device may calculate the difference value between the estimated load index and the response load index respectively corresponding to a specific target exercise session, and determine that the target entity is in an abnormal condition when the calculated difference value is greater than or equal to a predetermined first threshold value (e.g., 3). For example, when the level of exercise load actually felt by the target entity is excessively large compared to the level of exercise load predicted based on the data for movement such as kinematic information, there is a high possibility that the corresponding target entity can have problems. Therefore, the computing device may notify the same to the manager by outputting a notification message.

According to another aspect of the present disclosure, the step of outputting the notification message S3730 may be configured to output the notification message in response to the determination that the difference value between the estimated load index and the response load index for an exercise session that has been continued for more than a predetermined first threshold number of times is greater than or equal to a predetermined second threshold value. For example, even when the difference between the estimated load index and the response load index for the target entity is not too big, when a difference over a certain level is repeated, it may be determined that the target entity has some problems, or it may be determined as a sign that big problems such as an injury may occur. Therefore, the computing device may monitor the difference between the estimated load index and the response load index for a plurality of exercise sessions, and notify the same to the manager by outputting a notification message when an exercise session whose difference between the estimated load index and the response load index is greater than or equal to a predetermined second threshold value (e.g., 1) consecutively occurs more than a predetermined first threshold number of times (e.g., 5 times).

According to another aspect of the present disclosure, the step of outputting the notification message S3730 may be configured to output the notification message in response to the determination that a ratio of second exercise session whose difference value between the estimated load index and the response load index among a plurality of consecutive exercise sessions is greater than or equal to a third threshold value is greater than or equal to a ratio of the predetermined first threshold. For example, when monitoring the difference between the estimated load index and response load index for a plurality of exercise sessions, when a difference over a certain level occurs more frequently than an acceptable level even if it does not occur consecutively, it may be interpreted that the target entity is experiencing specific problems or as a sign for injury. Therefore, when the exercise session, whose difference value between the estimated load indices is, for example, greater than or equal to the third threshold value (e.g., 1.5) for a plurality of exercise sessions, for example exercise sessions continuing for 5 times or more, occurs, for example, 3 or more times, the computing device may determine that the plurality of exercise sessions correspond to a predetermined first threshold ratio (e.g., 0.6) or above, and notify the same to the manager by outputting a notification message. The exemplary standards for determining the abnormal condition have been explained in the above, but the technical idea of the present disclosure is not limited thereto, and it should be construed that various standards for determining the level of difference by utilizing the estimated load index and response load index for the target entity are all included in the technical idea of the present disclosure.

Meanwhile, the notification message according to an aspect of the present disclosure may be provided for various purposes. For example, the notification message may include a message for warning the risk of injury for the target entity. When the degree of difference between the estimated load index and response load index satisfies the standard, the corresponding target entity may be interpreted to show a sign for injury. Therefore, since the notification message includes a message for warning the risk of injury, the manager may take advance actions for preventing the injury of the corresponding target entity after checking the notification message. According to another aspect, the notification message may include a message for warning an abnormal condition for the target entity. Satisfaction of the standard for difference value may be interpreted such that the target entity is not in a normal condition. Therefore, since the notification message includes a message for warning the abnormal condition, the manager may utilize the same for determining a list of players for the next match session, or start an action for recovering the condition. According to another aspect, the notification message may include a message for requesting a coach interview for the target entity. The sign of abnormality of a target entity may be caused by psychological problems such as family matters or privacy issues as well as physical problems. Therefore, since the notification message includes a message for requesting the coach interview, the manager may start a coach interview for the corresponding entity.

According to another aspect, the notification may include a message for recommending a type change for the ANN. As mentioned above, according to an aspect of the present disclosure, the target entity is classified into a plurality of groups according to a certain standard, and each ANN may be trained using the training data set corresponding to each classification. Accordingly, when satisfying the standard for difference between the estimated load index and response load index for the target entity, since the notification message includes a message for recommending the type change for the ANN, a system manager may check whether a proper ANN is applied to the corresponding entity to calculate the estimated load index, and change the ANN applied as needed.

Meanwhile, according to an aspect, the target entity to be monitored or detected for a sign of abnormality may be at least one sports team, or one sports player. According to an aspect, when the target entity is at least one sports team, each of the estimated load index and response load index may be a representative index determined based on the indices for each of the at least some sports players belonging to the sports team. For example, said index may be an average value of indices for each of the plurality of sports players, but is not limited thereto, and any standard for representing the indices for each of the plurality of sports players may be utilized. According to an aspect, since the difference between the estimated load index and response load index of the sports team is monitored to detect a sign for abnormality, it is possible to determine whether there are any management-related problems in the corresponding sports team, and it is possible to check whether there are various problems such as meals or facilities, and non-training problems. According to an aspect, when the target entity is one sports player, it is possible to review problems of the corresponding sports player. The risk of injury of the corresponding sports player may be early detected, and whether there are any matters that may psychologically affect the corresponding sports player such as family problems or private issues may be reviewed.

By performing such monitoring on the target entity, the effects of preventing the target entity from being injured or improving the possibility of securing optimal conditions may be expected. Additionally, when operating a professional sports league recording an astronomical annual salary, the monitoring may be useful for improving efficiency in terms of cost invested in the target entity.

## Implementation

Hereinafter, exemplary embodiments of the process for providing exercise load information according to an aspect of the present disclosure will be explained. Meanwhile, it should be noted that the technical idea of the present disclosure is not limited to the following examples.

Fig. 40 is a schematic structure of the ANN for implementing the method for providing exercise load information according to an embodiment of the present disclosure. The dotted line in Fig. 40 indicates the information flow in a training phase, and the solid line indicates the information flow in an inference phase.

The activity information may indicate information reflecting the physical activity of a sports player performing the exercise. Like kinematic information related to the position and/or movement of the sports player, and physical condition information related to the body of the player, activity information may include information of any characteristics which change by the activity of the player.

"qRPE" may indicate an exercise load index obtained from a player through a questioning procedure following the end of an exercise session. For example, "qRPE" may be understood as a response load index.

"pRPE" may indicate a value of RPE predicted by the ANN model. For example, "pRPE" may be understood as an estimated load index.

As illustrated in Fig. 40, in the training phase, for example, activity information 3710 of a specific entity may be labeled with qRPE 3750. In other words, the activity information 3710 of a specific entity labeled with the qRPE 3750 value may be utilized as a learning data set for training the ANN 3720. Here, the ANN may be learned so that the pRPE 3730 value outputted from the ANN approaches the qRPE 3750 value.

When referring to Fig. 40 again, in the inference phase, activity information 3710 in a target exercise session of a target entity for which exercise load information is to be determined may be inputted to the ANN 3720. The ANN may output a predetermined pRPE 3730 value, and an estimated load index 3740 may be determined based on the pRPE 3730, so that the estimated exercise load index 3740 for the exercise load information of the target entity for the target exercise session may be provided.

Fig. 41 illustrates an example of providing exercise load information using kinematic information. As illustrated in Fig. 41, first, a wearable device corresponding to the target entity for which the exercise load information is to be determined may be mounted 4110. For the exercise session 4120, data for the position or orientation of the entity may be tracked 4130 based on the wearable device. And then, kinematic information 4140 may be determined using the tracking data.

Meanwhile, in the training phase, kinematic information may be labeled with a response load index (e.g., qRPE) 4150 value asking a question to the entity and receiving an answer. The ANN 4170 may be trained so that the estimated load index (e.g., pRPE) 4160 value outputted when the kinematic information 4140 is inputted approaches the response load index 4150 value.

Meanwhile, in the utilizing phase, when the kinematic information 4140 is inputted to the ANN 4170, the estimated load index 4180 value outputted may be provided as exercise load information. According to an aspect, by comparing the estimated load index 4180 outputted and response load index 4150, monitoring and early risk detection for the target entity may be performed based on whether a difference between the two satisfies a predetermined standard.

Additionally, according to an aspect, the pRPE value may not reflect individual characteristics or daily conditions of the entity. For example, assuming that the fitness level of the target entity is similar to the fitness levels of the entities corresponding to the learning data set utilized to train the ANN, information of the difference between the qRPE and pRPE may be utilized as a daily conditioning factor of the target entity.

Fig. 42 is an exemplary view illustrating the ANN in consideration of time information. As illustrated in Fig. 42, for example, like the sequence of kinematic information, the target data set may include a sequence of characteristic values corresponding to each of a plurality of time points or time units included in the target exercise session. The ANN used to implement the method for providing exercise load information according to an aspect of the present disclosure may divide the sequences included in the target data set into at least two groups according to time order so that embedding for a convolution neural network CNN may be performed for each group. As illustrated in Fig. 42, for example, the sequence 4210 of kinematic information may be divided into a first group 4211, a second group 4213, and a third group 4215 according to time order. A first group 4211, a second group 4213 and a third group 4215 may be embedded respectively for the sequence of kinematic information, and the embedding result 4230 may be inputted into a recurrent neural network RNN so that the exercise load index (for example, RPE with a value from 0 to 10) 4250 can be outputted. Here, the time interval T may be a predetermined time interval such as 5 minutes or 10 minutes.

According to an aspect of the present disclosure, when the target entity performs an exercise session, an estimate for the level of exercise load up to a current time point may be estimated in real time. For example, the real-time estimated load index for the current time point may be determined based on the sequence of kinematic information up to the current time point. Therefore, the real-time estimated load index may be utilized as a reference material on the need to replace players in the match session, or may be used to determine to stop performing the exercise session to prevent injury.

Fig. 43 is an exemplary view illustrating a method for providing real-time exercise load information according to an embodiment of the present disclosure, Fig. 44 is an exemplary view illustrating kinematic data to the N time point of Fig. 43, and Fig. 45 is an exemplary view illustrating kinematic data to the M time point of Fig. 43.

As illustrated in Fig. 43, first, a wearable device corresponding to a target entity for which exercise load information is to be determined may be mounted 4310. For the exercise session 4320, data for the position or orientation of the entity may be tracked 4330 based on the wearable device.

Next, the kinematic information 4340 to the N time point may be determined using tracking data to the N time point $T^N$. As illustrated in Fig. 44, the kinematic information 4340 to the $T^N$ may include a sequence of kinematic information of the target entity from the starting point of the exercise session to the $T^N$. As illustrated in Fig. 43, when the kinematic information 4340 of the target entity to the $T^N$ is inputted to the ANN 4350, the real-time estimated load index 4360 for the target entity at the $T^N$ may be determined. Here, since the data inputted to the ANN may be configured to have a predetermined length, for example, as illustrated in Fig. 44, zero-padding may be performed following or preceding the sequence of kinematic information of the target entity to the $T^N$.

When referring to Fig. 43 again, the kinematic information 4370 to the M time point may be determined using tracking data to the M time point $T^M$ following the $T^N$. As illustrated in Fig. 45, the kinematic information 4370 to the $T^M$ may include a sequence of kinematic information of the target entity from the starting point of the exercise session to the $T^M$. As illustrated in Fig. 43, when the kinematic information 4370 of the target entity to the $T^M$ is inputted to the ANN 4380, the real-time estimated load index 4390 for the target entity at the $T^M$ may be determined. Here, since the data inputted to the ANN is configured to have a predetermined length, for example, as illustrated in Fig. 45, zero-padding may be performed following or preceding the sequence of kinematic information of the target entity to the $T^M$.

As mentioned above, the real-time estimated load index for the $T^N$ or $T^M$, which is a specific time point of the exercise session, may be determined. Based on the real-time estimated load index for a specific time point, the procedure for monitoring the target entity and warning the threshold load may be performed. Fig. 46 illustrates a procedure for warning threshold load according to real-time exercise load measurement. As illustrated in Fig. 46, whether the threshold level has elapsed may be monitored by calculating the real-time estimated load index at a predetermined time interval after the start of the exercise session. When performing monitoring at the $T^N$, since the real-time estimated load index at $T^N$ does not elapse the threshold level, a warning message may not be delivered. On the other hand, when performing monitoring at the $T^M$, since the real-time estimated load index at $T^M$ elapses the threshold level, a warning message may be delivered. The interval of monitoring may be adjusted by various elements such as system performance or manager's setting. Through the monitoring procedure as above, it is possible to provide a reference as to whether a player needs to be replaced in a match. Additionally, it is possible to achieve an effect of preventing injury by stopping an exercise session for a player at risk of injury.

The method according to the present invention described above may be implemented as a computer-readable code on a computer-readable recording medium. The computer-readable recording medium includes any type of recording medium in which data that can be read by a computer system is stored, such as a read only memory (ROM), a random access memory (RAM), a magnetic tape, a magnetic disk, a flash memory, an optical data storing device, etc. Additionally, the computer-readable recording medium may be dispersed in the computer system connected by a computer communication network, and thus can be stored and executed as a code which can be read in a dispersed manner.

Although explained above with reference to the drawings or embodiments, it does not mean that the scope of protection of the present invention is limited by the drawings or embodiments, and it should be understood that a person skilled in the art can variously modify and change the present invention within a scope not deviating from the idea and area of the present invention as recited in the following claims.

Specifically, the characteristics explained may be executed in a digital electronic circuit, or a computer hardware, a firmware, or a combination thereof. The characteristics may be executed in a computer program product implemented within a storage device in a machine-readable storage device, for example, for execution by a programmable processor. Additionally, the characteristics may be performed by a programmable processor executing a program of instructions for performing functions of the explained embodiments by operating on the input data and generating the output. The explained characteristics may be executed within at least one computer programs which can be executed on a programmable system including at least one programmable processor, at least one input device, and at least one output device which are combined in order to receive data and instructions from the data storage system, and transmit data and instructions to the data storage system. The computer program includes a set of instructions which can be used directly or indirectly in a computer in order to perform a specific operation for a predetermined result. The computer program is written in any form of programming language including complied or integrated languages, and may be used in any form included as another unit suitable for use in a module, an element, a subroutine, or another computer environment, or as an independently-operating program.

Processors suitable for executing a program of instructions include, for example, both general and special purpose microprocessors, and either a single processor or multiprocessors of different types of computers. Also, storage devices suitable for implementing computer program instructions and data embodying the explained characteristics include, for example, semiconductor memory devices such as EPROM, EEPROM, and flash memory devices, magnetic devices such as internal hard disks and removable disks, optical magnetic disks, and all types of non-volatile memory including CD-ROM and DVD-ROM disks. The processor and memory may be integrated in application-specific integrated circuits (ASIC) or added by the ASICs. Although the above-mentioned present invention is explained based on a series of functional blocks, it is not limited by the aforementioned embodiments and attached drawings. Additionally, it would be obvious to a person skilled in the art to which the present invention pertains that various substitutions, modifications and changes are possible within a scope not deviating the technical idea of the present invention.

A combination of the above-mentioned embodiments is not limited to the aforementioned embodiments, and various types of combinations may be provided as well as the aforementioned embodiments according to implementation and/or necessity.

In the above-mentioned embodiments, the methods are explained based on a flow chart with a series of steps or blocks, but the present invention is not limited to the order of the steps, and some steps may be performed in a different order with other steps other than the above, or may be performed at the same time. Also, a person skilled in the art would understand that the steps in the flow chart are not exclusive, other steps can be included, or one or more steps in the flow chart can be deleted without affecting the scope of the present invention.

The above-mentioned embodiments include various aspects of examples. Although all possible combinations to express various aspects cannot be described, a person skilled in the art would recognize that other combinations are possible. Therefore, the present invention should include all other substitutions, modifications, and variations falling within the scope of the following claims.

## Claims

1. A method for providing exercise load information of a target entity, comprising:

   obtaining a target data set of the target entity for a target exercise session including a plurality of time units, wherein the target data set includes a sequence of kinematic information of the target entity for each of the plurality of time units; and
   determining, based on the target data set, an estimated load index reflecting a level of exercise load of the target entity for the target exercise session using an artificial neural network (ANN).

2. The method of claim 1, wherein the target entity is at least one sports team, and the method further comprises:
   determining a representative load index of the sports team for the target exercise session based on the estimated load indices for each of the at least some sports players belonging to the sports team.

3. The method of claim 1, wherein determining the estimated load index comprises:

   preparing the ANN trained based on a plurality of training data sets including a first training data set, wherein the first training data set includes a sequence of kinematic information of a first entity for a first exercise session, and is labeled with a score collected from the first entity for the exercise load of the first exercise session;
   inputting the target data set to an input layer of the ANN; and
   obtaining the estimated load index based on a result of an output layer of the ANN.

4. The method of claim 1, further comprising:
   measuring position information of the target entity for the target exercise session using a positioning device corresponding to the target entity, wherein the kinematic information is generated based on the position information.

**5.** The method of claim 1, wherein the kinematic information is measured using an inertial sensor corresponding to the target entity.

**6.** The method of claim 1, wherein the kinematic information comprises: first kinematic information related to a velocity of the target entity; and second kinematic information related to an acceleration of the target entity.

**7.** The method of claim 6, wherein the kinematic information further comprises third kinematic information related to a jerk of the target entity.

**8.** The method of claim 6, wherein the first kinematic information comprises information related to the magnitude of velocity in a target time unit; and information related to the angular change between a direction of velocity in a time unit prior to the target time unit and a direction of velocity in the target time unit.

**9.** The method of claim 6, wherein the second kinematic information comprises:

information related to the magnitude of acceleration in the target time unit; and
information related to the angular change between a direction of acceleration in the time unit prior to the target time unit and a direction of acceleration in the target time unit.

**10.** The method of claim 6, wherein the second kinematic information comprises:

information related to the magnitude of acceleration in the target time unit; and
information related to the angular change between a direction of velocity in the time unit prior to the target time unit and a direction of velocity in the target time unit.

**11.** The method of claim 7, wherein the third kinematic information comprises:
information related to the magnitude of jerk in the target time unit; and information related to the angular change between a direction of jerk in the time unit prior to the target time unit and a direction of jerk in the target time unit.

**12.** The method of claim 7, wherein the third kinematic information comprises:
information related to the magnitude of jerk in the target time unit; and information related to the angular change between a direction of acceleration in the time unit prior to the target time unit and a direction of acceleration in the target time unit.

**13.** The method of claim 7, wherein the third kinematic information comprises:
information related to the magnitude of jerk in the target time unit; and information related to the angular change between a direction of velocity in the time unit prior to the target time unit and a direction of velocity in the target time unit.

**14.** The method of claim 1, wherein the kinematic information is expressed based on a field-based coordinate system, comprising:

a first axis for a length of a field in which the exercise session is performed; and
a second axis for a width of the field in which the exercise session is performed.

**15.** The method of claim 1, wherein the kinematic information is expressed based on an entity-based coordinate system, comprising:

a forward-backward axis corresponding to a heading direction of the target entity; and
a left-right axis corresponding to a side direction of the target entity.

**16.** The method of claim 1, wherein the kinematic information comprises:

information related to at least one of a rotational movement in a roll direction, a rotational movement in a pitch direction, or a rotational movement in a yaw direction of the target entity; and
information related to at least one of angular velocity, angular acceleration, or
angular jerk of the target entity.

**17.** The method of claim 1, wherein the estimated load index is an estimate of a ratio of perceived exertion (RPE) of the target entity for the target exercise session.

**18.** The method of claim 1, wherein the target data set is configured to have a predetermined length by performing zero-padding prior to the sequence of the kinematic information.

**19.** An apparatus for providing exercise load information of a target entity, comprising a processor and a memory, wherein the processor is configured to:

obtain a target data set of the target entity for a target exercise session including a plurality of time units, wherein the target data set includes a sequence of kinematic information of the target entity for each of the plurality of time units; and determine, based on the target data set, an estimated load index reflecting a level of exercise load of the target entity for the target exercise session using an artificial neural network (ANN).

**20.** A non-transitory computer-readable storage medium having processor-executable instructions stored thereon, wherein the instructions are executed by the processor, allowing the processor to:

obtain a target data set of a target entity for a target exercise session including a plurality of time units, wherein the target data set includes a sequence of kinematic information of the target entity for each of the plurality of time units; and determine, based on the target data set, an estimated load index reflecting a level of exercise load of the target entity for the target exercise session using an artificial neural network (ANN).

FIG. 1

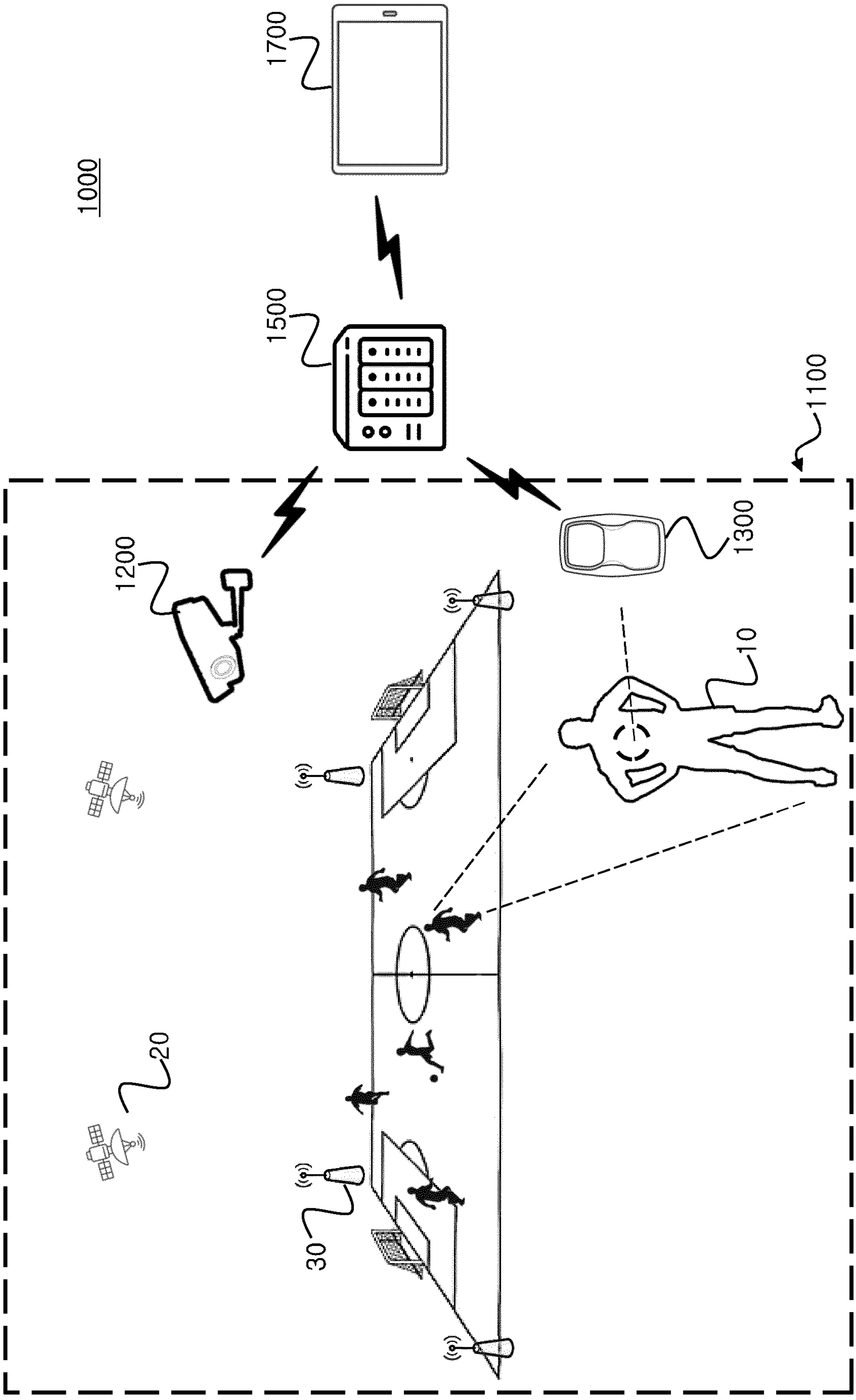

FIG. 2

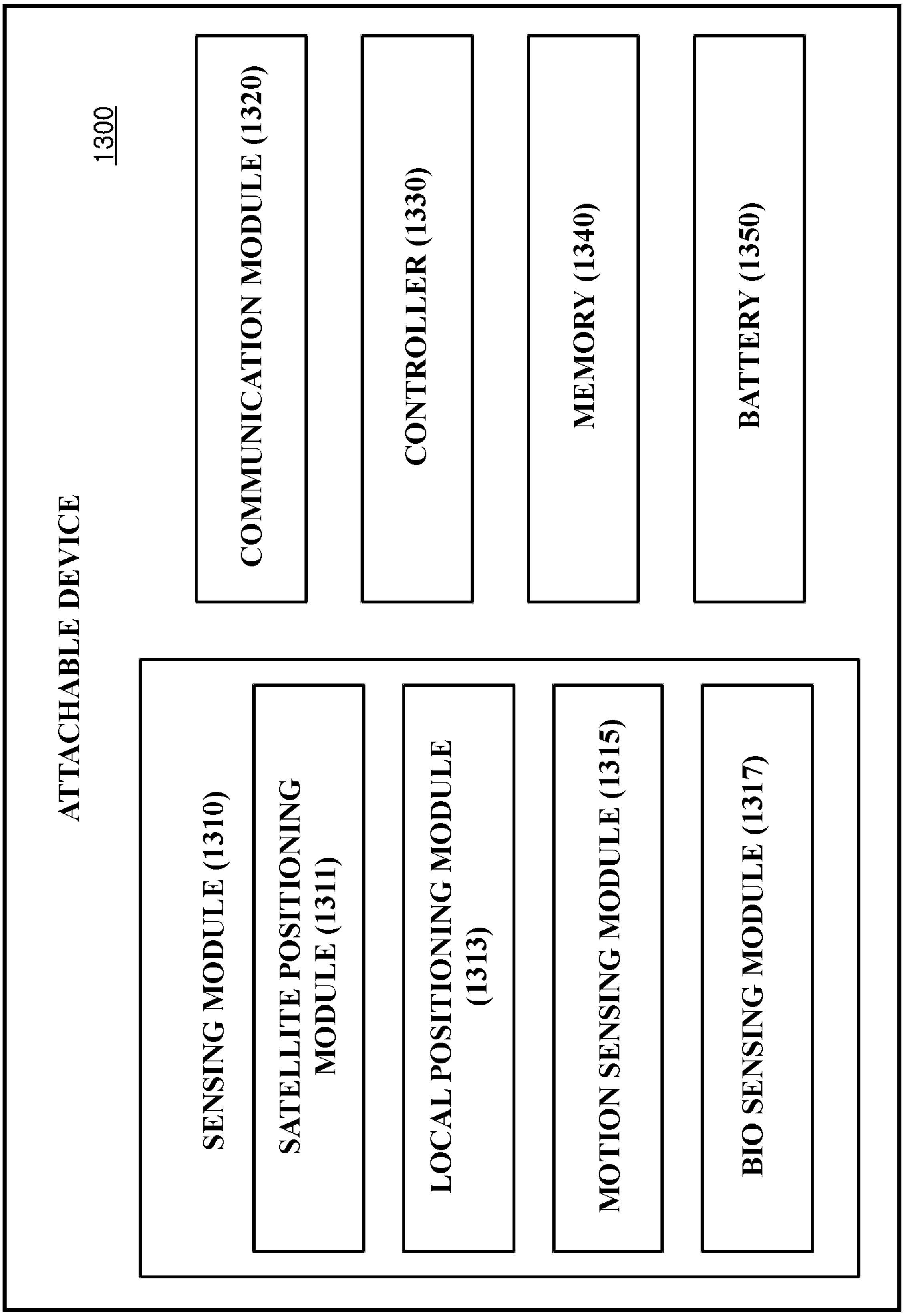
ATTACHABLE DEVICE
1300
SENSING MODULE (1310)
SATELLITE POSITIONING MODULE (1311)
LOCAL POSITIONING MODULE (1313)
MOTION SENSING MODULE (1315)
BIO SENSING MODULE (1317)
COMMUNICATION MODULE (1320)
CONTROLLER (1330)
MEMORY (1340)
BATTERY (1350)

FIG. 3

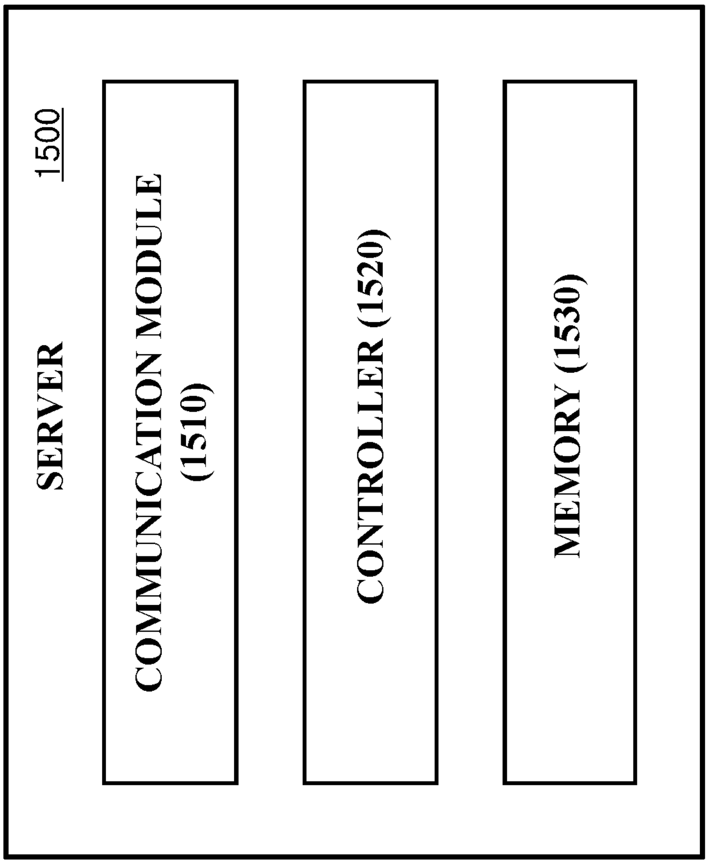
SERVER 1500
COMMUNICATION MODULE (1510)
CONTROLLER (1520)
MEMORY (1530)

# FIG. 4

| BORG RPE | Modified RPE | BREATHING | %MAX HR |
|---|---|---|---|
| 6 | 0 | No exertion | 50% - 60% |
| 7 | | Very Light | |
| 8 | 1 | | |
| 9 | | | |
| 10 | 2 | Notice breathing deeper, but still comfortable. Converstions possible. | 60% - 70% |
| 11 | | | |
| 12 | 3 | | |
| 13 | | Aware of breathing harder; More difficult to hold a conversation | 70% - 80% |
| 14 | 4 | | |
| 15 | 5 | Starting to breathe hard and get uncomfortable | 80% - 90% |
| 16 | 6 | | |
| 17 | 7 | Deep and forceful breathing, Uncomfortable, don't want to talk | 90% - 100% |
| 18 | 8 | | |
| 19 | 9 | Extremely hard | |
| 20 | 10 | Maximum exertion | |

FIG. 5

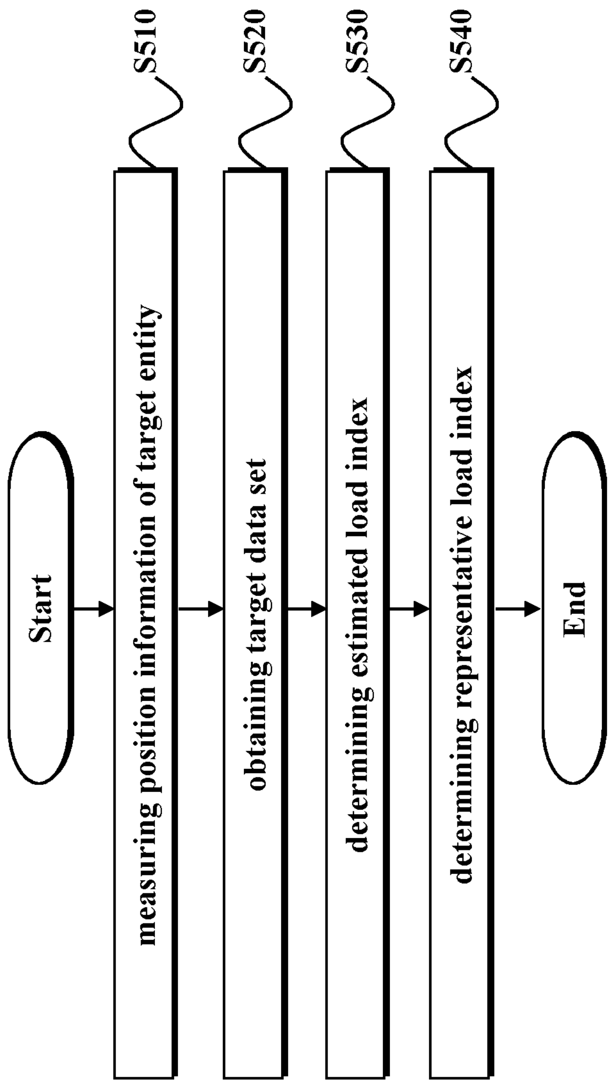
Start
measuring position information of target entity
S510
obtaining target data set
S520
determining estimated load index
S530
determining representative load index
S540
End

FIG. 6

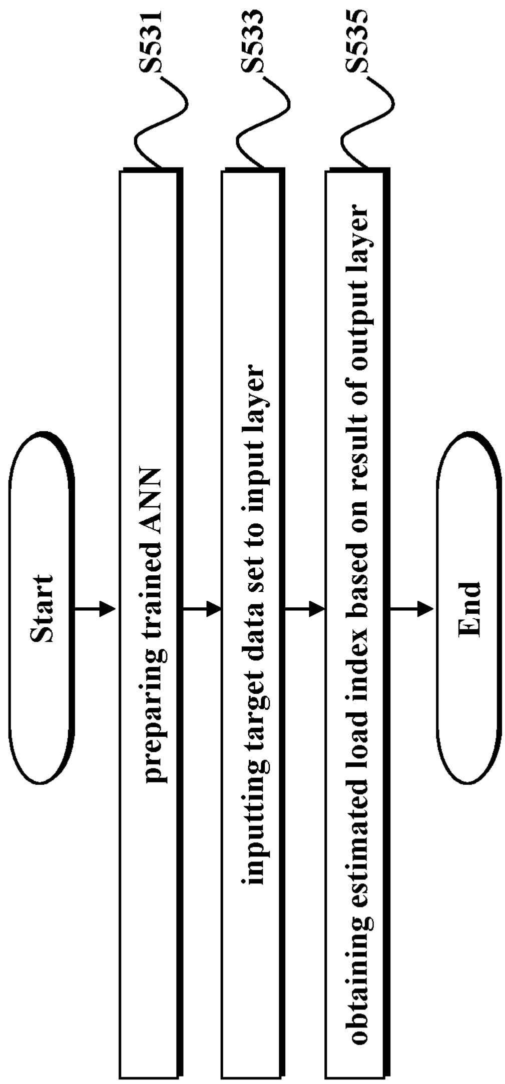
Start
preparing trained ANN
S531
inputting target data set to input layer
S533
obtaining estimated load index based on result of output layer
S535
End

FIG. 7

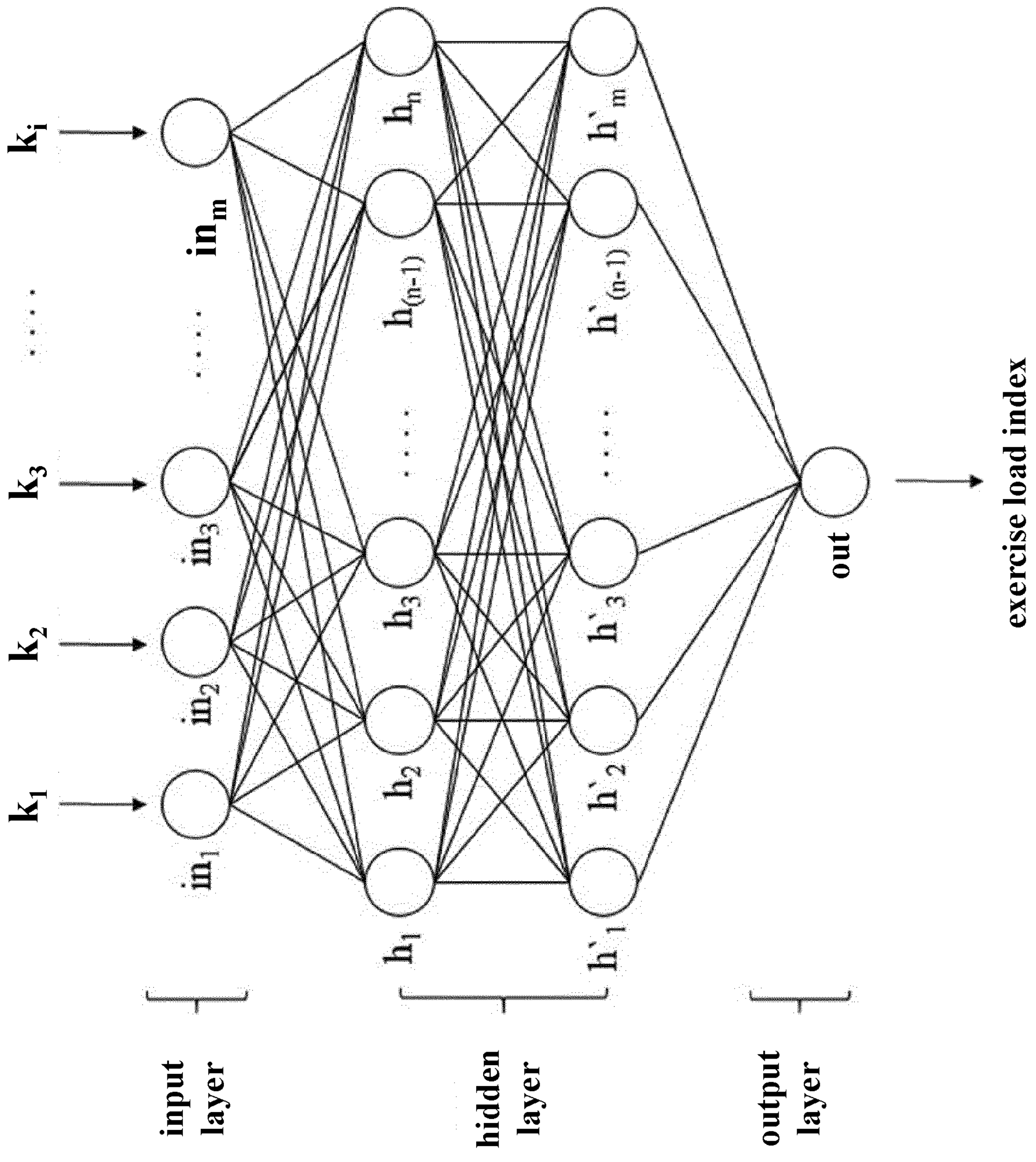
k1
k2
k3
....
ki
in1
in2
in3
....
inm
h1
h2
h3
....
h(n-1)
hn
h`1
h`2
h`3
....
h`(n-1)
h`m
out
exercise load index
input layer
hidden layer
output layer

FIG. 8

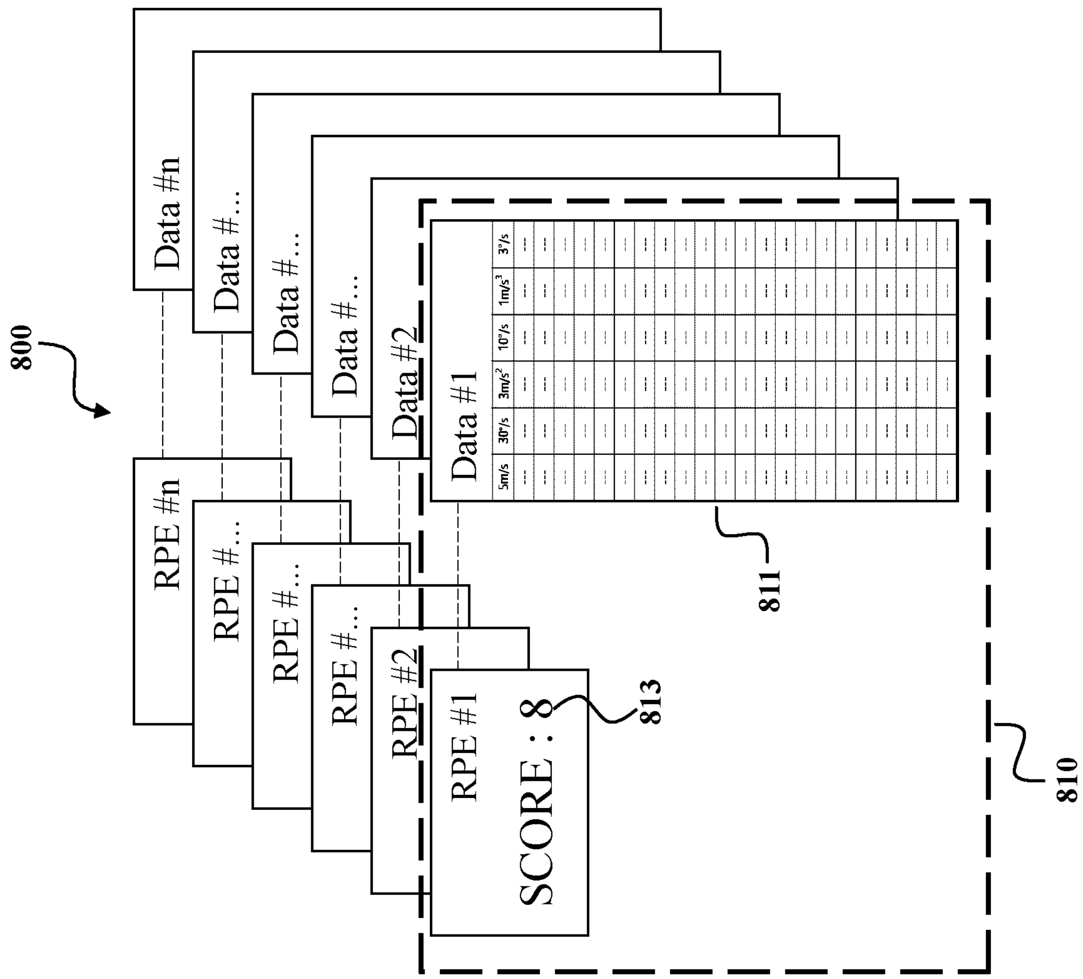
800
Data #n
Data #...
Data #...
Data #...
Data #2
Data #1
5m/s
30°/s
3m/s²
10°/s
1m/s³
3°/s
RPE #n
RPE #...
RPE #...
RPE #...
RPE #2
RPE #1
SCORE : 8
811
813
810

# FIG. 9

811

| # | \|V\| | Δθ(V) | \|A\| | Δθ(A) | \|J\| | Δθ(J) |
|---|---|---|---|---|---|---|
| 1 | 5m/s | 30°/s | $3m/s^2$ | 10°/s | $1m/s^3$ | 3°/s |
| --- | --- | --- | --- | --- | --- | --- |
| --- | --- | --- | --- | --- | --- | --- |
| --- | --- | --- | --- | --- | --- | --- |
| --- | --- | --- | --- | --- | --- | --- |
| --- | --- | --- | --- | --- | --- | --- |
| --- | --- | --- | --- | --- | --- | --- |
| --- | --- | --- | --- | --- | --- | --- |
| --- | --- | --- | --- | --- | --- | --- |
| --- | --- | --- | --- | --- | --- | --- |
| --- | --- | --- | --- | --- | --- | --- |
| --- | --- | --- | --- | --- | --- | --- |
| --- | --- | --- | --- | --- | --- | --- |
| --- | --- | --- | --- | --- | --- | --- |
| --- | --- | --- | --- | --- | --- | --- |
| --- | --- | --- | --- | --- | --- | --- |
| --- | --- | --- | --- | --- | --- | --- |
| --- | --- | --- | --- | --- | --- | --- |
| --- | --- | --- | --- | --- | --- | --- |
| --- | --- | --- | --- | --- | --- | --- |
| --- | --- | --- | --- | --- | --- | --- |
| --- | --- | --- | --- | --- | --- | --- |
| n | --- | --- | --- | --- | --- | --- |

811a

811n

# FIG. 10

| # | Vx | Vy | Ax | Ay | Zx | Zy |
|---|---|---|---|---|---|---|
| 1 | 0 | 0 | 0 | 0 | 0 | 0 |
| 2 | 0 | 0 | 0 | 0 | 0 | 0 |
| ... | ... | ... | ... | ... | ... | ... |
| 1,996 | 4.23 | 8.12 | 14.23 | -1.23 | -5.67 | 2.01 |
| 1,997 | 6.55 | 6.77 | 2.38 | -13.19 | -1.90 | 2.04 |
| 1,998 | 8.29 | 6.54 | 4.49 | -3.27 | 2.09 | 2.21 |
| 1,999 | 10.88 | 3.12 | -10.42 | 0.31 | 3.12 | 1.49 |
| 2,000 | 15.64 | -4.12 | -8.21 | -0.77 | 2.56 | -0.04 |

zero-padding

# FIG. 11

| # | Vx | Vy | Ax | Ay | Zx | Zy |
|---|---|---|---|---|---|---|
| 1 | 4.23 | 8.12 | 14.23 | -1.23 | -5.67 | 2.01 |
| 2 | 6.55 | 6.77 | 2.38 | -13.19 | -1.90 | 2.04 |
| 3 | 8.29 | 6.54 | 4.49 | -3.27 | 2.09 | 2.21 |
| 4 | 10.88 | 3.12 | -10.42 | 0.31 | 3.12 | 1.49 |
| 5 | 15.64 | -4.12 | -8.21 | -0.77 | 2.56 | -0.04 |
| ... | ... | ... | ... | ... | ... | ... |
| 1,999 | 0 | 0 | 0 | 0 | 0 | 0 |
| 2,000 | 0 | 0 | 0 | 0 | 0 | 0 |

zero-padding

FIG. 12

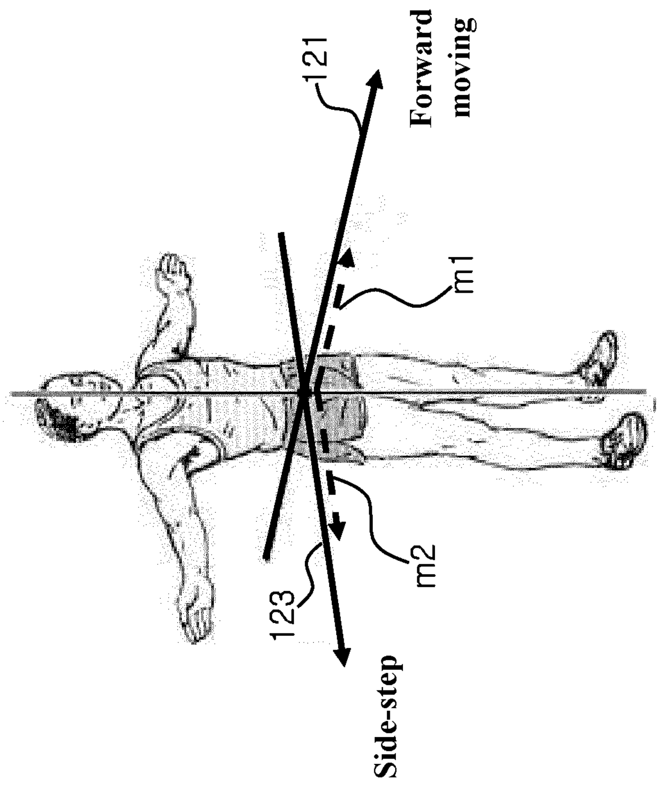

FIG. 13

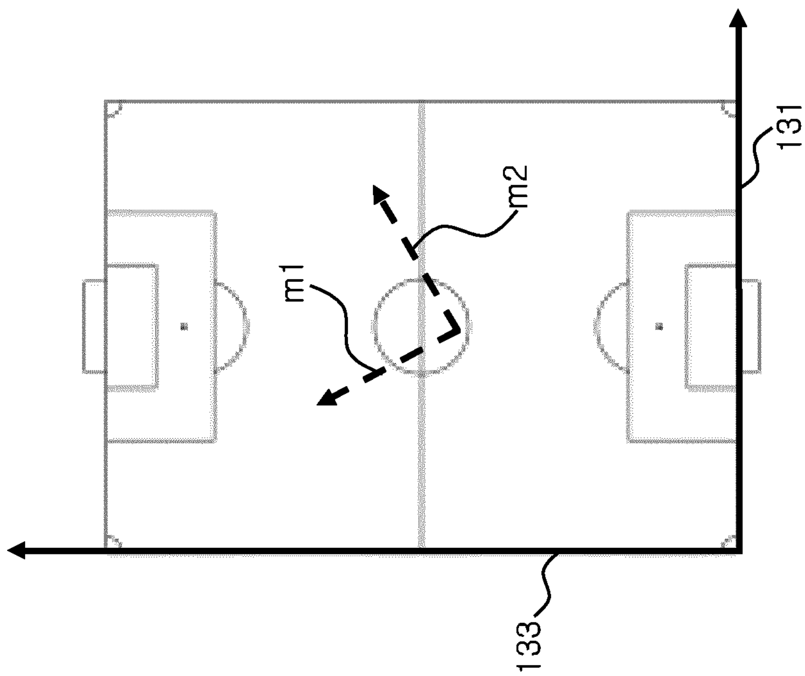

FIG. 14

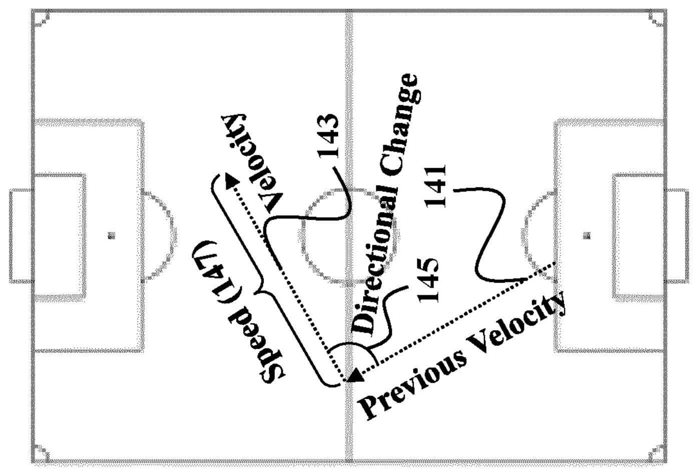
Velocity
143
Directional Change
141
145
Speed (147)
Previous Velocity

FIG. 15

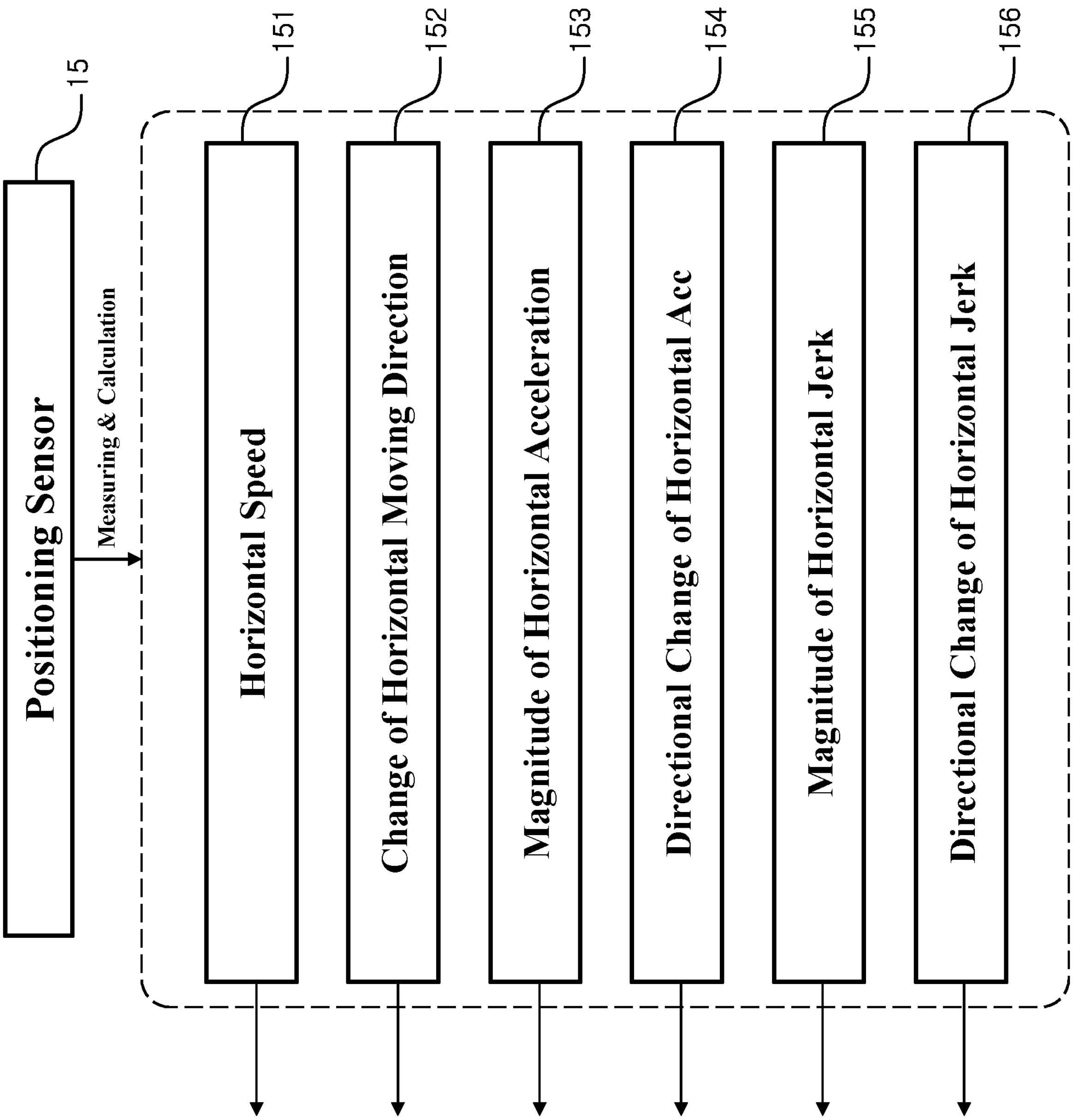


811

| # | \|V\| | Δθ(V) | \|A\| | Δθ(A) | \|J\| | Δθ(J) |
|---|---|---|---|---|---|---|
| 1 | 5m/s | 30°/s | $3m/s^2$ | 10°/s | $1m/s^3$ | 3°/s |
| --- | --- | --- | --- | --- | --- | --- |
| --- | --- | --- | --- | --- | --- | --- |
| --- | --- | --- | --- | --- | --- | --- |
| --- | --- | --- | --- | --- | --- | --- |

FIG. 16

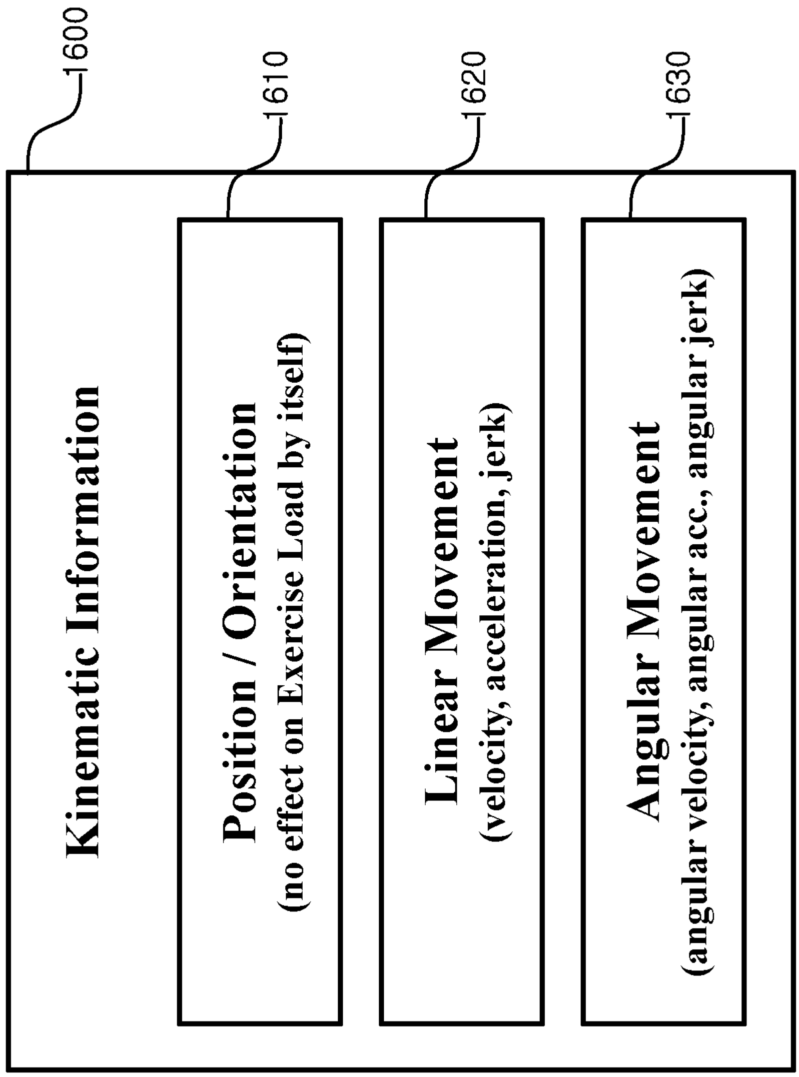
1600
Kinematic Information
1610
Position / Orientation
(no effect on Exercise Load by itself)
1620
Linear Movement
(velocity, acceleration, jerk)
1630
Angular Movement
(angular velocity, angular acc., angular jerk)

FIG. 17

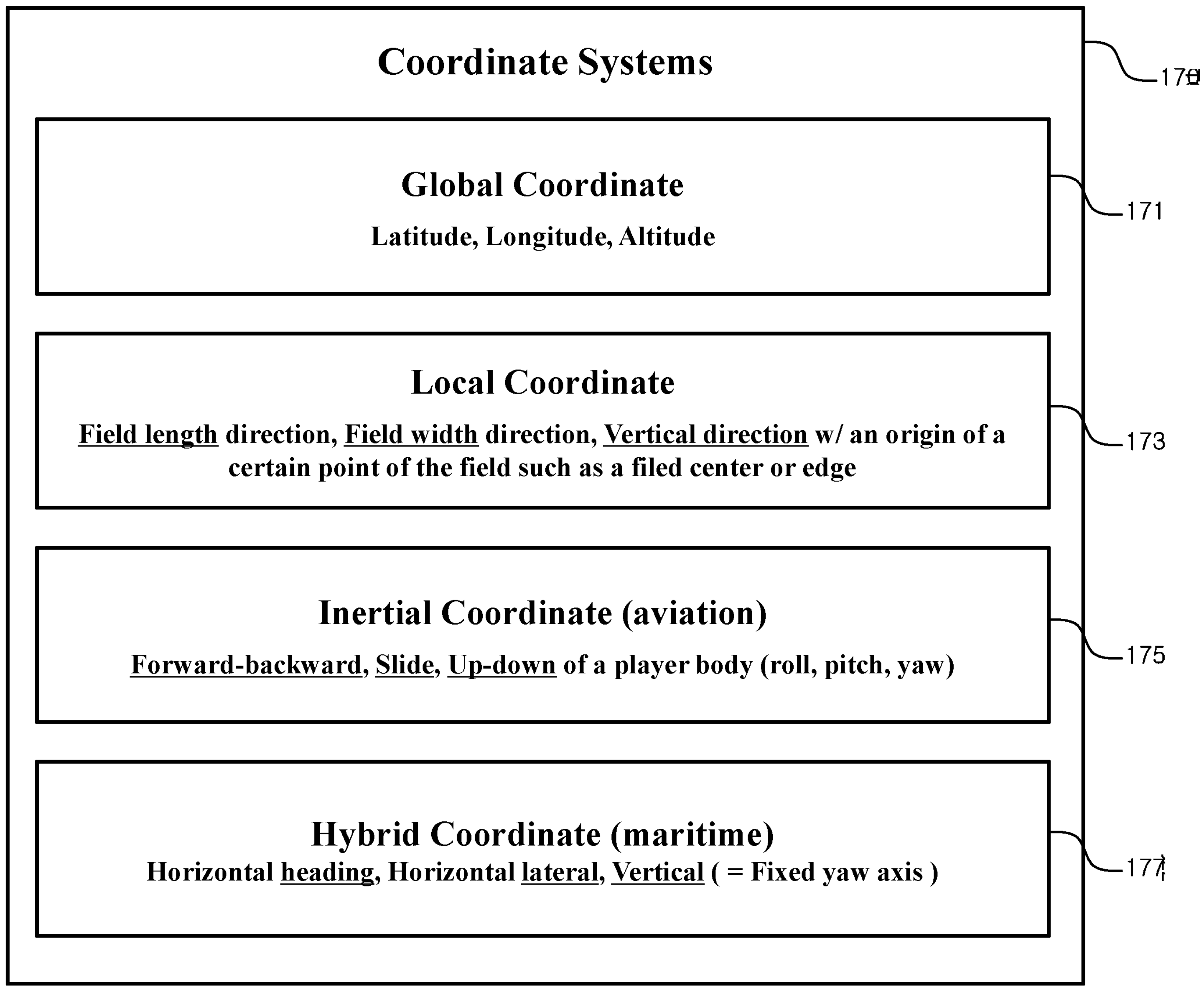
Coordinate Systems
170
Global Coordinate
Latitude, Longitude, Altitude
171
Local Coordinate
Field length direction, Field width direction, Vertical direction w/ an origin of a certain point of the field such as a filed center or edge
173
Inertial Coordinate (aviation)
Forward-backward, Slide, Up-down of a player body (roll, pitch, yaw)
175
Hybrid Coordinate (maritime)
Horizontal heading, Horizontal lateral, Vertical ( = Fixed yaw axis )
177

FIG. 18

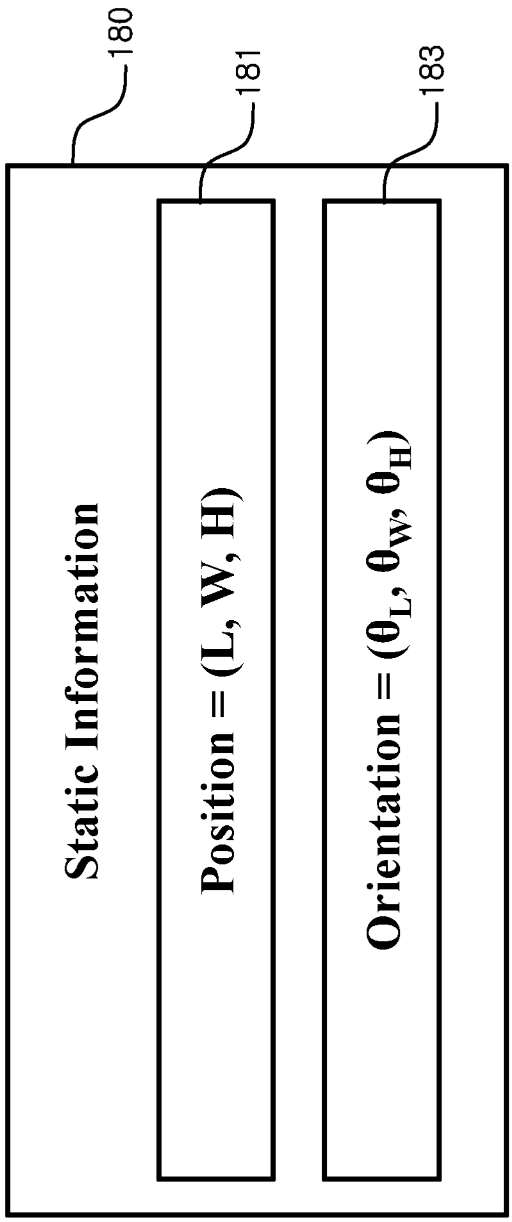

180
181
183
Static Information
Position = (L, W, H)
Orientation = ($\theta_L$, $\theta_W$, $\theta_H$)

FIG. 19

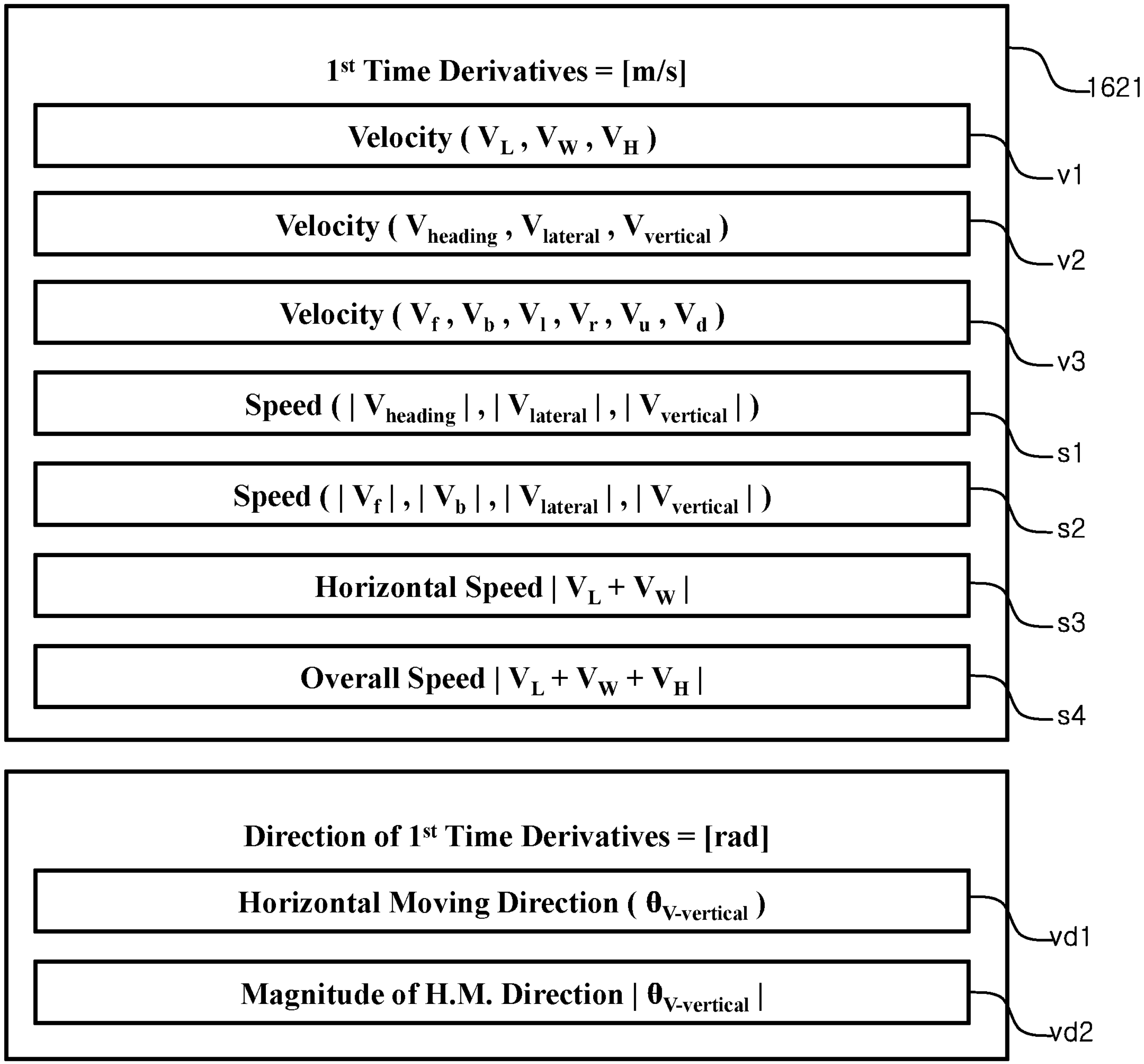
$1^{st}$ Time Derivatives = [m/s]
1621
Velocity ( $V_L$ , $V_W$ , $V_H$ )
v1
Velocity ( $V_{heading}$ , $V_{lateral}$ , $V_{vertical}$ )
v2
Velocity ( $V_f$ , $V_b$ , $V_l$ , $V_r$ , $V_u$ , $V_d$ )
v3
Speed ( $|V_{heading}|$ , $|V_{lateral}|$ , $|V_{vertical}|$ )
s1
Speed ( $|V_f|$ , $|V_b|$ , $|V_{lateral}|$ , $|V_{vertical}|$ )
s2
Horizontal Speed $|V_L + V_W|$
s3
Overall Speed $|V_L + V_W + V_H|$
s4
Direction of $1^{st}$ Time Derivatives = [rad]
Horizontal Moving Direction ( $\theta_{V\text{-}vertical}$ )
vd1
Magnitude of H.M. Direction $|\theta_{V\text{-}vertical}|$
vd2

FIG. 20

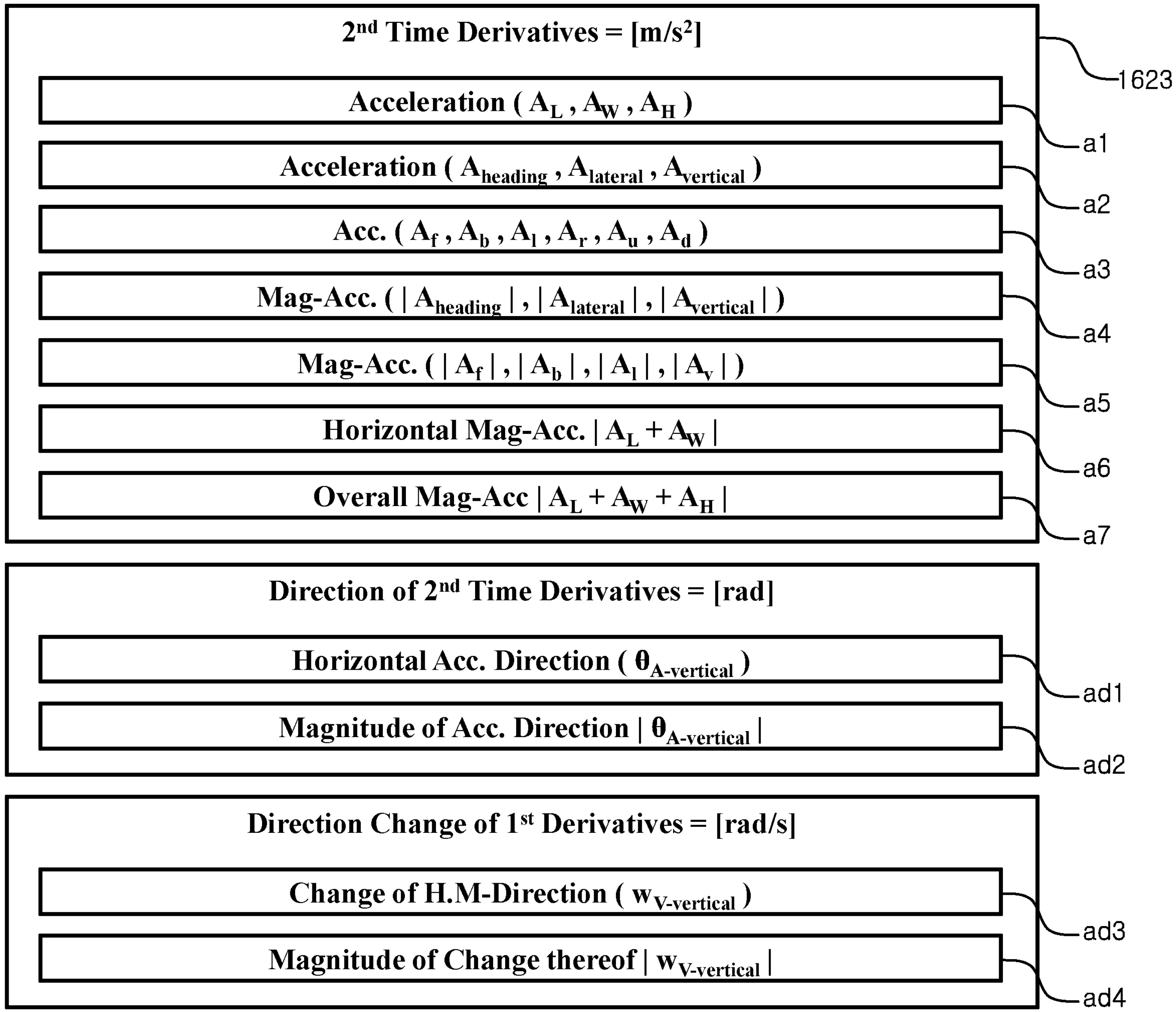
2nd Time Derivatives = [m/s²]
Acceleration ( $A_L$ , $A_W$ , $A_H$ )
Acceleration ( $A_{heading}$ , $A_{lateral}$ , $A_{vertical}$ )
Acc. ( $A_f$ , $A_b$ , $A_l$ , $A_r$ , $A_u$ , $A_d$ )
Mag-Acc. ( $|A_{heading}|$ , $|A_{lateral}|$ , $|A_{vertical}|$ )
Mag-Acc. ( $|A_f|$ , $|A_b|$ , $|A_l|$ , $|A_v|$ )
Horizontal Mag-Acc. $|A_L + A_W|$
Overall Mag-Acc $|A_L + A_W + A_H|$
Direction of 2nd Time Derivatives = [rad]
Horizontal Acc. Direction ( $\theta_{A\text{-}vertical}$ )
Magnitude of Acc. Direction $|\theta_{A\text{-}vertical}|$
Direction Change of 1st Derivatives = [rad/s]
Change of H.M-Direction ( $w_{V\text{-}vertical}$ )
Magnitude of Change thereof $|w_{V\text{-}vertical}|$
1623
a1
a2
a3
a4
a5
a6
a7
ad1
ad2
ad3
ad4

FIG. 21

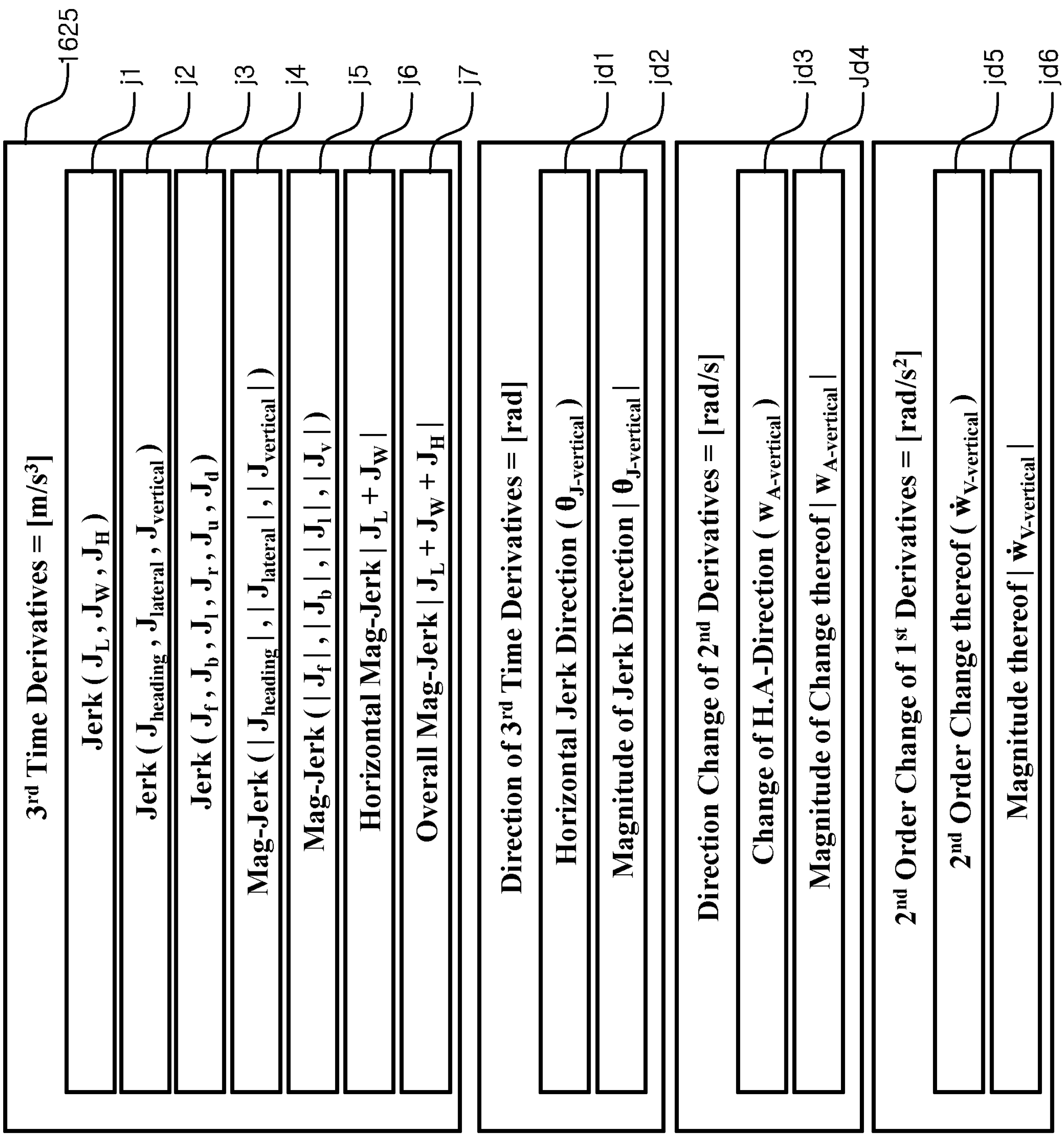
1625
$3^{rd}$ Time Derivatives = [$m/s^3$]
Jerk ( $J_L$ , $J_W$ , $J_H$ )
Jerk ( $J_{heading}$ , $J_{lateral}$ , $J_{vertical}$ )
Jerk ( $J_f$ , $J_b$ , $J_l$ , $J_r$ , $J_u$ , $J_d$ )
Mag-Jerk ( | $J_{heading}$ | , | $J_{lateral}$ | , | $J_{vertical}$ | )
Mag-Jerk ( | $J_f$ | , | $J_b$ | , | $J_l$ | , | $J_v$ | )
Horizontal Mag-Jerk | $J_L + J_W$ |
Overall Mag-Jerk | $J_L + J_W + J_H$ |
j1
j2
j3
j4
j5
j6
j7
Direction of $3^{rd}$ Time Derivatives = [rad]
Horizontal Jerk Direction ( $\theta_{J\text{-}vertical}$ )
Magnitude of Jerk Direction | $\theta_{J\text{-}vertical}$ |
jd1
jd2
Direction Change of $2^{nd}$ Derivatives = [rad/s]
Change of H.A-Direction ( $w_{A\text{-}vertical}$ )
Magnitude of Change thereof | $w_{A\text{-}vertical}$ |
jd3
Jd4
$2^{nd}$ Order Change of $1^{st}$ Derivatives = [$rad/s^2$]
$2^{nd}$ Order Change thereof ( $\dot{w}_{V\text{-}vertical}$ )
Magnitude thereof | $\dot{w}_{V\text{-}vertical}$ |
jd5
jd6

FIG. 22

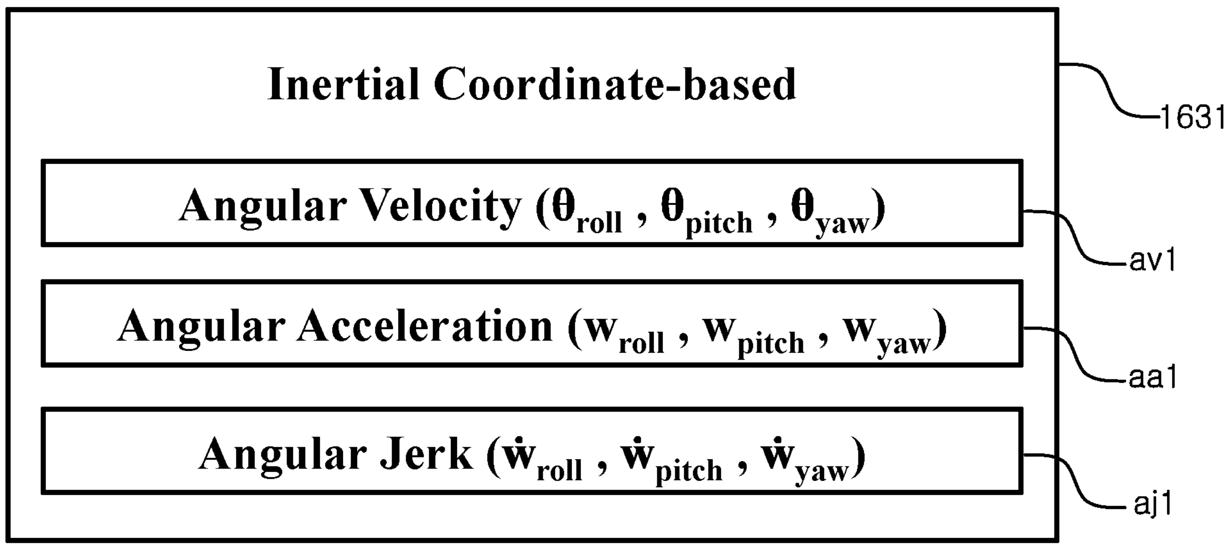
Inertial Coordinate-based
Angular Velocity ($\theta_{roll}$ , $\theta_{pitch}$ , $\theta_{yaw}$)
Angular Acceleration ($w_{roll}$ , $w_{pitch}$ , $w_{yaw}$)
Angular Jerk ($\dot{w}_{roll}$ , $\dot{w}_{pitch}$ , $\dot{w}_{yaw}$)
1631
av1
aa1
aj1

FIG. 23

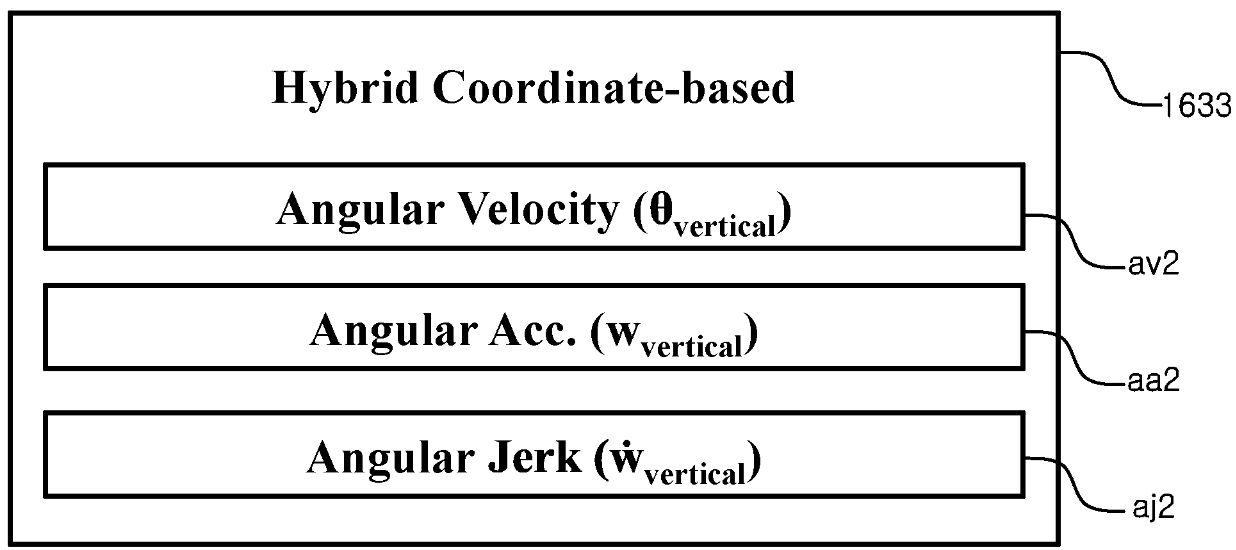
Hybrid Coordinate-based
Angular Velocity ($\theta_{vertical}$)
Angular Acc. ($w_{vertical}$)
Angular Jerk ($\dot{w}_{vertical}$)
1633
av2
aa2
aj2

FIG. 24

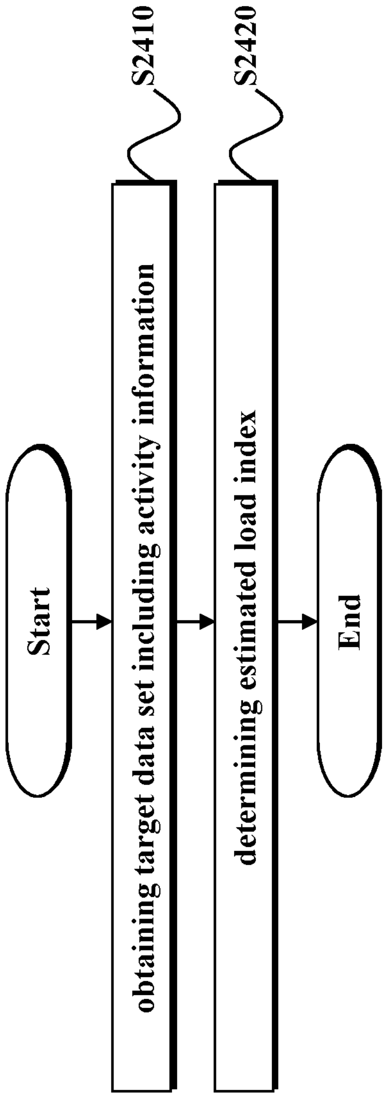
Start
obtaining target data set including activity information
S2410
determining estimated load index
S2420
End

FIG. 25

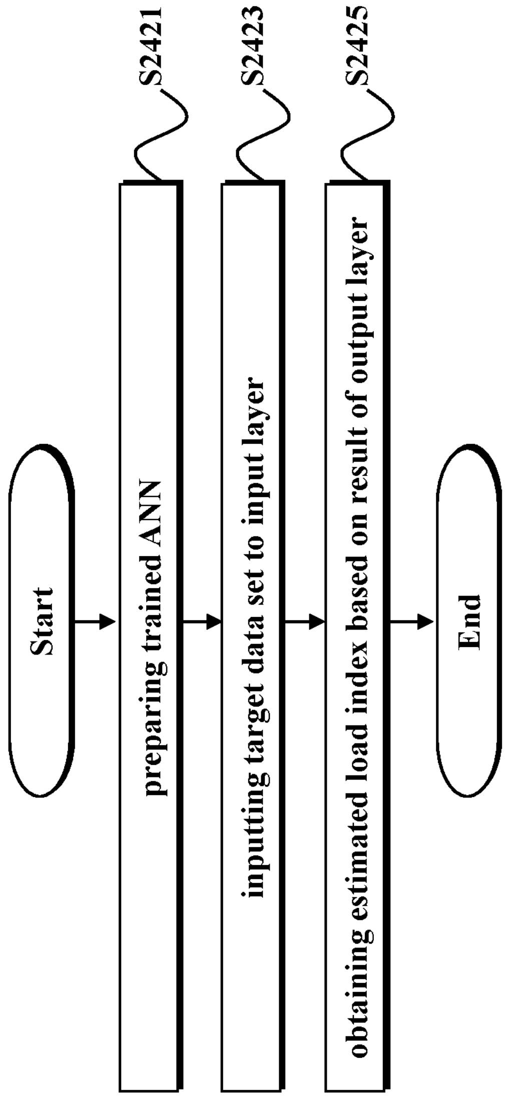
Start
preparing trained ANN
S2421
inputting target data set to input layer
S2423
obtaining estimated load index based on result of output layer
S2425
End

FIG. 26

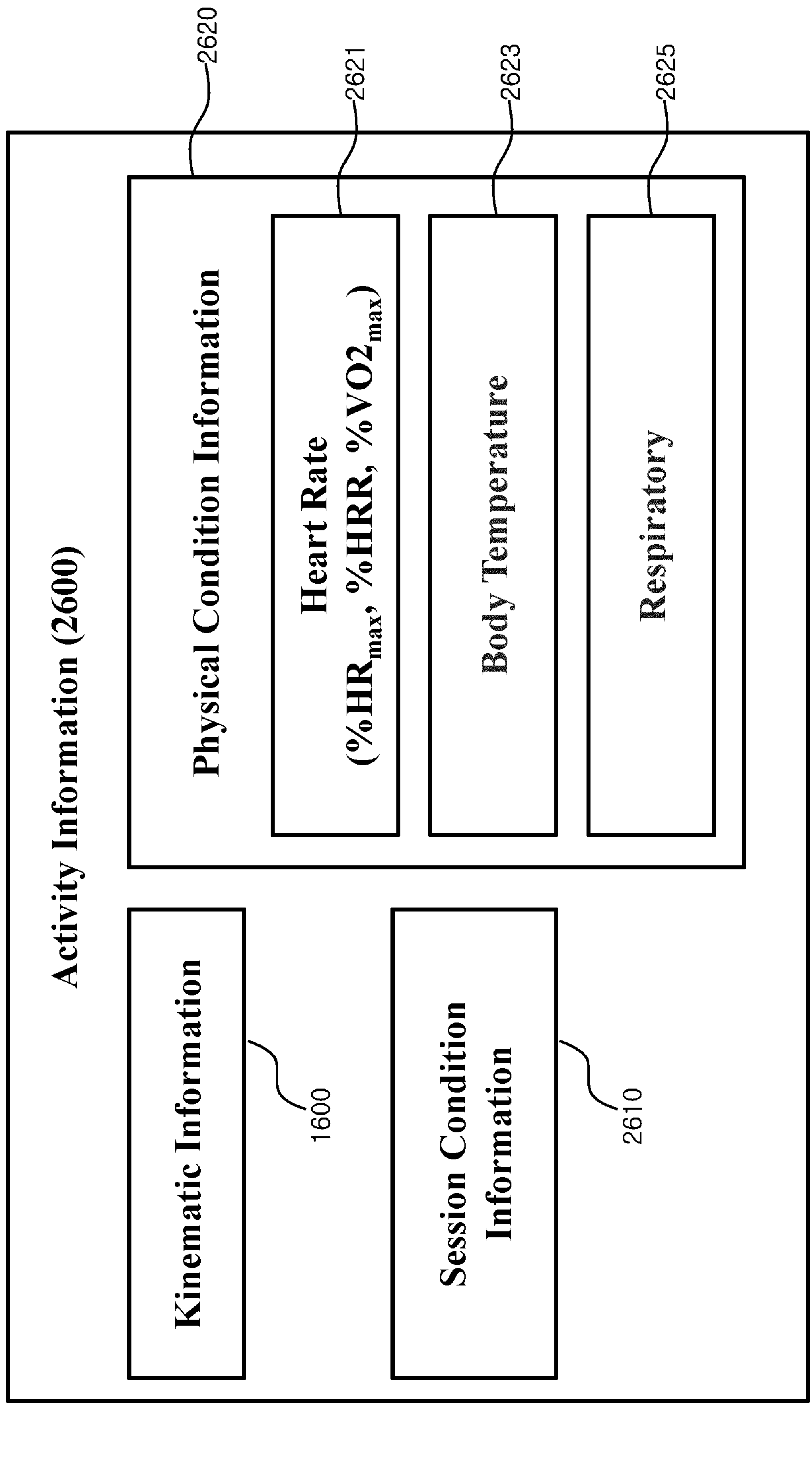
Activity Information (2600)
Kinematic Information
1600
Session Condition Information
2610
Physical Condition Information
2620
Heart Rate (%HR$_{max}$, %HRR, %VO2$_{max}$)
2621
Body Temperature
2623
Respiratory
2625

FIG. 27

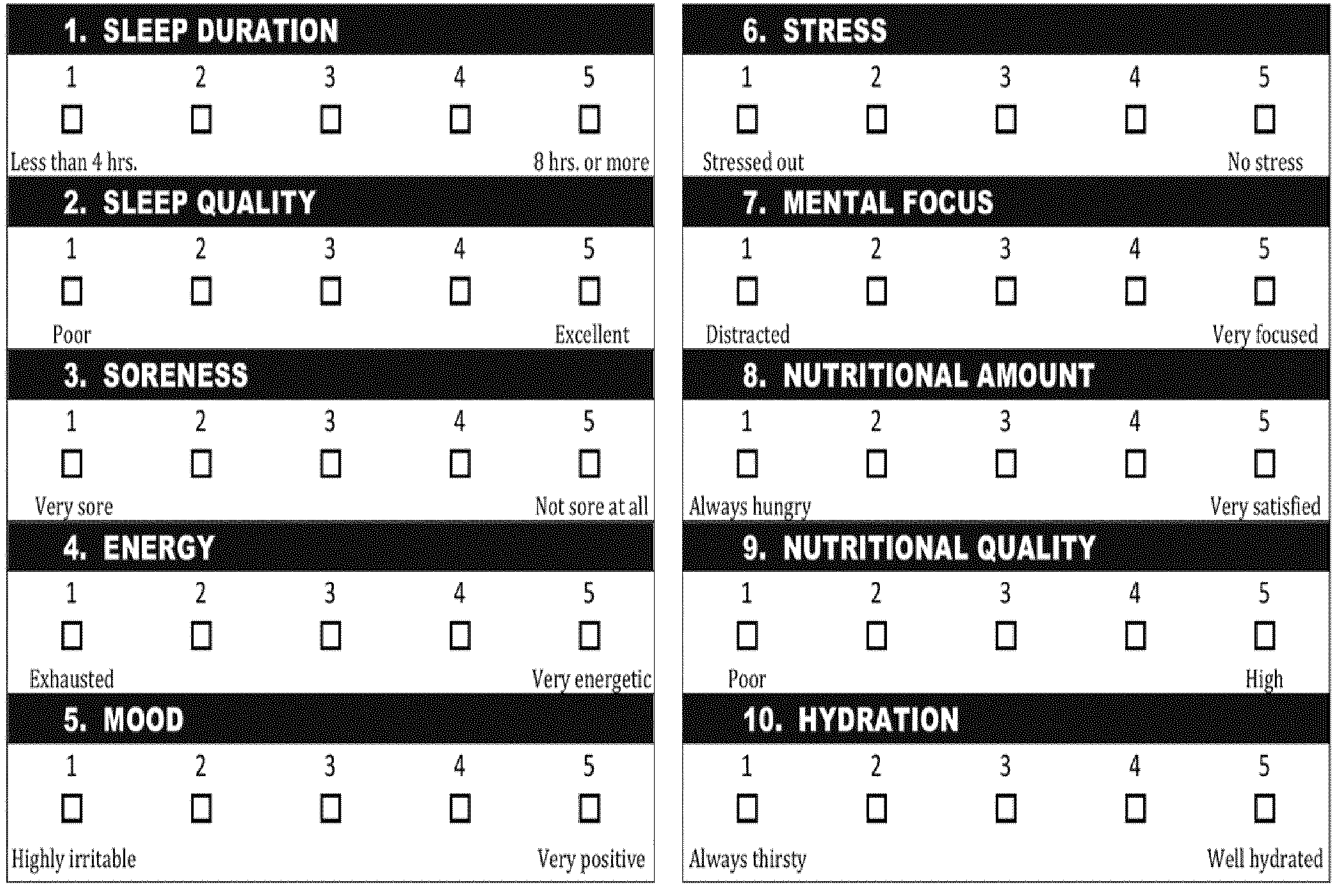
1. SLEEP DURATION
1 2 3 4 5
Less than 4 hrs.
8 hrs. or more
2. SLEEP QUALITY
1 2 3 4 5
Poor
Excellent
3. SORENESS
1 2 3 4 5
Very sore
Not sore at all
4. ENERGY
1 2 3 4 5
Exhausted
Very energetic
5. MOOD
1 2 3 4 5
Highly irritable
Very positive
6. STRESS
1 2 3 4 5
Stressed out
No stress
7. MENTAL FOCUS
1 2 3 4 5
Distracted
Very focused
8. NUTRITIONAL AMOUNT
1 2 3 4 5
Always hungry
Very satisfied
9. NUTRITIONAL QUALITY
1 2 3 4 5
Poor
High
10. HYDRATION
1 2 3 4 5
Always thirsty
Well hydrated

FIG. 28

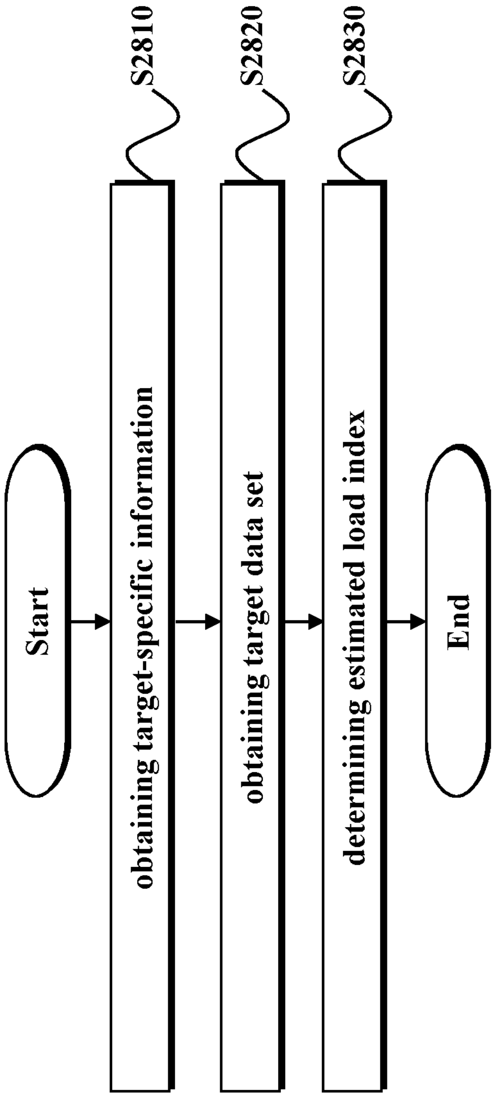
Start
obtaining target-specific information
S2810
obtaining target data set
S2820
determining estimated load index
S2830
End

FIG. 29

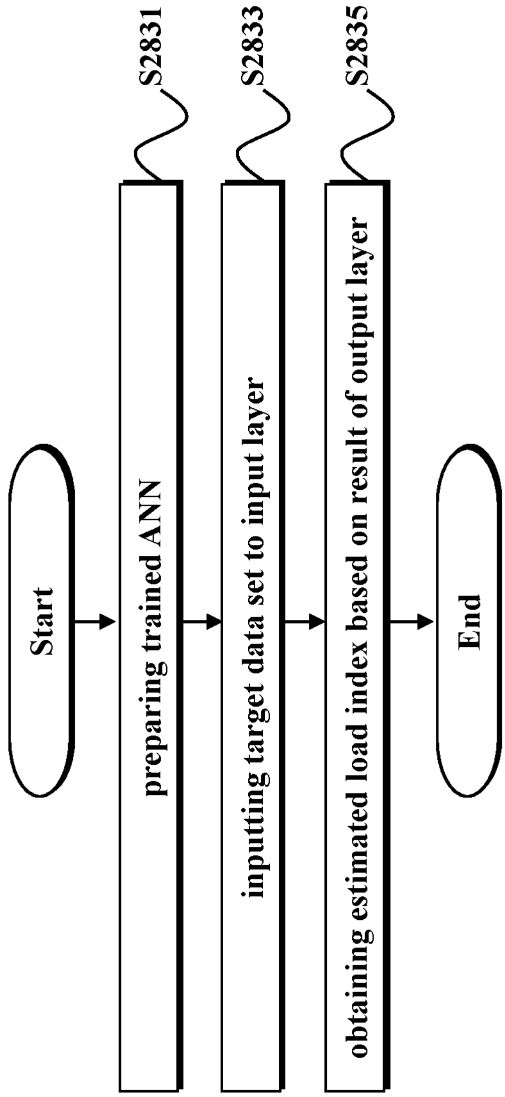
Start
preparing trained ANN
S2831
inputting target data set to input layer
S2833
obtaining estimated load index based on result of output layer
S2835
End

FIG. 30

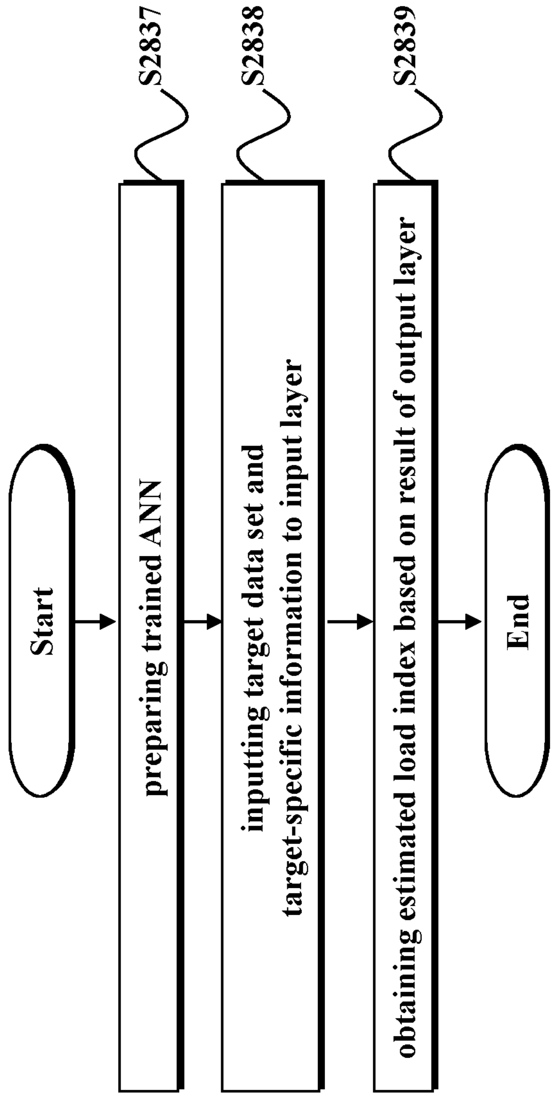
Start
preparing trained ANN
S2837
inputting target data set and
target-specific information to input layer
S2838
obtaining estimated load index based on result of output layer
S2839
End

FIG. 31

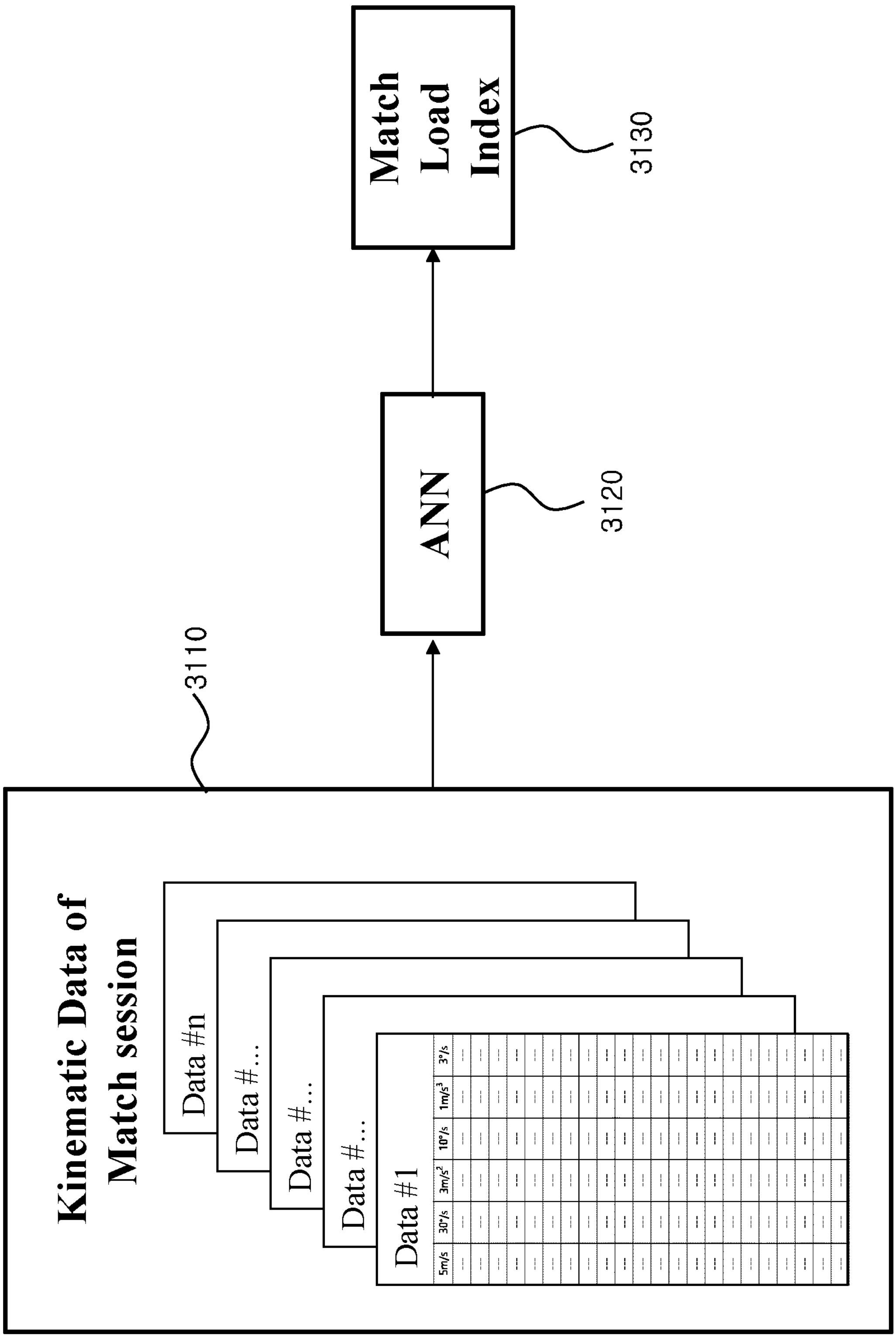
Kinematic Data of Match session
Data #n
Data #...
Data #...
Data #...
Data #1
3110
ANN
3120
Match Load Index
3130

FIG. 32

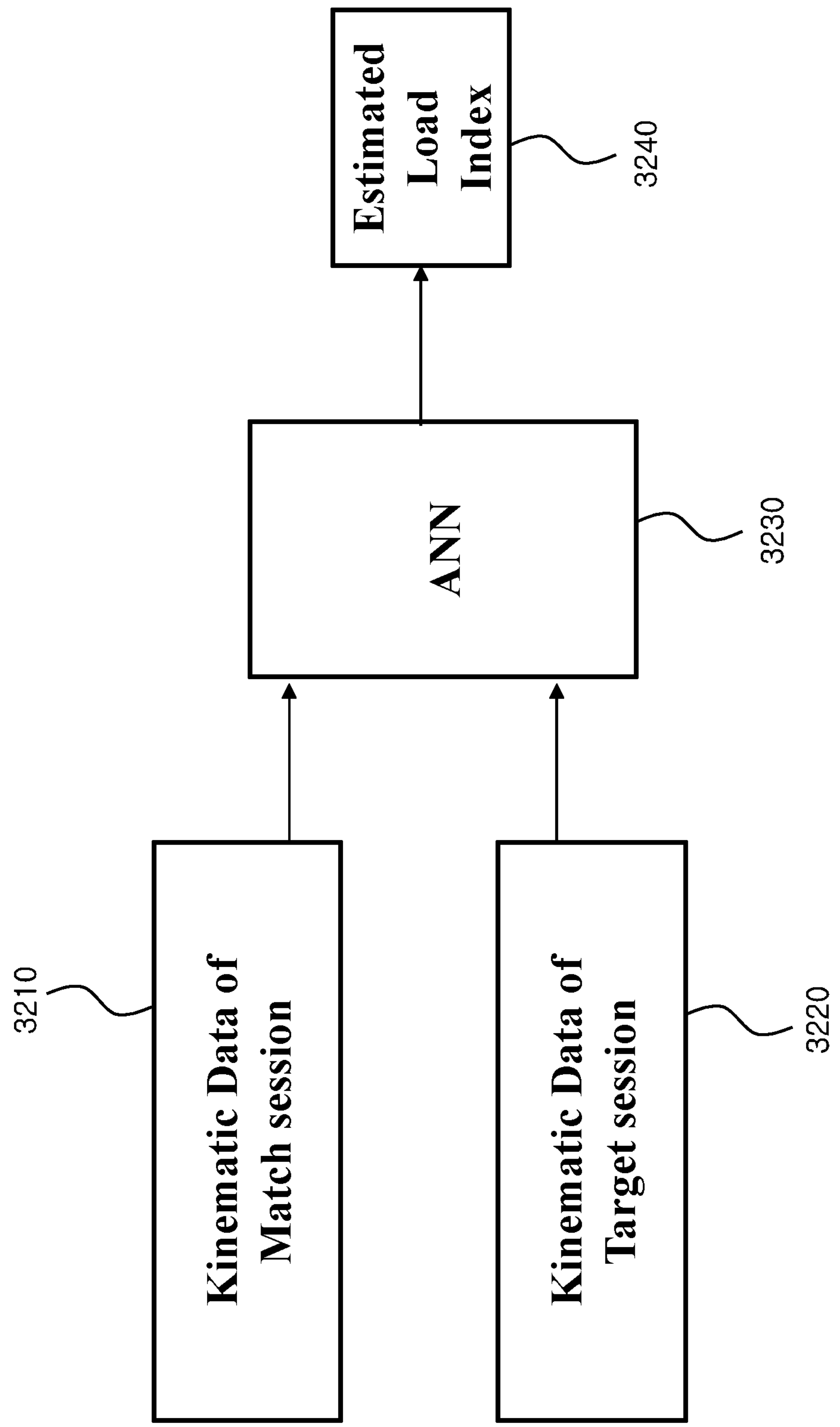
Kinematic Data of Match session
3210
Kinematic Data of Target session
3220
ANN
3230
Estimated Load Index
3240

FIG. 33

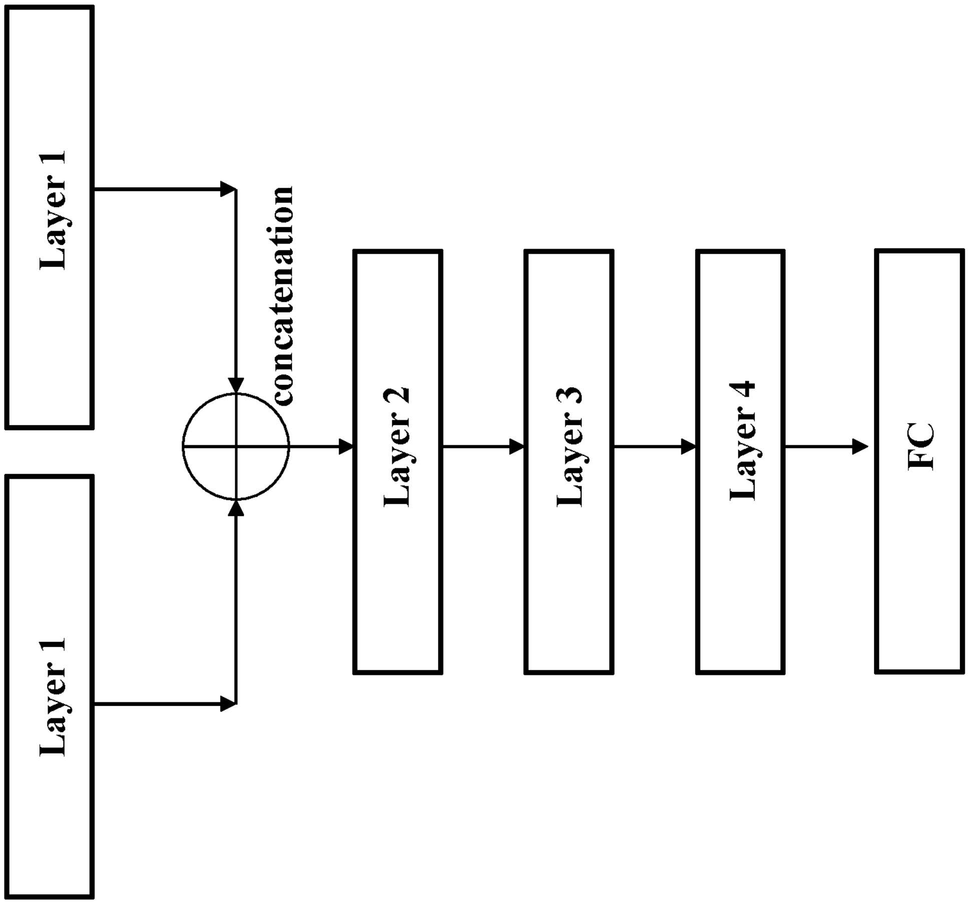

FIG. 34

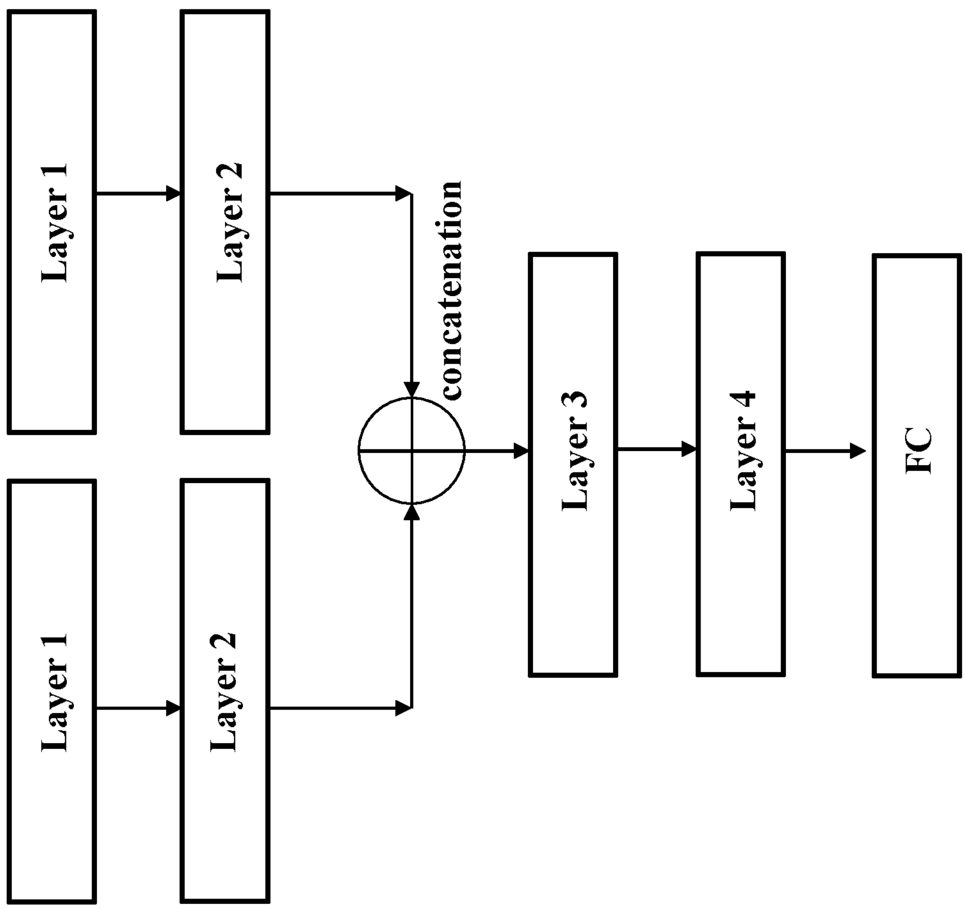
Layer 1
Layer 2
Layer 1
Layer 2
concatenation
Layer 3
Layer 4
FC

FIG. 35

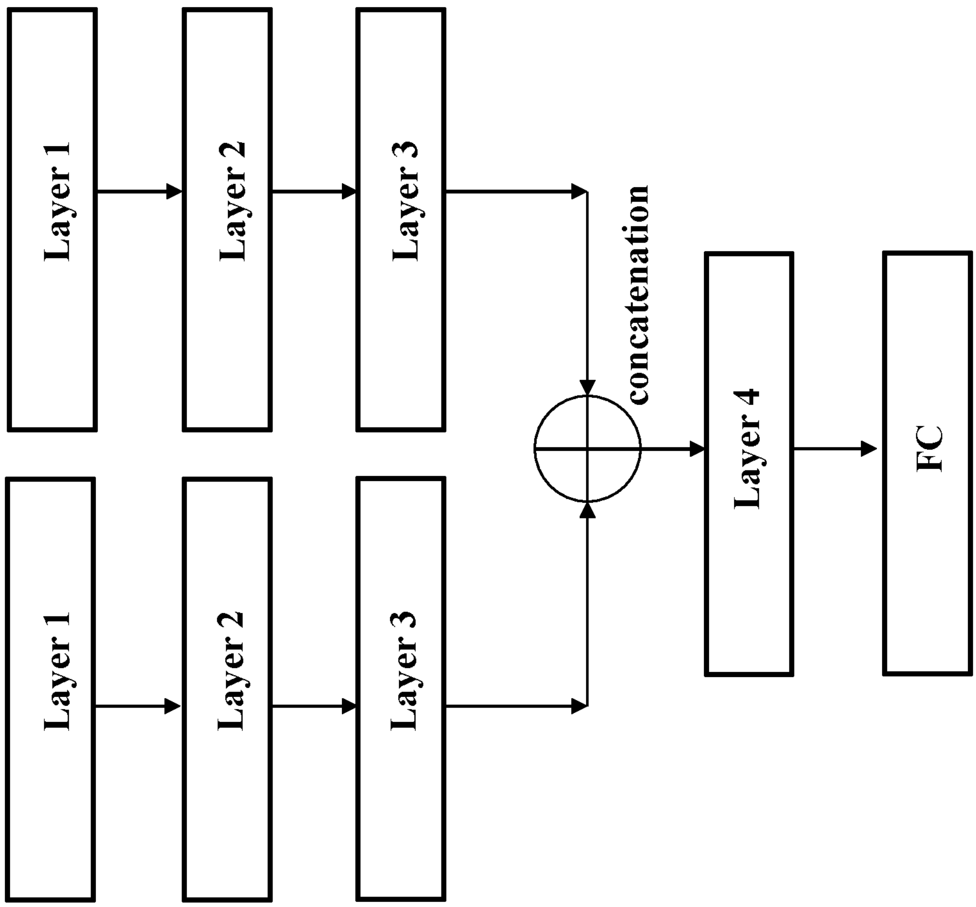
Layer 1
Layer 2
Layer 3
Layer 1
Layer 2
Layer 3
concatenation
Layer 4
FC

FIG. 36

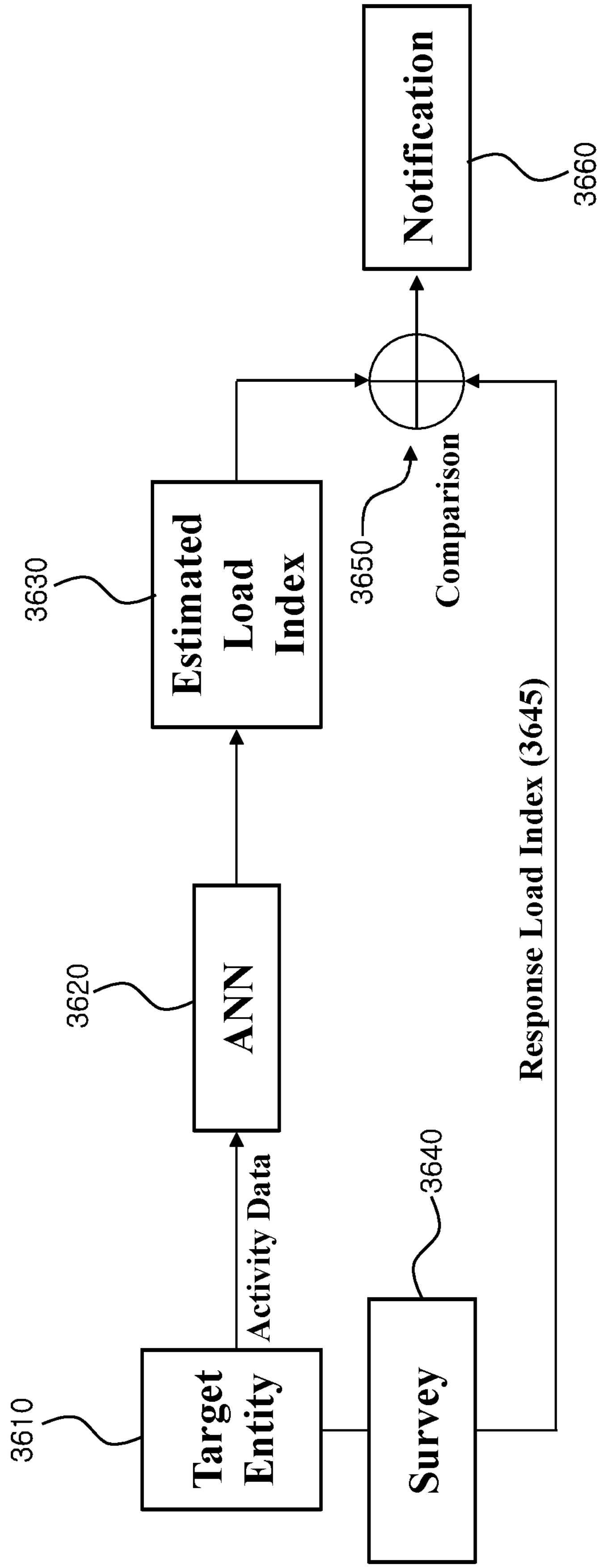
Target Entity
3610
Activity Data
ANN
3620
Estimated Load Index
3630
Comparison
3650
Notification
3660
Survey
3640
Response Load Index (3645)

FIG. 37

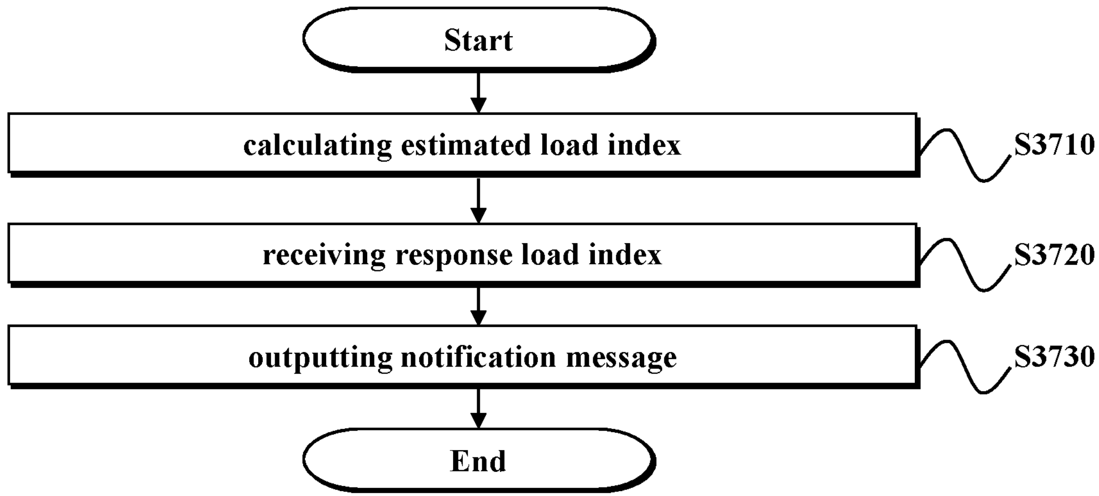
Start
calculating estimated load index
S3710
receiving response load index
S3720
outputting notification message
S3730
End

FIG. 38

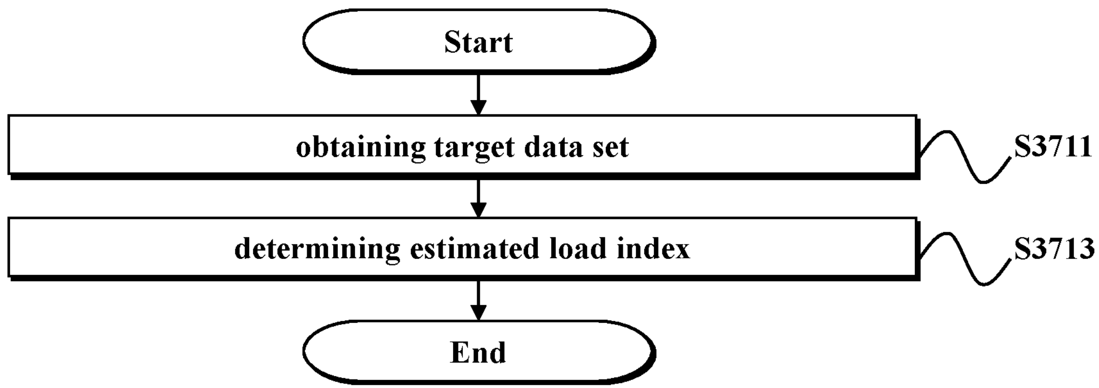
Start
obtaining target data set
S3711
determining estimated load index
S3713
End

FIG. 39

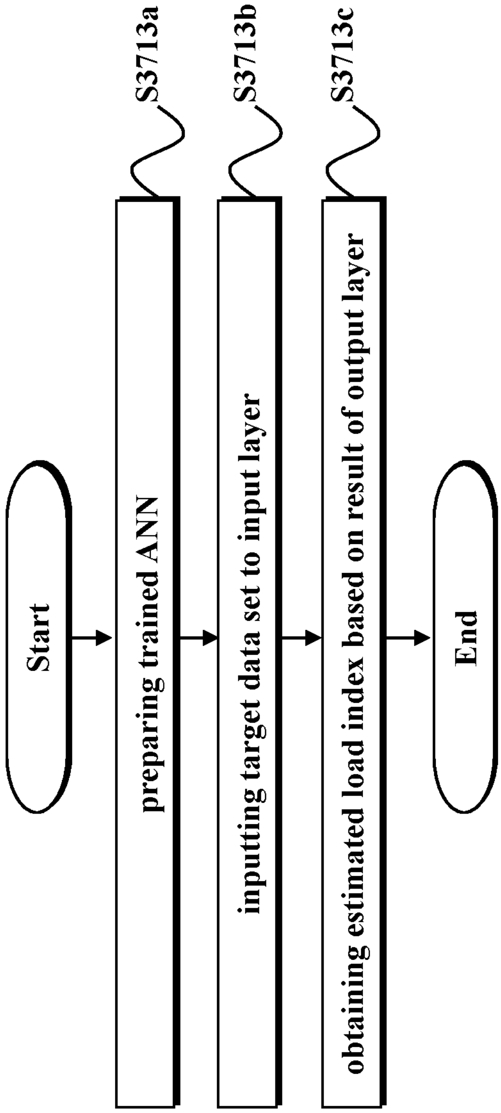
Start
preparing trained ANN
S3713a
inputting target data set to input layer
S3713b
obtaining estimated load index based on result of output layer
S3713c
End

FIG. 40

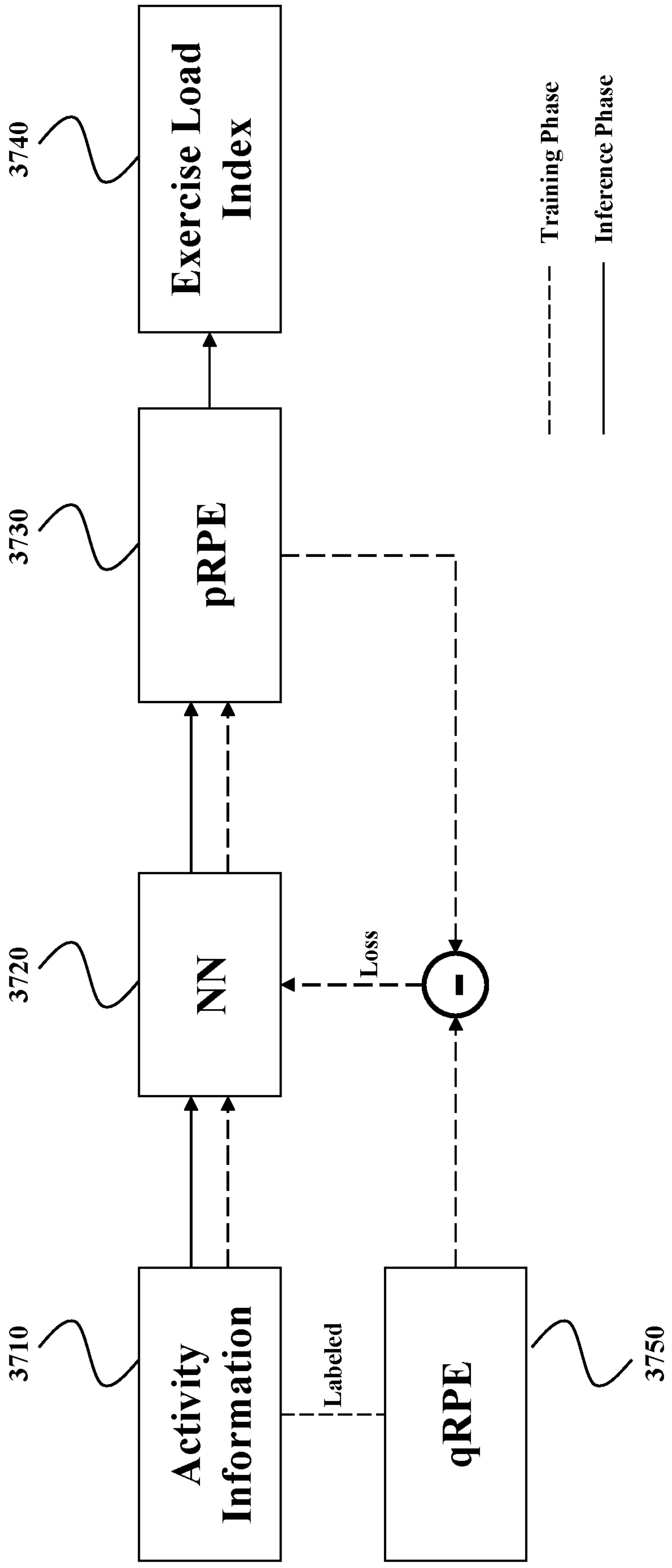
3740
Exercise Load Index
3730
pRPE
3720
NN
3710
Activity Information
3750
qRPE
Labeled
Loss
-
Training Phase
Inference Phase

FIG. 41

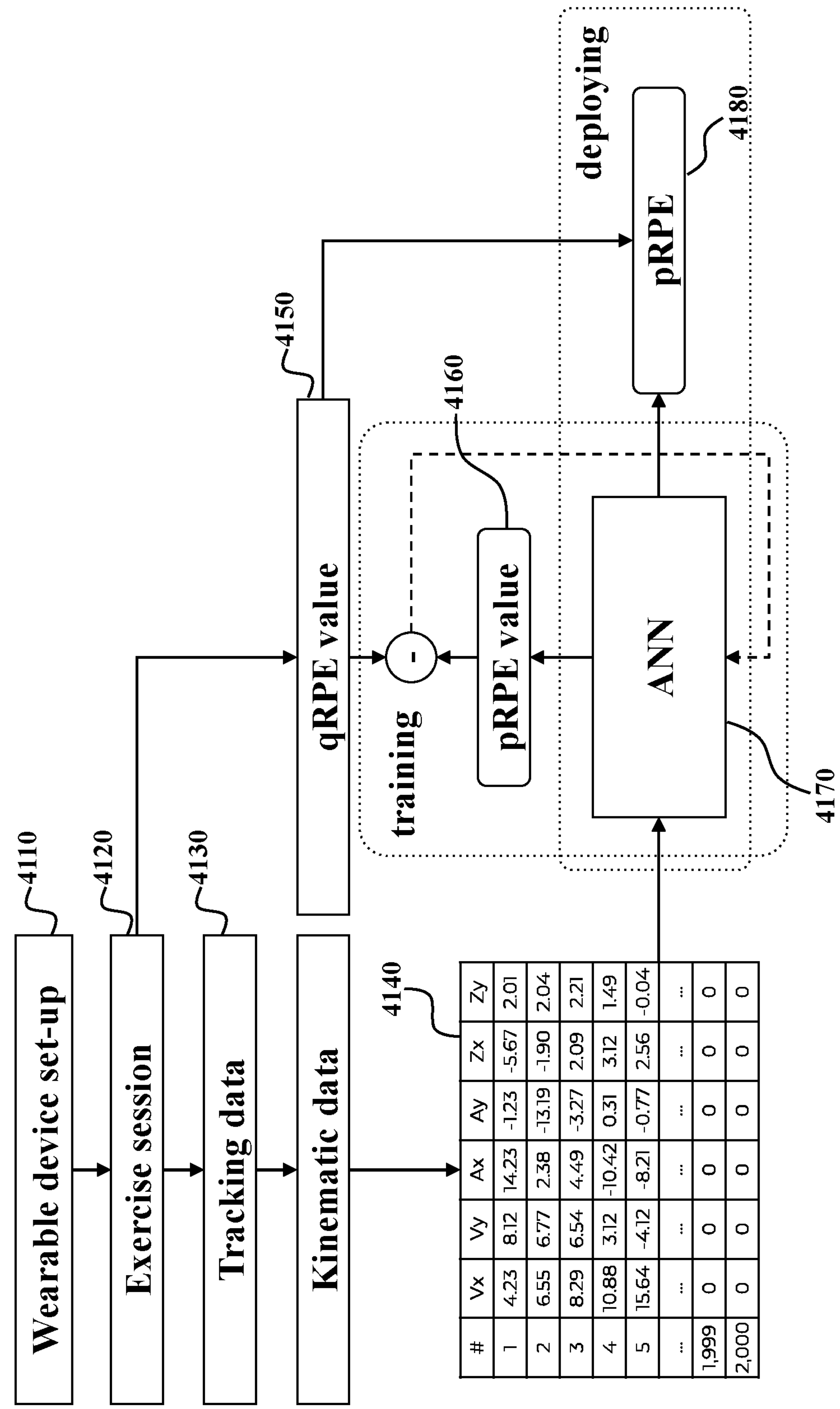


| # | Vx | Vy | Ax | Ay | Zx | Zy |
|---|---|---|---|---|---|---|
| 1 | 4.23 | 8.12 | 14.23 | -1.23 | -5.67 | 2.01 |
| 2 | 6.55 | 6.77 | 2.38 | -13.19 | -1.90 | 2.04 |
| 3 | 8.29 | 6.54 | 4.49 | -3.27 | 2.09 | 2.21 |
| 4 | 10.88 | 3.12 | -10.42 | 0.31 | 3.12 | 1.49 |
| 5 | 15.64 | -4.12 | -8.21 | -0.77 | 2.56 | -0.04 |
| ... | ... | ... | ... | ... | ... | ... |
| 1,999 | 0 | 0 | 0 | 0 | 0 | 0 |
| 2,000 | 0 | 0 | 0 | 0 | 0 | 0 |

FIG. 42

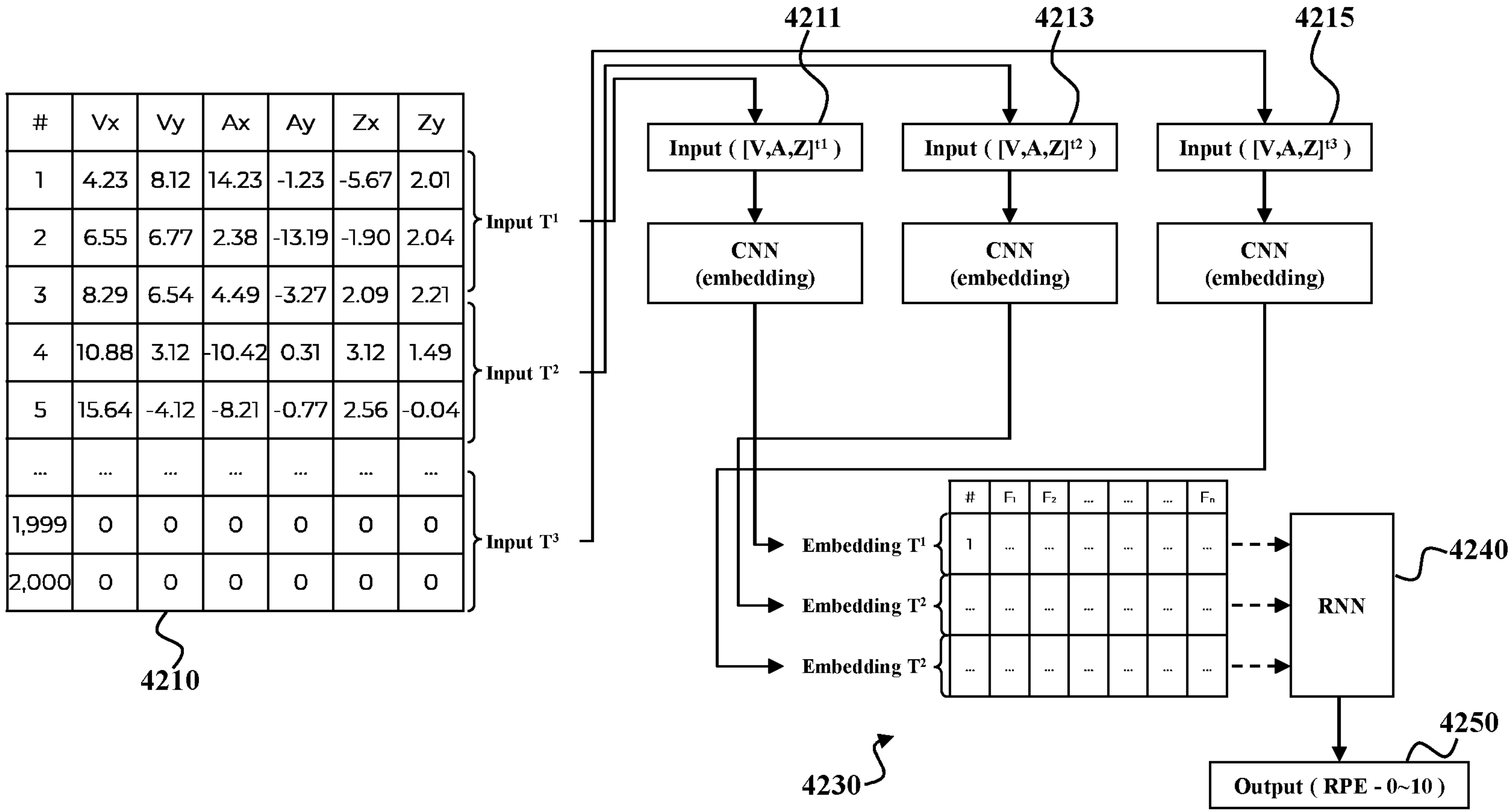

| # | Vx | Vy | Ax | Ay | Zx | Zy |
| 1 | 4.23 | 8.12 | 14.23 | -1.23 | -5.67 | 2.01 |
| 2 | 6.55 | 6.77 | 2.38 | -13.19 | -1.90 | 2.04 |
| 3 | 8.29 | 6.54 | 4.49 | -3.27 | 2.09 | 2.21 |
| 4 | 10.88 | 3.12 | -10.42 | 0.31 | 3.12 | 1.49 |
| 5 | 15.64 | -4.12 | -8.21 | -0.77 | 2.56 | -0.04 |
| ... | ... | ... | ... | ... | ... | ... |
| 1,999 | 0 | 0 | 0 | 0 | 0 | 0 |
| 2,000 | 0 | 0 | 0 | 0 | 0 | 0 |
4210
Input $T^1$
Input $T^2$
Input $T^3$
4211
4213
4215
Input ( $[V,A,Z]^{t1}$ )
Input ( $[V,A,Z]^{t2}$ )
Input ( $[V,A,Z]^{t3}$ )
CNN (embedding)
CNN (embedding)
CNN (embedding)
Embedding $T^1$
Embedding $T^2$
Embedding $T^2$
# F₁ F₂ ... ... ... Fₙ
1
4230
RNN
4240
Output ( RPE - 0~10 )
4250

FIG. 43

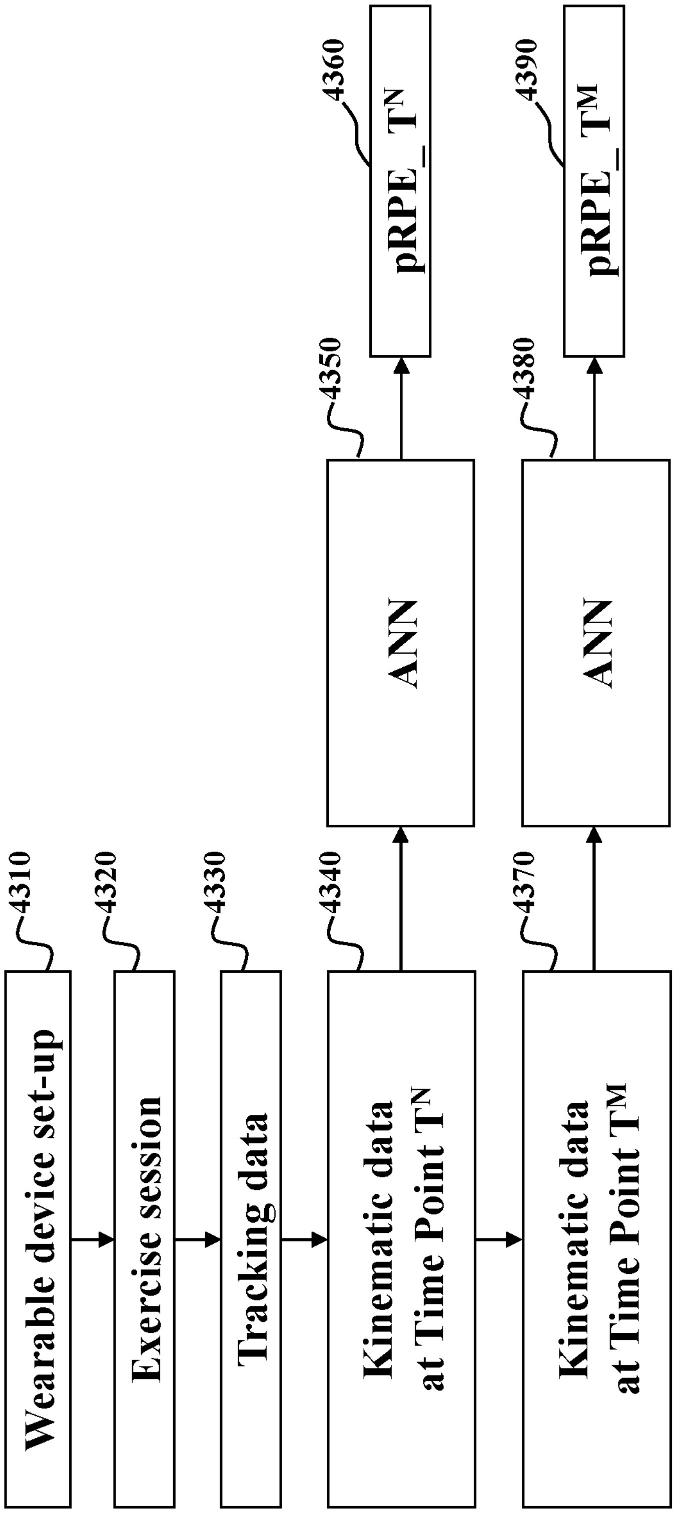
Wearable device set-up
4310
Exercise session
4320
Tracking data
4330
Kinematic data at Time Point T^N
4340
ANN
4350
pRPE_ T^N
4360
Kinematic data at Time Point T^M
4370
ANN
4380
pRPE_ T^M
4390

# FIG. 44

4340

| Kinematic data _ $T^N$ | | | | | | |
|---|---|---|---|---|---|---|
| # | Vx | Vy | Ax | Ay | Zx | Zy |
| 1 | 4.23 | 8.12 | 14.23 | -1.23 | -5.67 | 2.01 |
| ... | ... | ... | ... | ... | ... | ... |
| N | 8.29 | 6.54 | 4.49 | -3.27 | 2.09 | 2.21 |
| N+1 | 0 | 0 | 0 | 0 | 0 | 0 |
| N+2 | 0 | 0 | 0 | 0 | 0 | 0 |
| ... | ... | ... | ... | ... | ... | ... |
| 1,999 | 0 | 0 | 0 | 0 | 0 | 0 |
| 2,000 | 0 | 0 | 0 | 0 | 0 | 0 |

**FIG. 45**

4370

| Kinematic data _ at $T^M$ | | | | | | |
|---|---|---|---|---|---|---|
| # | Vx | Vy | Ax | Ay | Zx | Zy |
| 1 | 4.23 | 8.12 | 14.23 | -1.23 | -5.67 | 2.01 |
| ... | ... | ... | ... | ... | ... | ... |
| N | 8.29 | 6.54 | 4.49 | -3.27 | 2.09 | 2.21 |
| ... | ... | ... | ... | ... | ... | ... |
| M | 10.88 | 3.12 | -10.42 | 0.31 | 3.12 | 1.49 |
| M+1 | 0 | 0 | 0 | 0 | 0 | 0 |
| ... | ... | ... | ... | ... | ... | ... |
| 2,000 | 0 | 0 | 0 | 0 | 0 | 0 |

# FIG. 46

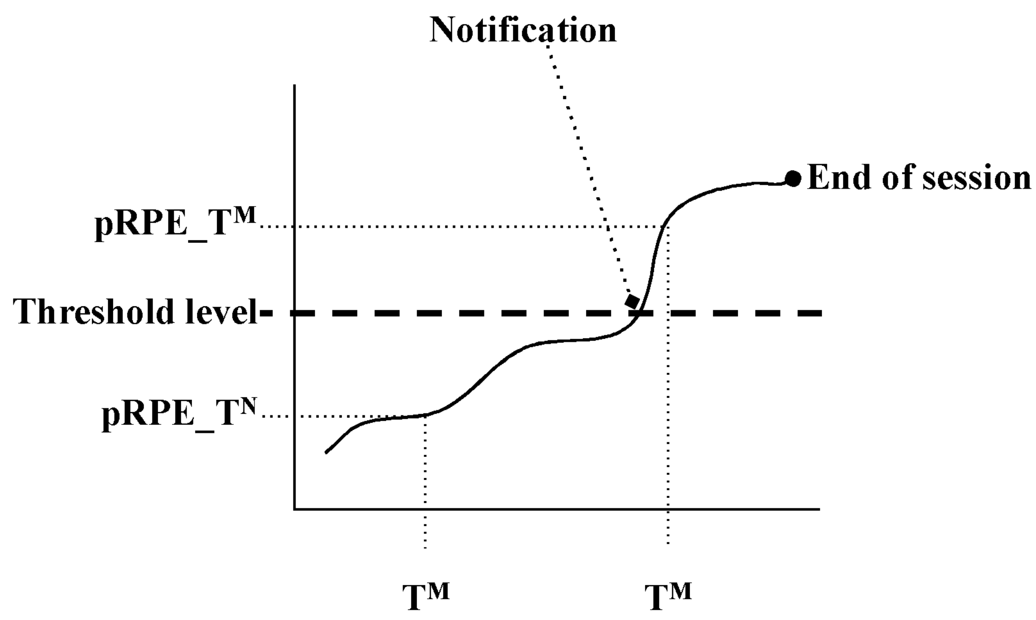
Notification
End of session
pRPE_T^M
Threshold level
pRPE_T^N
T^M
T^M

## INTERNATIONAL SEARCH REPORT

International application No.
**PCT/KR2022/012766**

**A. CLASSIFICATION OF SUBJECT MATTER**

**G16H 50/50**(2018.01)i; **G16H 50/70**(2018.01)i; **G16H 50/20**(2018.01)i; **G16H 50/30**(2018.01)i; **G06N 3/08**(2006.01)i; **A61B 5/11**(2006.01)i; **A61B 5/00**(2006.01)i

According to International Patent Classification (IPC) or to both national classification and IPC

**B. FIELDS SEARCHED**

Minimum documentation searched (classification system followed by classification symbols)

G16H 50/50(2018.01); A61B 5/00(2006.01); A61B 5/11(2006.01); A63B 24/00(2006.01); A63B 69/00(2006.01); A63B 71/06(2006.01); G06K 9/00(2006.01); G09B 19/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 운동학적 정보(kinematic information), 추정 부하 인덱스(estimated load index), 인공 신경망(artificial neural network), 포지셔닝 디바이스(positioning device), 속도(velocity), 저크(jerk)

**C. DOCUMENTS CONSIDERED TO BE RELEVANT**

| Category* | Citation of document, with indication, where appropriate, of the relevant passages | Relevant to claim No. |
|---|---|---|
| Y | KR 10-2020-0087436 A (POSTECH RESEARCH AND BUSINESS DEVELOPMENT FOUNDATION) 21 July 2020 (2020-07-21) See paragraphs [0037]-[0068] and claims 1-2 and 8. | 1-20 |
| Y | KR 10-2019-0100447 A (NIKE INNOVATE C.V.) 28 August 2019 (2019-08-28) See paragraph [0070] and claim 1. | 1-20 |
| Y | KR 10-2020-0109199 A (P&C SOLUTION) 22 September 2020 (2020-09-22) See paragraph [0055] and claim 1. | 1-20 |
| Y | JP 2014-014683 A (ADIDAS AG) 30 January 2014 (2014-01-30) See paragraphs [0052]-[0162] and claim 1. | 2,14-15 |
| Y | KR 10-2020-0122328 A (F5 SPORTS, INC.) 27 October 2020 (2020-10-27) See claim 8. | 7,11-13 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 November 2022** | **22 November 2022** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.
**PCT/KR2022/012766**

| Patent document cited in search report | Publication date (day/month/year) | Patent family member(s) | Publication date (day/month/year) |
|---|---|---|---|
| KR 10-2020-0087436 A | 21 July 2020 | KR 10-2163240 B1 | 08 October 2020 |
| KR 10-2019-0100447 A | 28 August 2019 | CN 107205661 A | 26 September 2017 |
| | | CN 107205661 B | 09 March 2021 |
| | | EP 3242595 A1 | 15 November 2017 |
| | | EP 3242595 B1 | 23 March 2022 |
| | | JP 2018-501889 A | 25 January 2018 |
| | | JP 2020-018862 A | 06 February 2020 |
| | | JP 7015812 B2 | 03 February 2022 |
| | | KR 10-2017-0101996 A | 06 September 2017 |
| | | KR 10-2303952 B1 | 24 September 2021 |
| | | US 10803090 B2 | 13 October 2020 |
| | | US 2016-0196325 A1 | 07 July 2016 |
| | | WO 2016-112021 A1 | 14 July 2016 |
| KR 10-2020-0109199 A | 22 September 2020 | KR 10-2226623 B1 | 11 March 2021 |
| JP 2014-014683 A | 30 January 2014 | CN 102728047 A | 17 October 2012 |
| | | CN 102728047 B | 03 August 2016 |
| | | CN 103520897 A | 22 January 2014 |
| | | CN 103520897 B | 20 October 2017 |
| | | CN 105944366 A | 21 September 2016 |
| | | CN 105944366 B | 21 May 2019 |
| | | EP 2518651 A1 | 31 October 2012 |
| | | EP 2682052 A2 | 08 January 2014 |
| | | EP 2682052 A3 | 21 January 2015 |
| | | EP 3839968 A1 | 23 June 2021 |
| | | JP 2012-216208 A | 08 November 2012 |
| | | JP 2016-179195 A | 13 October 2016 |
| | | JP 6228355 B2 | 08 November 2017 |
| | | JP 6306833 B2 | 04 April 2018 |
| | | US 10556150 B2 | 11 February 2020 |
| | | US 10576329 B2 | 03 March 2020 |
| | | US 10957439 B2 | 23 March 2021 |
| | | US 11011263 B2 | 18 May 2021 |
| | | US 2012-0253484 A1 | 04 October 2012 |
| | | US 2013-0041590 A1 | 14 February 2013 |
| | | US 2015-0352406 A1 | 10 December 2015 |
| | | US 2016-0236035 A1 | 18 August 2016 |
| | | US 2017-0216669 A1 | 03 August 2017 |
| | | US 2018-0015329 A1 | 18 January 2018 |
| | | US 2018-0193697 A1 | 12 July 2018 |
| | | US 2020-0121988 A1 | 23 April 2020 |
| | | US 2020-0171352 A1 | 04 June 2020 |
| | | US 2021-0272673 A1 | 02 September 2021 |
| | | US 2021-0280293 A1 | 09 September 2021 |
| | | US 9141759 B2 | 22 September 2015 |
| | | US 9317660 B2 | 19 April 2016 |
| | | US 9630059 B2 | 25 April 2017 |
| | | US 9802080 B2 | 31 October 2017 |
| | | US 9937383 B2 | 10 April 2018 |
| KR 10-2020-0122328 A | 27 October 2020 | CA 3091635 A1 | 22 August 2019 |
| | | EP 3753005 A1 | 23 December 2020 |

**INTERNATIONAL SEARCH REPORT**
**Information on patent family members**

International application No.
**PCT/KR2022/012766**

| Patent document cited in search report | Publication date (day/month/year) | Patent family member(s) | Publication date (day/month/year) |
|---|---|---|---|
| | | JP 2021-514279 A | 10 June 2021 |
| | | US 11344769 B2 | 31 May 2022 |
| | | US 2019-0258905 A1 | 22 August 2019 |
| | | WO 2019-161201 A1 | 22 August 2019 |